# EUROPEAN PATENT SPECIFICATION

(11) **EP 2 452 609 B1**
(45) Date of publication and mention of the grant of the patent: **31.08.2016**
(21) Application number: 11732907.8
(22) Date of filing: 13.01.2011
(51) Int. Cl.: A61B 1/00, A61B 1/04, A61B 5/07

(54) **IN-VIVO INFORMATION ACQUIRING SYSTEM**
INTRAKORPORALES INFORMATIONSERFASSUNGSSYSTEM
SYSTÈME D'ACQUISITION D'INFORMATIONS INTRACORPORELLES

(30) Priority: 15.01.2010 JP 2010007503
(43) Date of publication of application: 16.05.2012
(73) Proprietor: Olympus Corporation, Shibuya-ku Tokyo (JP)
(72) Inventor: IKAI Takuto, Tokyo 151-0072 (JP); CHIBA, Atsushi, Tokyo 151-0072 (JP)
(74) Representative: Schicker, Silvia
(86) International application number: PCT/JP2011/050403
(87) International publication number: WO 2011/087037

(56) References cited:
- EP-A1- 2 143 371
- JP-A- 4 081 711
- JP-A- 4 357 924
- JP-A- 2005 001 255
- JP-A- 2008 247 201
- JP-A- 2009 031 377

## Description

### TECHNICAL FIELD

The present invention relates to an in-vivo information acquiring system that includes a body-insertable apparatus introduced into a subject and transmitting information acquired inside the subject to the outside thereof.

### BACKGROUND ART

Hitherto, in the field of an endoscope, a capsule endoscope has been introduced which has a capturing function and a wireless communication function inside a capsule-shaped casing formed in a size small enough to be introduced into an alimentary canal of a subject such as a patient. Such a capsule endoscope sequentially acquires images (hereinafter, also referred to as in-vivo images) inside intestines of the subject and sequentially transmits the acquired in-vivo images to a wireless receiving device outside the subject in a wireless manner from when the endoscope is introduced into the alimentary canal of the subject until the endoscope is excreted from the subject. Each in-vivo image captured by the capsule endoscope is received by an image display device through the receiving device. The image display device displays each received in-vivo image on a display in the form of a still image or a moving image. An operator such as a doctor or a nurse observes each in-vivo image of the subject displayed on the image display device, and examines the inside of the intestines of the subject through the observation of each in-vivo image.

In recent years, an in-vivo image acquiring system has been proposed which guides (hereinafter, referred to as "magnetic guiding") a capsule endoscope inside a subject using a magnetic force. In this case, the capsule endoscope further includes a permanent magnet inside a capsule-shaped casing, and an image display device displays in real time each of in-vivo images sequentially captured by the capsule endoscope inside the subject. In the in-vivo image acquiring system, a magnetic field is applied to the capsule endoscope inside the subject, and the capsule endoscope inside the subject is magnetically guided to a desired position using a magnetic force of the magnetic field. A user magnetically guides the capsule endoscope by using an operation unit while referencing the in-vivo image displayed on the image display device. Furthermore, another capsule endoscope has been also proposed which has not only a function of acquiring an in-vivo image but also a function of picking a cell or a tissue inside a subject (for example, refer to Patent Literature 1).

### Citation List

### Patent Literature

Patent Literature 1: Japanese Laid-open Patent Publication No. 2009-131415
Patent Literature 2: J Japanese Laid-open Patent Publication No. H7-221103

EP 2 143 371 A1 discloses a system for guiding a capsule medical device, wherein a display unit displays information indicating that a magnetic field generator is in a state of generating a guidance magnetic field and information indicating that the magnetic field generator is in a state of stopping generating the guidance magnetic field.

### DISCLOSURE OF INVENTION

### PROBLEM TO BE SOLVED BY THE INVENTION

In such an in-vivo image acquiring system, when a magnetic field is generated to guide a capsule endoscope, the operator is notified of generation of the magnetic field by light of an alarm lamp. Therefore, even if the user has a magnetic body therewith, the possibility is decreased that the magnetic body is accidentally attracted to the magnetic field generator by an attracting force act on the magnetic field body. In most cases, the display method uses light to appeal to the eyes. However, it has a problem that light of the alarm lamp may prevent the operator's image observation when the light is reflected on the display, etc.

The present invention has been achieved to solve the above problems and it is an object of the present invention to provide an in-vivo information acquiring system that accurately notifies an operator of generation of a magnetic field and allows the operator to smoothly observe subject information.

### MEANS FOR SOLVING PROBLEM

An in-vivo information acquiring system according to the present invention that solves the above problems and achieves the above object has the features given in claim 1.

Moreover, in the in-vivo information acquiring system according to the present invention, the body-insertable apparatus may be a capsule endoscope that takes an in-vivo image, which is an inner image of a subject, and transmits the taken in-vivo image to outside, and the processing unit is an image processing unit that processes the in-vivo image that is taken by the body-insertable apparatus.

### EFFECT OF THE INVENTION

An in-vivo information acquiring system includes a body-insertable apparatus that has a magnetic field responding unit and that acquires in-vivo information and transmits the acquired in-vivo information to the outside thereof; a magnetic field generator that generates a magnetic field for the magnetic field responding unit; a magnetic field control unit that controls a generation of the magnetic field using the magnetic field generator; a receiving unit that receives the in-vivo information transmitted from the body-insertable apparatus; a processing unit that processes the in-vivo information received by the receiving unit; a display unit that displays the in-vivo information processed by the processing unit on a predetermined display screen; a notifying unit that notifies, using light, an operator of generation of a magnetic field by the magnetic field generator. Because at least one alarm lamp, the processing unit or the display unit is controlled such that the operator, who visually identifies the in-vivo information, is less disturbed by the light from the alarm lamp, it accurately notifies the operator of generation of a magnetic field and allows the operator to smoothly observe subject information.

### BRIEF DESCRIPTION OF DRAWINGS

[Fig. 1] FIG. 1 is a block diagram schematically illustrating a configuration example of an in-vivo image acquiring system according to a first embodiment.
[Fig. 2] FIG. 2 is a schematic diagram illustrating a configuration example of a magnetic field generator illustrated in FIG. 1.
[FIG. 3] FIG. 3 is a schematic cross-sectional view illustrating a configuration example of a capsule endoscope illustrated in FIG. 1.
[FIG. 4] FIG. 4 is a diagram illustrating a position of an alarm lamp illustrated in FIG. 1.
[FIG. 5] FIG. 5 is a flowchart illustrating a process procedure of a magnetic field generation notifying process of the in-vivo image acquiring system illustrated in FIG. 1.
[FIG. 6] FIG. 6 is a diagram illustrating a different position of the alarm lamp illustrated in FIG. 1.
[FIG. 7] FIG. 7 is a diagram illustrating a different position of the alarm lamp illustrated in FIG. 1.
[FIG. 8] FIG. 8 is a block diagram schematically illustrating a configuration example of an in-vivo image acquiring system according to a second embodiment.
[FIG 9] FIG. 9 is a diagram illustrating light emission control of an alarm lamp of the in-vivo image acquiring system illustrated in FIG. 8.
[FIG. 10] FIG. 10 is a flowchart illustrating a process procedure of a magnetic field generation notifying process of the in-vivo image acquiring system illustrated in FIG. 8.
[FIG. 11] FIG. 11 is a block diagram schematically illustrating a configuration example of an in-vivo image acquiring system according to a first modification of the second embodiment.
[FIG. 12] FIG. 12 is a diagrams illustrating light emission control of an alarm lamp of the in-vivo image acquiring system illustrated in FIG. 11.
[FIG. 13] FIG. 13 is a flowchart illustrating a process procedure of a magnetic field generation notifying process of the in-vivo image acquiring system illustrated in FIG. 11.
[FIG. 14] FIG. 14 is a block diagram schematically illustrating a configuration example of an in-vivo image acquiring system according to a second modification of the second embodiment.
[FIG. 15] FIG. 15 is a diagram illustrating light emission control of an alarm lamp of the in-vivo image acquiring system illustrated in FIG. 14.
[FIG. 16] FIG. 16 is a flowchart illustrating a process procedure of a magnetic field generation notifying process of the in-vivo image acquiring system illustrated in FIG. 14.
[FIG. 17] FIG. 17 is a block diagram schematically illustrating a configuration example of an in-vivo image acquiring system according to a third embodiment.
[FIG. 18] FIG 18 is a diagram illustrating alarm lamp transferring control of the in-vivo image acquiring system illustrated in FIG. 17.
[FIG. 19] FIG. 19 is a flowchart illustrating a process procedure of a magnetic field generation notifying process of the in-vivo image acquiring system illustrated in FIG. 17.
[FIG. 20] FIG. 20 is a diagram illustrating another example of the alarm lamp transferring control of the in-vivo image acquiring system illustrated in FIG. 17.
[FIG. 21] FIG. 21 is a flowchart illustrating another process procedure of the magnetic field generation notifying process of the in-vivo image acquiring system illustrated in FIG. 17.
[FIG. 22] FIG. 22 is a diagram illustrating a treatment position setting process illustrated in FIG. 21.
[FIG. 23] FIG. 23 is a block diagram schematically illustrating a configuration example of an in-vivo image acquiring system according to a first modification of the third embodiment.
[FIG. 24] FIG. 24 is a diagram illustrating alarm lamp transferring control of the in-vivo image acquiring system illustrated in FIG. 23.
[FIG. 25] FIG. 25 is a flowchart illustrating a process procedure of a magnetic field generation notifying process of the in-vivo image acquiring system illustrated in FIG. 23.
[FIG. 26] FIG. 26 is a block diagram schematically illustrating a configuration example of an in-vivo image acquiring system according to a second modification of the third embodiment.
[FIG. 27] FIG. 27 is a diagram illustrating alarm lamp transferring control of the in-vivo image acquiring system illustrated in FIG. 26.
[FIG. 28] FIG. 28 is a flowchart illustrating a process procedure of a magnetic field generation notifying process of the in-vivo image acquiring system illustrated in FIG. 26.
[FIG. 29] FIG. 29 is a block diagram schematically illustrating a configuration example of an in-vivo image acquiring system according to a fourth embodiment.
[FIG 30] FIG. 30 is a diagram illustrating alarm lamp transferring control and light emission control of the in-vivo image acquiring system illustrated in FIG. 29, and diagrams provided for explanation of a light emission control.
[FIG. 31] FIG. 31 is a diagram illustrating the alarm lamp transferring control and the light emission control of the in-vivo image acquiring system illustrated in FIG. 29.
[FIG. 32] FIG. 32 is a block diagram schematically illustrating another configuration example of the in-vivo image acquiring system according to the fourth embodiment.
[FIG. 33] FIG. 33 is a block diagram schematically illustrating another configuration example of the in-vivo image acquiring system according to the fourth embodiment.
[FIG. 34] FIG. 34 is a block diagram schematically illustrating a configuration example of an in-vivo image acquiring system according to a fifth embodiment.
[FIG. 35] FIG. 35 is a diagram illustrating an image display process of the in-vivo image acquiring system illustrated in FIG. 34.
[FIG. 36] FIG. 36 is a flowchart illustrating a process procedure of a magnetic field generation notifying process of the in-vivo image acquiring system illustrated in FIG. 34.
[FIG. 37] FIG. 37 is a block diagram schematically illustrating a configuration example of an in-vivo image acquiring system according to a first modification of the fifth embodiment.
[FIG. 38] FIG. 38 is a diagram illustrating an image display process of the in-vivo image acquiring system illustrated in FIG. 37.
[FIG. 39] FIG. 39 is a flowchart illustrating a process procedure of a magnetic field generation notifying process of the in-vivo image acquiring system illustrated in FIG. 37.
[FIG. 40] FIG. 40 is a block diagram schematically illustrating a configuration example of the in-vivo image acquiring system according to a second modification of the fifth embodiment.
[FIG. 41] FIG. 41 is a diagram illustrating an image display process of the in-vivo image acquiring system illustrated in FIG. 40.
[FIG. 42] FIG. 42 is a flowchart illustrating a process procedure of a magnetic field generation notifying process of the in-vivo image acquiring system illustrated in FIG. 40.
[FIG. 43] FIG. 43 is a block diagram schematically illustrating a configuration example of an in-vivo image acquiring system according to a sixth embodiment.
[FIG. 44] FIG. 44 is a diagram illustrating a screen control process of the in-vivo image acquiring system illustrated in FIG. 43.
[FIG. 45] FIG. 45 is a flowchart illustrating a process procedure of a magnetic field generation notifying process of the in-vivo image acquiring system illustrated in FIG. 43.
[FIG. 46] FIG. 46 is a block diagram schematically illustrating a configuration example of an in-vivo image acquiring system according to a first modification of the sixth embodiment.
[FIG. 47] FIG. 47 is a diagram illustrating a screen control process of the in-vivo image acquiring system illustrated in FIG. 46.
[FIG. 48] FIG. 48 is a flowchart illustrating a process procedure of a magnetic field generation notifying process of the in-vivo image acquiring system illustrated in FIG. 46.
[FIG. 49] FIG. 49 is a block diagram schematically illustrating a configuration example of an in-vivo image acquiring system according to a second modification of the sixth embodiment.
[FIG. 50] FIG. 50 is a diagram illustrating a screen control process of the in-vivo image acquiring system illustrated in FIG. 49.
[FIG. 51] FIG. 51 is a flowchart illustrating a process procedure of a magnetic field generation notifying process of the in-vivo image acquiring system illustrated in FIG. 49.
[FIG. 52] FIG. 52 is a block diagram schematically illustrating a configuration example of an in-vivo image acquiring system according to a seventh embodiment.
[FIG. 53] FIG. 53 is a diagram illustrating time dependence of an operation amount of a joystick.
[FIG. 54] FIG. 54 is a flowchart illustrating a process procedure of a magnetic field generation notifying process of the in-vivo image acquiring system illustrated in FIG. 52.
[FIG. 55] FIG. 55 is a block diagram schematically illustrating a configuration example of an in-vivo image acquiring system according to an eighth embodiment.
[FIG. 56] FIG. 56 is a diagram illustrating time dependence of an operation amount of a joystick.
[FIG. 57] FIG. 57 is a flowchart illustrating a process procedure of a magnetic field generation notifying process of the in-vivo image acquiring system illustrated in FIG. 55.
[FIG. 58] FIG. 58 is a block diagram schematically illustrating a configuration example of an in-vivo image acquiring system according to a first modification of the eighth embodiment.
[FIG. 59] FIG. 59 is a flowchart illustrating a process procedure of a magnetic field generation notifying process of the in-vivo image acquiring system illustrated in FIG. 58.
[FIG. 60] FIG. 60 is a block diagram schematically illustrating a configuration example of an in-vivo image acquiring system according to a ninth embodiment.
[FIG. 61] FIG. 61 is a diagram illustrating time dependence of each of an operation amount of a joystick, an operation amount exceeding signal, a magnitude of a magnetic field generated by a magnetic field generator, and an alarm lamp on signal.
[FIG. 62] FIG. 62 is a flowchart illustrating a process procedure of a magnetic field generation notifying process of the in-vivo image acquiring system illustrated in FIG. 60.
[FIG. 63] FIG. 63 is a diagram illustrating time dependence of each of a restraint releasing mode instruction signal, a magnitude of a magnetic field generated by the magnetic field generator, and an alarm lamp on signal for turning on the alarm lamp.
[FIG. 64] FIG. 64 is a flowchart illustrating another process procedure of the magnetic field generation notifying process of the in-vivo image acquiring system illustrated in FIG. 60.
[FIG. 65] FIG. 65 is a block diagram schematically illustrating a configuration example of an in-vivo image acquiring system according to a tenth embodiment.
[FIG. 66] FIG. 66 is a diagram illustrating time dependence of an operation amount of a joystick.
[FIG. 67] FIG. 67 is a flowchart illustrating a process procedure of a magnetic field generation notifying process of the in-vivo image acquiring system illustrated in FIG. 65.
[FIG. 68] FIG. 68 is a block diagram schematically illustrating a configuration example of an in-vivo image acquiring system according to an eleventh embodiment.
[FIG. 69] FIG. 69 is a flowchart illustrating a process procedure of a magnetic field generation notifying process of the in-vivo image acquiring system illustrated in FIG. 68.
[FIG. 70] FIG. 70 is a block diagram schematically illustrating a configuration example of an ultrasonic diagnosis device according to the invention.

### BEST MODE(S) FOR CARRYING OUT THE INVENTION

Hereinafter, an in-vivo information acquiring system according to an embodiment of the invention will be described by exemplifying an in-vivo image acquiring system that uses a capsule endoscope as a body-insertable apparatus introduced into a subject, where the capsule endoscope is introduced into the subject from an oral cavity and captures an in-vivo image. However, the invention is not limited thereto. For example, the invention may be applied to a case of obtaining an in-vivo image by capturing an image while moving inside a lumen from an esophagus of the subject to an anus thereof and a case of using an ultrasonic endoscope by inserting a probe into a body and emitting an ultrasonic wave in the body. Furthermore, the invention is not limited to the embodiments. Further, in the description of the drawings, the same reference numerals are given to the same components.

### First Embodiment

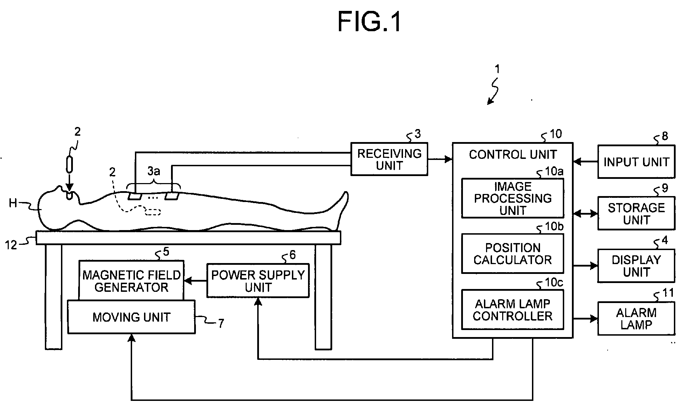

FIG. 1 is a block diagram schematically illustrating a configuration example of an in-vivo image acquiring system according to the first embodiment. An in-vivo image acquiring system 1 according to the first embodiment is a system that introduces a capsule endoscope into a subject and captures living body information on a desired portion inside the subject using the introduced capsule endoscope. In the in-vivo image acquiring system according to the invention, an alarm lamp, an image processing unit, or a display unit is controlled so that an operator who visually identifies an in-vivo image is less disturbed by light emitted from the alarm lamp when a magnetic field generator generates a magnetic field.

Specifically, as illustrated in FIG. 1, the in-vivo image acquiring system 1 includes a capsule endoscope 2 that is introduced into a subject H such as a patient and captures the inside of the subject H, a receiving unit 3 that receives information transmitted from the capsule endoscope 2 through an antenna 3a, and a display unit 4 that displays an in-vivo image of the subject H captured by the capsule endoscope 2. Further, the in-vivo image acquiring system 1 includes a magnetic field generator 5 that generates a magnetic field for guiding the capsule endoscope 2 inside the subject H, a power supply unit 6 that supplies power to the magnetic field generator 5, a moving unit 7 that moves the magnetic field generator 5, an input unit 8 that inputs various information items, a storage unit 9 that stores various information items such as an in-vivo image of the subject H, a control unit 10 that controls respective components of the in-vivo image acquiring system 1, and an alarm lamp 11 that notifies the generation of the magnetic field using the magnetic field generator 5 by light.

The capsule endoscope 2 is formed in a size small enough to be inserted into the subject H. Further, the capsule endoscope 2 includes a capturing function and a wireless communication function inside a capsule-shaped casing. The capsule endoscope 2 is introduced into the subject H through an oral cavity or the like, sequentially captures the in-vivo image of the subject H while moving inside an alimentary canal of the subject H through a peristaltic action or the like, and sequentially transmits an image signal including the in-vivo image obtained to the outside (specifically, to the antenna 3a of the receiving unit 3) of the subject H in a wireless manner each time it captures an image. Later, the capsule endoscope 2 moves inside the alimentary canal of the subject H, and is naturally excreted from the subject H at last.

The receiving unit 3 is connected to the plurality of antennas 3a disposed on the body surface of the subject H, receives a wireless signal from the capsule endoscope 2 through the plurality of antennas 3a, and acquires an image signal included in the received wireless signal. Specifically, the receiving unit 3 compares the received electric field intensities of the wireless signals sequentially received through the plurality of antennas 3a, and selects the antenna having the highest received electric field strength from the plurality of antennas 3a. The receiving unit 3 performs a decoding process or the like on the wireless signal received from the capsule endoscope 2 through the antenna selected in this manner, and extracts an image signal included in the wireless signal. The receiving unit 3 transmits the extracted image signal to the control unit 10. Here, the image signal extracted (decoded) by the receiving unit 3 includes the in-vivo image which is captured by the above-described capsule endoscope 2 inside the subject H.

The plurality of antennas 3a is used to catch a wireless signal from the capsule endoscope 2 introduced into the subject H, and is disposed to be distributed on the body surface of the subject H into which the capsule endoscope 2 is introduced. At least one antenna of the antennas 3a catches a wireless signal from the capsule endoscope 2 located inside the subject H (for example, inside an alimentary canal such as an esophagus, a stomach, a small intestine, and a large intestine), and transmits the caught wireless signal to the receiving unit 3.

The display unit 4 is realized by various displays such as a CRT display, a liquid crystal display, a plasma display, and an organic EL display, and displays various information items instructed to display thereon by the control unit 10. Specifically, the display unit 4 displays an in-vivo image group of the subject H captured by the capsule endoscope 2, patient information and examination information of the subject H input by the input unit 8, current position information of the capsule endoscope 2 inside the subject H, and the like.

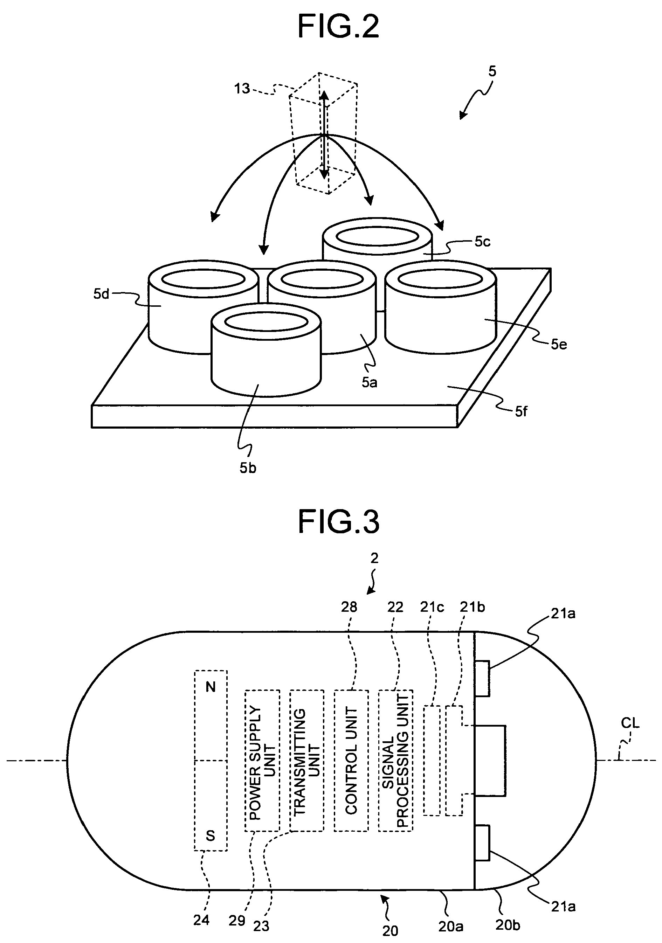

The magnetic field generator 5 is realized by a plurality of electromagnets, and generates a magnetic field to be applied to the capsule endoscope 2 inside the subject H placed on a bed 12. Specifically, as illustrated in FIG. 2, the magnetic field generator 5 includes a plurality of electromagnets 5a to 5e disposed on a plane of a table 5f. Each of the plurality of electromagnets 5a to 5e is a coil-shaped electromagnet that generates a magnetic field by power supplied from the power supply unit 6. The electromagnet 5a is disposed at the substantially center of the table 5f so as to be surrounded by the other electromagnets 5b to 5e. The electromagnets 5b and 5c are disposed at positions symmetrical to each other with respect to the electromagnet 5a, and the electromagnets 5d and 5e are disposed at positions symmetrical to each other with respect to the electromagnet 5a, at the positions where the electromagnets 5b and 5c are rotated about the electromagnet 5a by 90 degrees. The electromagnets 5a to 5e form a rotary magnetic field and a gradient magnetic field inside a three-dimensional space 13 near the center axis of the coil-shaped electromagnet 5a.

Furthermore, since the electromagnets 5a to 5e are disposed on the same surface (that is, on the surface of the table 5f), the electromagnets 5a to 5e on the table 5f increase or decrease a force (a magnetic attracting force) of attracting a magnet (for example, a magnet 24 to be described later inside the capsule endoscope 2) located inside the three-dimensional space 13 toward the electromagnet 5a or a force (a magnetic repelling force) of separating the magnet from the electromagnet 5a while generating a rotary magnetic field inside the three-dimensional space 13. The magnetic attracting force or the magnetic repelling force is a magnetic force generated by the gradient magnetic field formed by the electromagnets 5a to 5e, and serves as a force that presses the above-described capsule endoscope 2 against a portion inside the body of the subject H.

The power supply unit 6 supplies power for forming a magnetic field (a rotary magnetic field and a gradient magnetic field) to be applied to the capsule endoscope 2 inside the subject H to the magnetic field generator 5. Specifically, the power supply unit 6 supplies an AC current to the electromagnets 5a to 5e of the magnetic field generator 5 on the basis of the control of the control unit 10, so that the rotary magnetic field and the gradient magnetic field are formed in the magnetic field generator 5. That is, the rotary magnetic field and the gradient magnetic field formed by the above-described magnetic field generator 5 are controlled by the AC current supplied from the power supply unit 6 (the power feeding amount from the power supply unit 6).

The moving unit 7 is used to move the magnetic field generator 5 relative to the subject H so that the rotary magnetic field and the gradient magnetic field are applied to the capsule endoscope 2 inside the subject H. Specifically, an XY plane is set so as to be substantially parallel to the placement surface of the bed 12 on which the subject H is placed, and the moving unit 7 moves the magnetic field generator 5 to a coordinate position within the XY plane on the basis of the control of the control unit 10. In this case, the moving unit 7 moves the magnetic field generator 5 so that the capsule endoscope 2 inside the subject H is located in the above-described three-dimensional space 13 (refer to FIG. 2).

The input unit 8 is realized by an input device such as a keyboard and a mouse, and various information items are input to the control unit 10 in accordance with the input operation of a user such as a doctor or a nurse. Examples of the various information items input to the control unit 10 by the input unit 8 include instruction information for the control unit 10, patient information of the subject, examination information of the subject, and the like. Furthermore, the patient information of the subject is information that identifies the subject, and includes, for example, patient name, patient ID, date of birth, sex, age, and the like of the subject. Further, the examination information of the subject is information that identifies a living body examination performed by using living body information obtained by capturing the inside of the body using the capsule endoscope 2 introduced into the subject, and includes, for example, an examination ID, an examination date, and the like. Further, the input unit 6 inputs operation information for magnetic guiding using the above-described magnetic field generator 2 inside the capsule endoscope 10. Then, the input unit 8 further includes an operation input section with a joystick. For example, an operator operates the joystick so as to input operation information for magnetically guiding the capsule endoscope 2 to the control unit 10, where the operation information includes the magnetic guiding direction or the magnetic guiding position of the capsule endoscope 2 to be magnetically guided.

The storage unit 9 is realized by various storage media such as a RAM, an EEPROM, a flash memory, or a hard disk for storing information in a rewritable manner. The storage unit 9 stores various information items instructed to be stored by the control unit 10, and provides the control unit 10 with the information the control unit 10 instructs to read out from the stored information. The storage unit 9 stores, for example, the in-vivo image group of the subject H, the patient information and the examination information of the subject H, and the current position of the capsule endoscope 2 inside the subject H on the basis of the control of the control unit 10.

The control unit 10 controls the respective components (the capsule endoscope 2, the receiving unit 3, the display unit 4, the magnetic field generator 5, the power supply unit 6, the moving unit 7, the input unit 8, the storage unit 9, and the alarm lamp 11) of the in-vivo image acquiring system 1, and controls an input and output of a signal between the respective components. Specifically, the control unit 10 controls respective operations of the receiving unit 3, the display unit 4, the moving unit 7, the storage unit 9, and the alarm lamp 11, and controls the power feeding amount of the power supply unit 6 for the magnetic field generator 5 on the basis of the instruction information input by the input unit 8. The control unit 10 controls the magnetic field direction and the magnetic field intensity of the magnetic field generator 5 through the control of the power feeding amount of the power supply unit 6. The control unit 10 controls the magnetic guiding of the capsule endoscope 2 inside the subject H by the control of the magnetic field generator 5.

Further, the control unit 10 includes an image processing unit 10a that creates the in-vivo image of the subject H, a position calculator 10b that calculates the position of the capsule endoscope 2 inside the subject H, and an alarm lamp controller 10c that controls the alarm lamp 11. The image processing unit 10a acquires an image signal decoded from the wireless signal transmitted from the capsule endoscope 2 through the receiving unit 3, performs predetermined image processing on the acquired image signal, and creates (reconstructs) image information corresponding to the image signal, that is, the in-vivo image of the subject H. As described above, the in-vivo image group created by the image processing unit 10a is displayed on the display unit 4 and is stored in the storage unit 9.

From the receiving unit 3, the position calculator 10b acquires the received electric field strength (for example, the three highest received electric field intensities of the plurality of antennas 3a) of each antenna measured at the time when the receiving unit 3 sequentially receives the wireless signal from the capsule endoscope 2 through the plurality of antennas 3a, and calculates the current position of the capsule endoscope 2 inside the subject H on the basis of trigonometry based on position information of each antenna inside the plurality of antennas 3a and the received electric field strength. The control unit 10 correlates the current position information calculated by the position calculator 10b with the in-vivo image of the subject H captured by the capsule endoscope 2 located at the current position. The in-vivo image of the subject H and the current position information of the capsule endoscope 2 correlated with each other by the control unit 10 are displayed on the display unit 4, and are stored in the storage unit 9.

Next, a configuration of the above-described capsule endoscope 2 will be described in detail. FIG. 3 is a schematic diagram illustrating a configuration example of the capsule endoscope 2 according to the first embodiment. As illustrated in FIG. 3, the capsule endoscope 2 according to the first embodiment includes a capsule-shaped casing 20 including a cylindrical casing 20a and a dome-shaped casing 20b. Further, the capsule endoscope 2 includes an illumination unit 21a that illuminates a subject, an optical system 21b that images an optical image of the subject, an imaging unit 21c that captures an image of the subject, a signal processing unit 22 that generates an image signal including the image captured by the imaging unit 21c, and a transmitting unit 23 that wirelessly transmits the image signal to the outside. Furthermore, the capsule endoscope 2 includes the magnet 24 that rotationally drives the capsule-shaped casing 20 in accordance with the external rotary magnetic field, a control unit 28 that controls the respective components of the capsule endoscope 2, and a power supply unit 29 that is realized by a battery or the like.

The capsule-shaped casing 20 is a capsule-shaped casing that is formed in a size small enough to be introduced into the subject H, and is formed in a manner such that the other end (opening end) of the cylindrical casing 20a of which one end is formed in a dome shape is blocked by the dome-shaped casing 20b. The dome-shaped casing 20b is an optical dome that is transparent under the light of a predetermined wavelength bandwidth (for example, visible light). On the other hand, the cylindrical casing 20a is a substantially opaque casing. Inside the capsule-shaped casing 20 including the cylindrical casing 20a and the dome-shaped casing 20b, the illumination unit 21a, the optical system 21b, and the imaging unit 21c are disposed near the dome-shaped casing 20b, and the signal processing unit 22, the transmitting unit 23, the magnet 24, the control unit 28, and the power supply unit 29 are disposed near the cylindrical casing 20a.

The magnet 24 serves as a rotational driving unit that generates a rotational force in the circumferential direction of the capsule-shaped casing 20 in response to the external rotary magnetic field. Specifically, the magnet 24 is realized by a permanent magnet, an electromagnet, or a magnetic body, and is fixed into the cylindrical casing 20a so as to be magnetized in the direction perpendicular to the center axis CL in the longitudinal direction of the capsule-shaped casing 20, that is, in the radial direction of the capsule-shaped casing 20. The magnet 24 presses the capsule-shaped casing 20 against a portion inside the body of the subject H in response to the gradient magnetic field of the above-described magnetic field generator 5, and rotates the capsule-shaped casing 20 in the circumferential direction of the capsule-shaped casing 20 in response to the rotary magnetic field generated by the above-described magnetic field generator 5.

The illumination unit 21a is realized by a light emitting element such as an LED, and illuminates the subject (specifically, inside of the intestines of the subject H) through the dome-shaped casing 20b. The optical system 21b is realized by a condenser lens, a lens scope, and the like, condenses light reflected from the subject illuminated by the illumination unit 21a, and images an optical image of the subject onto a photosensitive surface of the imaging unit 21c. The imaging unit 21c is realized by a solid-state imaging element such as a CCD or a CMOS, and captures the optical image of the subject imaged by the optical system 21b, that is, the in-vivo image of the subject H. The signal processing unit 22 acquires a signal photoelectrically converted by the imaging unit 21c and performs predetermined signal processing on the acquired signal so as to generate an image signal including the in-vivo image of the subject H.

The transmitting unit 23 acquires the image signal generated by the signal processing unit 22, and performs a predetermined modulation process or the like on the acquired image signal so as to generate a wireless signal including the image signal. The transmitting unit 23 transmits thus generated wireless signal to the outside. As described above, the wireless signal transmitted by the transmitting unit 23 is received by the receiving unit 3 through the plurality of antennas 3a.

The control unit 28 controls the illumination unit 21a, the imaging unit 21c, the signal processing unit 22, and the transmitting unit 23. Specifically, the control unit 28 controls the illumination unit 21a and the imaging unit 21c so that the image (that is, the in-vivo image) of the subject illuminated by the illumination unit 21a is captured by the imaging unit 21c, and controls the signal processing unit 22 and the transmitting unit 23 so that the image signal including the in-vivo image of the subject H captured by the imaging unit 21c is wirelessly transmitted to the outside.

The power supply unit 29 is realized by a switch circuit and a button type battery, and starts supplying power to the illumination unit 21a, the imaging unit 21c, the signal processing unit 22, the transmitting unit 23, and the control unit 28 when it is switched on by the switch circuit.

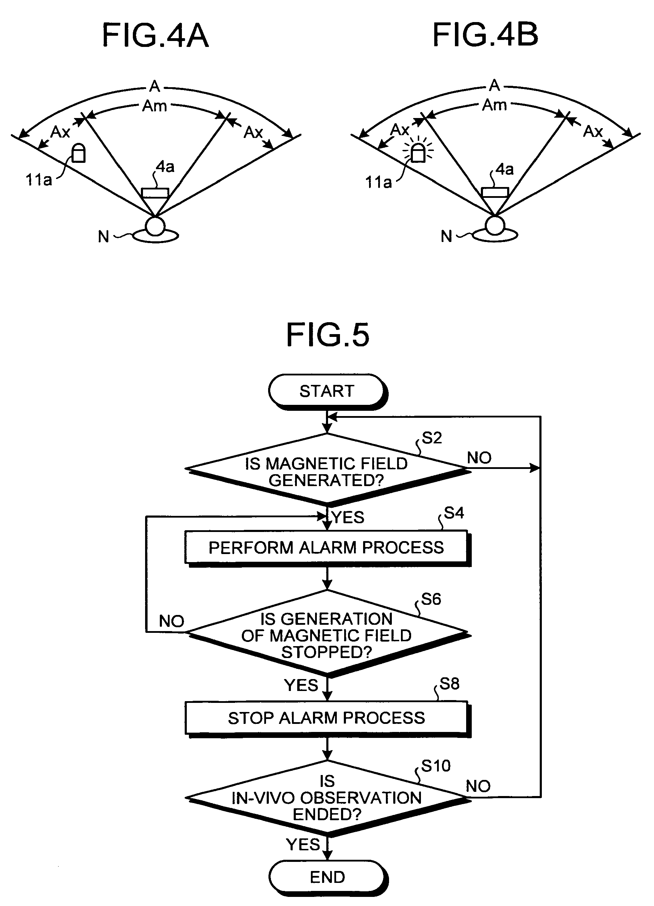

In the in-vivo image acquiring system 1 according to the first embodiment, the alarm lamp 11 is controlled so that the operator who visually identifies the in-vivo image is less disturbed by the light emitted from the alarm lamp when the magnetic field is generated by the magnetic field generator 5. Now, the position of the alarm lamp 11 illustrated in FIG. 1 will be described. FIG. 4 is a diagram illustrating the position of the alarm lamp 11 in the in-vivo image acquiring system 1 according to the first embodiment. In FIG. 4, an example is illustrated in which a single alarm lamp 11a is disposed.

As illustrated in FIG. 4(1), for example, the operator N is located at a position in front of a display 4a so that the operator N can operate the input unit 8 of the in-vivo image acquiring system 1. Here, the alarm lamp 11a is disposed at an outside observation area Ax, an area excluding the straight extension line connecting the display 4a constituting the display unit 4 and the operator N within the viewing area A for the operator N of the in-vivo image acquiring system 1. That is, the alarm lamp 11a is disposed at a position deviated from the center of the viewing range of the operator N.

Then, the alarm lamp controller 10c causes the alarm lamp 11a, which is turned off as illustrated in FIG. 4(1), to emit light as illustrated in FIG. 4(2) when the magnetic field is generated by the magnetic field generator 5. Subsequently, the alarm lamp controller 10c causes the alarm lamp 11a to stop light emission when the generation of the magnetic field stops at the magnetic field generator 5.

Since the alarm lamp 11a is installed inside the viewing range of the operator N, the generation of the magnetic field may be accurately notified to the operator by emitting light from the alarm lamp 11a. Further, the alarm lamp 11a is installed in the outside observation area Ax, an area excluding the display corresponding area Am formed by the straight extension lines connecting the operator N and the display 4a constituting the display unit 4. For this reason, the light of the alarm lamp 11a hardly enters the display corresponding area Am even when light is emitted from the alarm lamp 11a. Further, since the light emitted from the alarm lamp 11a is hardly reflected to the display screen of the display 4a, the operator N who observes the image is not disturbed. In this manner, in the in-vivo image acquiring system 1 according to the first embodiment, the display 4a is installed at a position that is included in the viewing range of the operator N and is excluded from a position where the operator N who visually identifies the in-vivo image in the viewing range is less disturbed. Further, the light emission process of the alarm lamp 11a is controlled so that the operator N who visually identifies the in-vivo image is less disturbed by the light emitted from the alarm lamp 11a. Accordingly, it is possible to accurately notify the operator N of the generation of the magnetic field and allow the operator N to smoothly observe the subject information.

Although the display corresponding area Am is exemplified as the straight lines connecting the operator N and the display 4a constituting the display unit 4, a range where the operator N is less disturbed may be set as the display corresponding area. For example, a curve connecting the operator N and the display 4a constituting the display unit 4 may be adopted.

Next, referring to FIG. 5, a magnetic field generation notifying process of the in-vivo image acquiring system 1 will be described. FIG. 5 is a flowchart illustrating a process procedure of a magnetic field generation notifying process of the in-vivo image acquiring system 1 illustrated in FIG. 1.

As illustrated in FIG. 5, after the in-vivo observation is started, the alarm lamp controller 10c determines whether the magnetic field generator 5 generates the magnetic field (step S2). The alarm lamp controller 10c repeats the determination process of step S2 until the magnetic field generator 5 generates the magnetic field. When it is determined that the magnetic field generator 5 generates the magnetic field (step S2: Yes), the alarm lamp controller 10c causes the alarm lamp 11 to perform an alarm process (step S4). As the alarm process, in the example described in FIG. 4, the alarm lamp 11a installed at the outside observation area Ax emits light.

Subsequently, the alarm lamp controller 10c determines whether the magnetic field generator 5 stops the generation of the magnetic field (step S6). The alarm lamp controller 10c continues the alarm process of step S4 when it is determined that the magnetic field generator 5 does not stop the generation of the magnetic field (step S6: No). On the other hand, the alarm lamp controller 10c causes the alarm lamp 11 to stop the alarm process (step S8) when it is determined that the magnetic field generator 5 stops the generation of the magnetic field (step S6: Yes). For example, in the example illustrated in FIG 4, the alarm lamp 11a stops the generation of light.

Then, the control unit 10 determines whether the in-vivo observation is to be ended on the basis of instruction information or the like from the input unit 8 (step S10). When the control unit 10 determines that the in-vivo observation is not to be ended (step S10: No), the current step returns to step S2, so that the alarm lamp controller 10c determines whether the magnetic field generator 5 generates the magnetic field. On the other hand, the control unit 10 determines that the in-vivo observation is to be ended (step S10: Yes), it ends the process of controlling the alarm lamp 11a using the alarm lamp controller 10c in accordance with the end of the in-vivo observation. The in-vivo image acquiring system 1 may perform the alarm process using the alarm lamp 11 by performing the respective process procedures illustrated in FIG. 5.

Furthermore, in FIG. 4, for example, an example has been described in which a single alarm lamp is provided as the alarm lamp 11, but of course, a plurality of alarm lamps 11 may be provided. In this case, a plurality of alarm lamps may be installed inside the viewing area by providing an alarm lamp even in the display corresponding area Am other than the outside observation area Ax in addition to the plurality of alarm lamps provided in the outside observation area Ax as illustrated in FIG. 4.

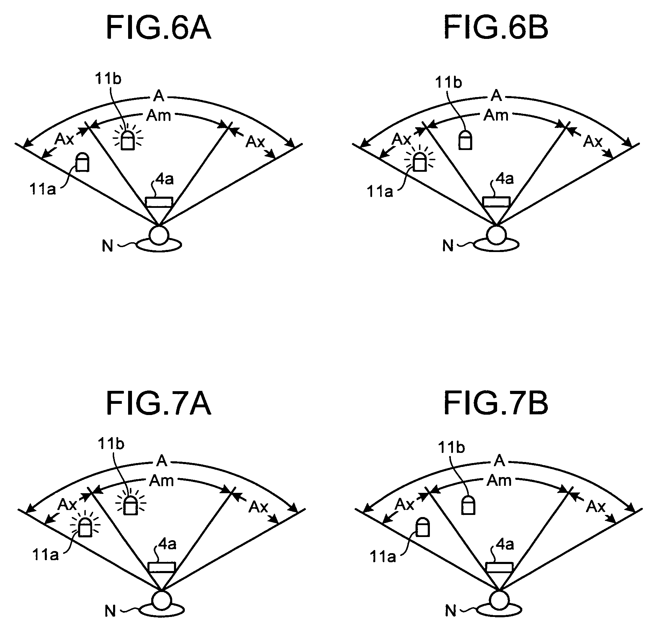

Specifically, as illustrated in FIG. 6, an alarm lamp 11b is provided even in the display corresponding area Am in addition to the alarm lamp 11a in the outside observation area Ax. The alarm lamp 11b is located within the range between the straight lines connecting the operator N and the display 4a. In this case, as illustrated in FIG. 6(1), the alarm lamp controller 10c causes the alarm lamp 11b to light among the alarm lamps 11a and 11b when the magnetic field generator 5 does not generate the magnetic field. Then, as illustrated in FIG. 6(2), the alarm lamp controller 10c causes the alarm lamp 11b to stop lighting when the magnetic field generator 5 generates the magnetic field. That is, when the magnetic field generator 5 does not generate the magnetic field, the alarm lamp controller 10c causes the alarm lamp 11b to light which is at least an alarm lamp located at a position where the operator N who visually identifies the in-vivo image is disturbed by the light from the alarm lamps 11a and 11b among the alarm lamps. Then, the alarm lamp controller 10c causes the alarm lamp 11b to stop lighting when the magnetic field generator 5 stops the generation of the magnetic field.

Furthermore, a case has been described in which the display corresponding area Am is formed by the straight extension lines connecting the operator N and the display 4a constituting the display unit 4, the range where the operator N is less disturbed may be set as the display corresponding area. As the display corresponding area Am, for example, an area formed by the curved extension line connecting the operator N and the display 4a constituting the display unit 4 may be set.

In this case, as in the case of FIG. 4, the alarm lamp 11a located in the outside observation area Ax stops lighting when the magnetic field is not generated as illustrated in FIG. 6(1) and lights when the magnetic field is generated as illustrated in FIG. 6(2) under the control of the alarm lamp controller 10c. In the magnetic field generator 5, the case where the magnetic field is generated indicates a case where the capsule endoscope 2 is guided by the magnetic field and acquiring an image, and corresponds to the case where the in-vivo image is displayed on the display unit 4. Therefore, since the alarm lamp 11b disposed in the display corresponding area Am stops the emission of light when the magnetic field is generated, the operator N performing the image observation through the image acquired by the capsule endoscope 2 and displayed on the display 4a is not disturbed by the light which is emitted from the alarm lamp 11b and reflected on the screen of the display 4a. Also, it is possible to accurately notify the operator N of the generation of the magnetic field by the alarm process of the alarm lamps 11a and 11b.

### Second Embodiment

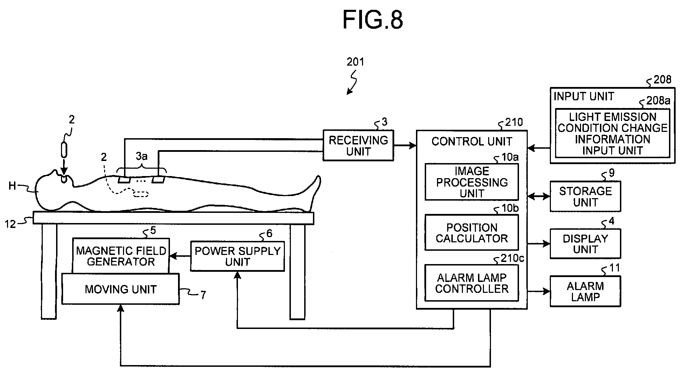

Next, a second embodiment will be described. In the second embodiment, a light emission process of the alarm lamp is controlled so that the light emitted from the alarm lamp is restricted in the outside observation area in order to reduce an influence of the light from the alarm lamp on the operator who visually identifies the in-vivo image. FIG. 8 is a block diagram schematically illustrating a configuration example of an in-vivo image acquiring system according to the second embodiment.

As illustrated in FIG. 8, compared to the in-vivo image acquiring system 1 illustrated in FIG. 1, an in-vivo image acquiring system 201 according to the second embodiment includes an input unit 208 including a light emission condition change information input unit 208a instead of the input unit 8, and includes a control unit 210 instead of the control unit 10. The light emission condition change information input unit 208a inputs instruction information for instructing a change of the light emission condition for the alarm lamp 11. Compared to the control unit 10, the control unit 210 includes an alarm lamp controller 210c instead of the alarm lamp controller 10c. The alarm lamp controller 210c controls the light emission process of the alarm lamp 11 on the basis of the instruction information input by the light emission condition change information input unit 208a.

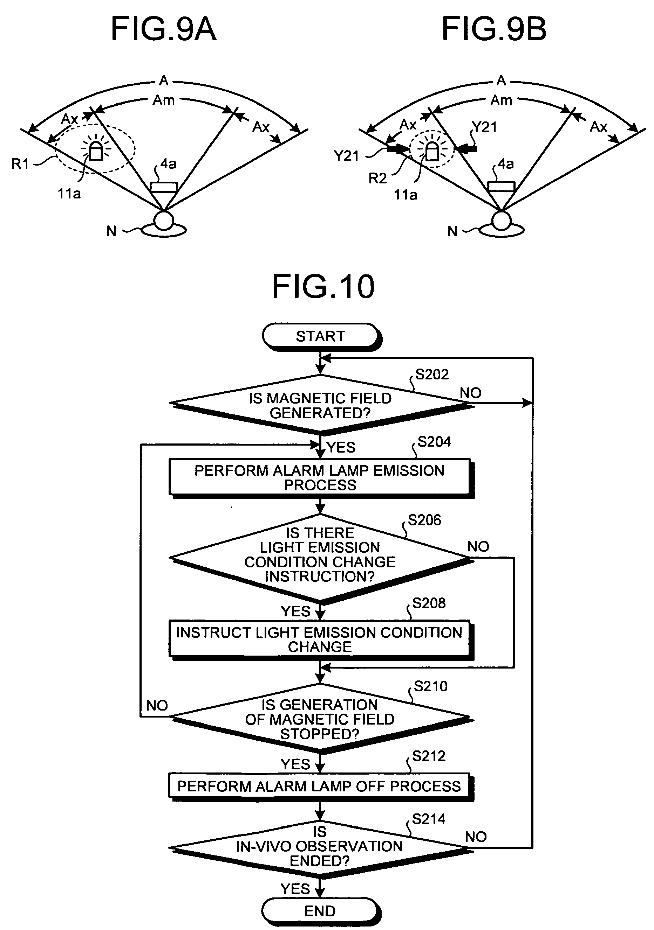

Specifically, referring to FIG 9, the light emission control of the alarm lamp 11 of the in-vivo image acquiring system 201 illustrated in FIG. 8 will be described. As illustrated in FIG. 9, in the second embodiment, the single alarm lamp 11a is disposed at the outside observation area Ax as in FIG. 4.

Then, as illustrated in FIG. 9(1), when the light emission range of the alarm lamp 11a reaches an area R1 exceeding the outside observation area Ax, the operator N operates the light emission condition change information input unit 208a to instruct the alarm lamp controller 210c to narrow the light emission range of the alarm lamp 11a to a desired range. As a result, the instruction information is input from the light emission condition change information input unit 208a to change the light emission condition so that the light emission range of the alarm lamp 11a is narrowed. In accordance with this instruction information, the alarm lamp controller 210c performs control so that the light intensity of the alarm lamp 11a is decreased to restrict the light emitted from the alarm lamp 11a in the outside observation area Ax as illustrated in FIG. 9(2). Accordingly, the light emission range of the alarm lamp 11a is narrowed from the area R1 to the area R2 as illustrated by the arrow Y21. Furthermore, the alarm lamp controller 210c may adjust the emission color of the alarm lamp 11a in addition to the adjustment of the light intensity of the alarm lamp 11a so that the light emission range is not included in the display area Am, but is included in the outside observation area Ax even if the whole part is not included.

In this manner, in the second embodiment, the light emission process of the alarm lamp 11a is controlled so that the light emitted from the alarm lamp 11a is restricted in the outside observation area Ax. Accordingly, the light of the alarm lamp 11a is prevented from entering the display corresponding area Am, and the light of the alarm lamp 11a is prevented from being reflected on the screen of the display 4a, so that an influence of the light of the alarm lamp 11a on the operator is reduced.

Next, referring to FIG. 10, the magnetic field generation notifying process of the in-vivo image acquiring system 201 will be described. FIG. 10 is a flowchart illustrating a process procedure of a magnetic field generation notifying process of the in-vivo image acquiring system 201 illustrated in FIG. 8.

As illustrated in FIG. 10, the alarm lamp controller 210c determines whether the magnetic field generator 5 generates the magnetic field as in step S2 illustrated in FIG. 5 as in step S2 illustrated in FIG. 5 (step S202) and repeats the determination process of step S202 until the magnetic field generator 5 generates the magnetic field. Then, when it is determined that the magnetic field generator 5 generates the magnetic field (step S202: Yes), the alarm lamp controller 210c performs an alarm lamp emission process of emitting light from the alarm lamp 11a of the outside observation area Ax in order to alarm the generation of the magnetic field (step S204).

Then, the alarm lamp controller 210c determines whether there is an instruction for changing the light emission condition of the alarm lamp 11a on the basis of the presence of the instruction information from the light emission condition change information input unit 208a (step S206). When it is determined that there is an instruction for changing the light emission condition of the alarm lamp 11a (step S206: Yes), the alarm lamp controller 210c performs a light emission condition change process of changing the light emission condition of the alarm lamp 11a on the basis of the instruction information from the light emission condition change information input unit 208a (step S208). In this case, the alarm lamp controller 210c changes the emission color in addition to the light intensity of the alarm lamp 11a. As a result, the alarm lamp 11a performs a light emission process on the condition corresponding to the instruction information input from the light emission condition change information input unit 208a.

When it is determined that there is no instruction for changing the light emission condition of the alarm lamp 11a (step S206: No) or the light emission condition change process of step S208 is ended, the alarm lamp controller 210c determines whether the magnetic field generator 5 stops the generation of the magnetic field (step S210). When it is determined that the magnetic field generator 5 does not stop the generation of the magnetic field (step S210: No), the alarm lamp controller 210c continues the alarm lamp emission process of step S204. On the other hand, when it is determined that the magnetic field generator 5 stops the generation of the magnetic field (step S210: Yes), the alarm lamp controller 210c performs an alarm lamp off process of turning off the alarm lamp 11a so as to stop the alarm process (step S212). Then, the control unit 210 determines whether the in-vivo observation is ended (step S214). When it is determined that the in-vivo observation is not ended (step S214: No), the current step returns to step S202. When it is determined that the in-vivo observation is to be ended (step S214: Yes), the process of controlling the alarm lamp 11a by the alarm lamp controller 210c is ended in accordance with the end of the in-vivo observation.

The in-vivo image acquiring system 201 controls the light emission process of the alarm lamp 11a so that the light emitted from the alarm lamp 11a is restricted in the outside observation area Ax by performing the respective process procedures illustrated in FIG. 10.

### First Modified Example of Second Embodiment

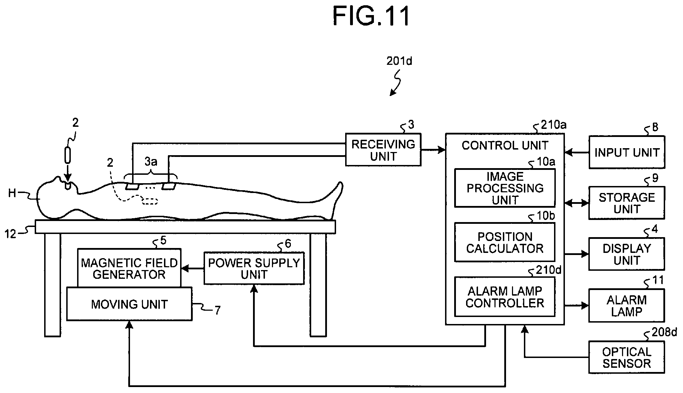

Next, a first modification of the second embodiment will be described. In the first modification of the second embodiment, the light emission state of the alarm lamp is detected by using an optical sensor, and the light emission process of the alarm lamp is performed so that the light emitted from the alarm lamp is restricted in the outside observation area on the basis of the detection result. FIG. 11 is a block diagram schematically illustrating a configuration example of an in-vivo image acquiring system according to the first modification of the second embodiment.

As illustrated in FIG. 11, compared to the in-vivo image acquiring system 201 illustrated in FIG. 8, an in-vivo image acquiring system 201d according to the first modification of the second embodiment includes the input unit 8 instead of the input unit 208, and includes a control unit 210a instead of the control unit 210. The control unit 210a includes an alarm lamp controller 210d instead of the alarm lamp controller 210c. The in-vivo image acquiring system 201a further includes an optical sensor 208d. The optical sensor 208d detects the light emission state of the alarm lamp. The alarm lamp controller 210d controls the light emission process of the alarm lamp 11a on the basis of the detection result of the optical sensor 208d.

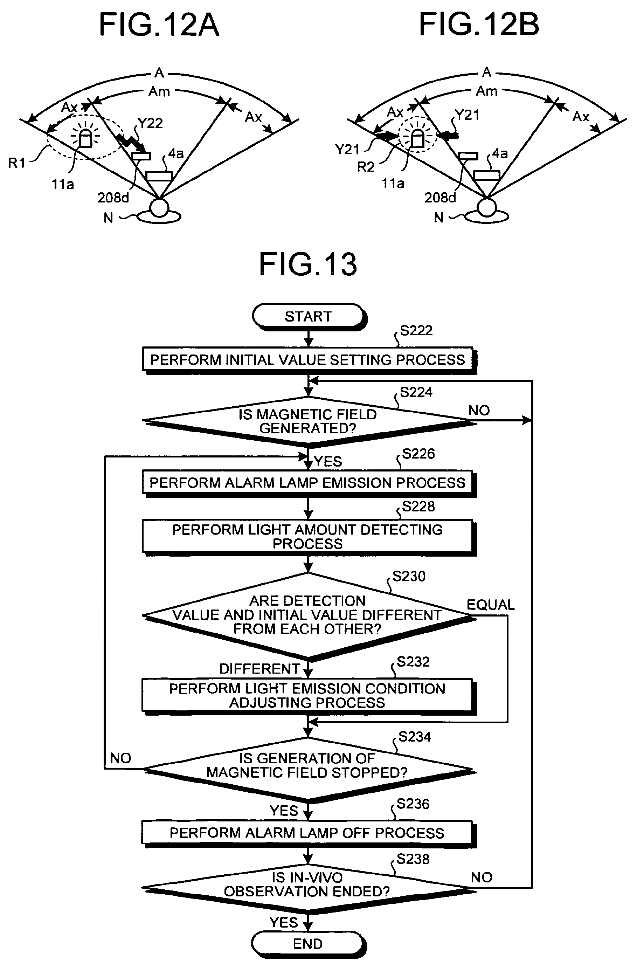

Specifically, referring to FIG. 12, the light emission control on the alarm lamp 11a in the in-vivo image acquiring system 201d illustrated in FIG. 11 will be described. As illustrated in FIG. 12(1), the optical sensor 208d is disposed, for example, near the display 4a of the display corresponding area Am. Then, as illustrated in FIG. 12(1), the optical sensor 208d detects the light amount of the light reaching the optical sensor 208d as depicted by the arrow Y22 as the light emission state of the alarm lamp 11a.

The alarm lamp controller 210d compares the light amount (initial value) when the light emitted from the alarm lamp 11a is restricted in the outside observation area Ax detected in advance by the optical sensor 208d and the light amount (detection value) detected by the optical sensor 208d.

When the initial value and the detection value are different from each other, and the detection value becomes larger than the initial value, as illustrated in FIG. 12(1), the light emission range of the alarm lamp 11a reaches the area R1 exceeding the outside observation area Ax. For this reason, the alarm lamp controller 210d decreases the light intensity of the alarm lamp 11a in accordance with a difference value between the initial value and the detection value, so that the light emitted from the alarm lamp 11a is restricted in the outside observation area Ax. Accordingly, as illustrated in FIG. 12(2), the light emission range of the alarm lamp 11a is narrowed from the area R1 to the area R2 as depicted by the arrow Y21. Furthermore, the optical sensor 208d may detect the emission color instead of the light intensity of the alarm lamp 11a. Further, the optical sensor 208d is not limited to be installed near the display 4a since it only needs to detect the light emission state of the alarm lamp 11a. Accordingly, the optical sensor 208d may be installed near the operator N or may be directly attached to the head of the operator N.

In this manner, in the first modification of the second embodiment, since the light emission process of the alarm lamp 11a is automatically controlled so that the light emitted from the alarm lamp 11a is restricted in the outside observation area Ax on the basis of the detection result of the optical sensor 208d, the operator may concentrate on the in-vivo observation without performing the light emission adjusting process of the alarm lamp 11a.

Next, referring to FIG. 13, the magnetic field generation notifying process of the in-vivo image acquiring system 201d will be described. FIG. 13 is a flowchart illustrating a process procedure of a magnetic field generation notifying process of the in-vivo image acquiring system 201d illustrated in FIG. 11.

As illustrated in FIG. 13, in the in-vivo image acquiring system 201d, the light amount of the alarm lamp 11a is controlled so that the light emitted from the alarm lamp 11a is restricted in the outside observation area Ax under the control of the control unit 210a, and the light amount in this case is detected by the optical sensor 208d, and the initial value setting process is performed to set the detected light amount as the initial value (step S222). This initial value setting process needs not to be performed for each in-vivo observation, but may be performed once when the operator is changed. Further, the process may be periodically performed in order to maintain precision constant. Further, the alarm lamp controller 210d turns off the alarm lamp 11a after the initial value setting process.

Subsequently, as in step S2 illustrated in FIG. 5, the alarm lamp controller 210d determines whether the magnetic field generator 5 generates the magnetic field (step S224) and repeats the determination process of step S224 until the magnetic field generator 5 generates the magnetic field. Then, when it is determined that the magnetic field generator 5 generates the magnetic field (step S224: Yes), the alarm lamp controller 210d performs an alarm lamp emission process of emitting light from the alarm lamp 11a in the outside observation area Ax in order to alarm the generation of the magnetic field (step S226).

Then, the alarm lamp controller 210d performs a light amount detecting process on the optical sensor 208d so as to detect the light amount of the light reaching the optical sensor 208d (step S228). Then, the alarm lamp controller 210d determines whether the detection value and the initial value are different from each other by comparing the detection value and the initial value with each other (step S230). Furthermore, in step S230, the matching degree between the detection value and the initial value may be set by taking account of variations in accordance with the detection precision of the optical sensor 208d, the posture of the operator N, the emission precision of the alarm lamp 11a, and the like.

When it is determined that the detection value and the initial value are different from each other (step S230: different), the alarm lamp controller 210d performs a light emission condition adjusting process of adjusting the light intensity of the alarm lamp 11a in accordance with a difference value between the initial value and the detection value so that the light emitted from the alarm lamp 11a is restricted in the outside observation area Ax (step S232). For example, in the light emission condition adjusting process, a table is stored in advance in which the change amount of the light intensity of the alarm lamp 11a is correlated to each difference value between the initial value and the detection value, and the alarm lamp controller 210d sets the change amount of the light intensity of the alarm lamp 11a by referring to the table. As a result, the alarm lamp 11a performs a light emission process with the changed light intensity.

When it is determined that the detection value and the initial value are substantially equal to each other (step S230: equal) or the light emission condition adjusting process of step S232 is ended, the alarm lamp controller 210d determines whether the magnetic field generator 5 stops the generation of the magnetic field (step S234). When it is determined that the magnetic field generator 5 does not stop the generation of the magnetic field (step S234: No), the alarm lamp controller 210d continues the alarm lamp emission process of step S226. On the other hand, when it is determined that the magnetic field generator 5 stops the generation of the magnetic field (step S234: Yes), the alarm lamp controller 210d performs an alarm lamp off process of turning off the alarm lamp 11a in order to stop the alarm process (step S236). Then, the control unit 210a determines whether the in-vivo observation is ended (step S238). When it is determined that the in-vivo observation is not ended (step S238: No), the current step returns to step S224. When it is determined that the in-vivo observation is ended (step S238: Yes), the process of controlling the alarm lamp 11a of the alarm lamp controller 210d is ended in accordance with the end of the in-vivo observation.

The in-vivo image acquiring system 201d automatically controls the light emission process on the alarm lamp 11a so that the light emitted from the alarm lamp 11a is restricted in the outside observation area Ax by performing the respective process procedures illustrated in FIG. 13. Further, since the in-vivo image acquiring system 201d may automatically adjust the light intensity of the alarm lamp 11a so that the alarm lamp 11a appropriately emits light within the outside observation area Ax by performing the respective process procedures illustrated in FIG. 13 not only in the case where the light emission range of the alarm lamp 11a exceeds the outside observation area Ax, but also in the case where the light emission range is narrower than the initial setting range, it is possible to more accurately notify the operator of the generation of the magnetic field.

Furthermore, as the initial value setting process of step S222 illustrated in FIG. 13, the emission color of the alarm lamp 11a may be adjusted so that the light emitted from the alarm lamp 11a is restricted in the outside observation area Ax, and the emission color in this case may be detected by the optical sensor 208d, and the detected emission color may be set as the initial value. In this case, the optical sensor 208d performs an emission color detecting process instead of the light amount detecting process, and as the light emission condition adjusting process, the alarm lamp controller 210d adjusts the emission color instead of the light intensity of the alarm lamp 11a on the basis of the detection result of the optical sensor 208d. Further, the in-vivo image acquiring system 201d may adjust both the emission color and the light intensity of the alarm lamp 11a so that the light emitted from the alarm lamp 11a is restricted in the outside observation area Ax.

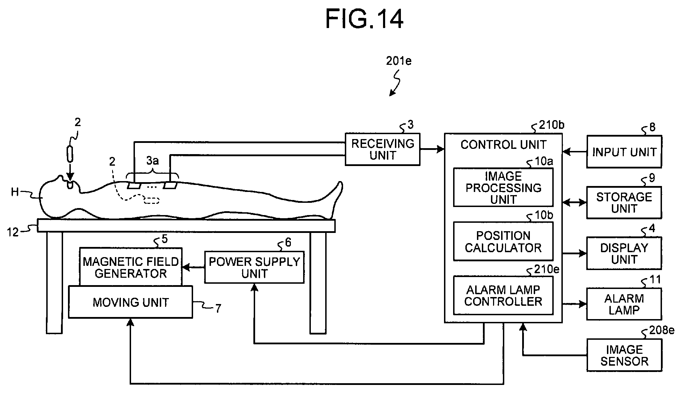

Second Modified Example of Second Embodiment Next, a second modification of the second embodiment will be described. In the second modification of the second embodiment, the motions of the eyes of the operator are detected by using an image sensor instead of the optical sensor, a variation in distance between the eyes of the operator and the alarm lamp 11a is calculated on the basis of the detection result, and the light emission process of the alarm lamp 11a is controlled on the basis of the calculated distance variation. FIG. 14 is a block diagram schematically illustrating a configuration example of an in-vivo image acquiring system according to the second modification of the second embodiment.

As illustrated in FIG. 14, compared to the in-vivo image acquiring system 201d illustrated in FIG. 11, an in-vivo image acquiring system 201e according to the second modification of the second embodiment includes an image sensor 208e instead of the optical sensor 208d, and includes a control unit 210b instead of the control unit 210a. The control unit 210b includes an alarm lamp controller 210e instead of the alarm lamp controller 210d. The image sensor 208e detects the motion of the eyes of the operator. The alarm lamp controller 210e controls the light emission process of the alarm lamp 11a on the basis of the detection result using the image sensor 208e.

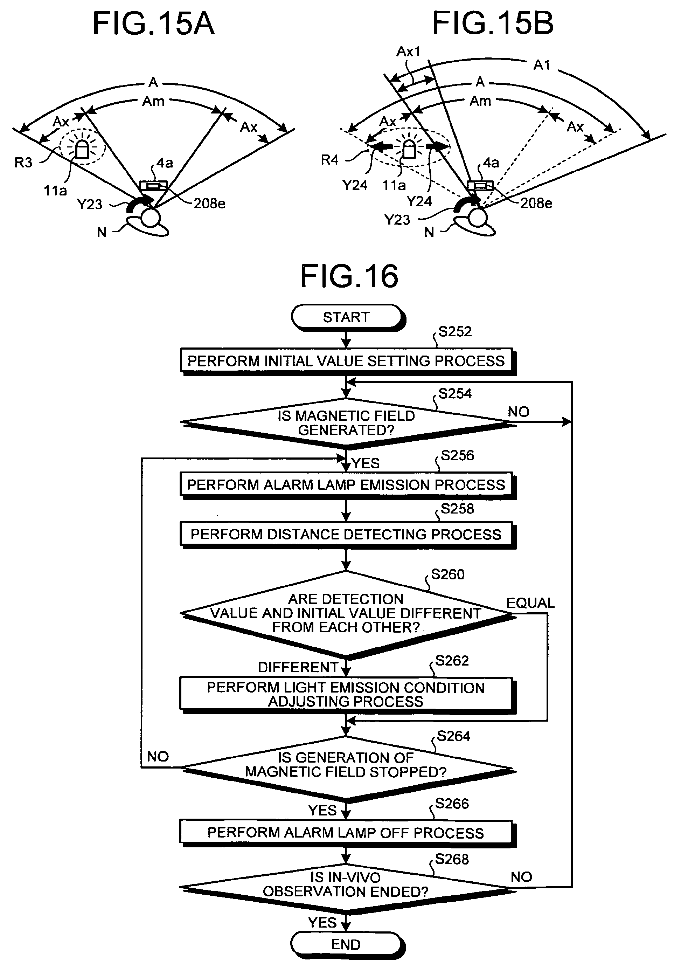

Specifically, referring to FIG. 15, the light emission control on the alarm lamp 11a by the in-vivo image acquiring system 201e illustrated in FIG. 14 will be described. As illustrated in FIG. 15(1), the image sensor 208e is installed at the upper portion of the display 4a so that the eyes of the operator N are included in the imaging field. Then, the image sensor 208e detects the motions of the eyes of the operator N by continuously detecting the eyes of the operator N. The image sensor 208e outputs a series of captured images to the alarm lamp controller 210e.

The alarm lamp controller 210e calculates a variation in distance between the eyes of the operator N and the alarm lamp 11a by processing the series of images captured by the image sensor 208e. For example, when the operator N turns to the right side of the drawing as depicted by the arrow Y23 of FIG. 15(1), the eyes of the operator N also move to the right side of the drawing. Accordingly, also in the series of captured images of the image sensor 208e, the positions of the eyes of the facing operator N move to the left, and the area of the region occupied by the eyes in the captured image changes. The alarm lamp controller 210e obtains the moved directions and angles of the eyes of the operator N by processing the series of captured images captured by the image sensor 208e, and calculates a variation in distance between the eyes of the operator N and the alarm lamp 11a in accordance with the motions of the eyes of the operator N on the basis of the obtained angle and the obtained motions of the eyes of the operator N. Then, the alarm lamp controller 210e adjusts the light emission process of the alarm lamp 11a on the basis of the calculated distance variation.

For example, as illustrated in FIG. 15(2), when the operator N turns to the right side of the drawing as depicted by the arrow Y23, the eyes of the operator N also move to the right side of the drawing. Accordingly, the viewing area of the operator N moves from the viewing area A to the viewing area A1 to the right of the drawing, therefore, the distance between the eyes of the operator N and the alarm lamp 11a becomes larger than that before the operator N turns to the right side of the drawing. In this case, the alarm lamp controller 210e increases the light intensity of the alarm lamp 11a by the increased distance between the eyes of the operator N and the alarm lamp 11a in accordance with the motion of the operator N. Accordingly, the light emission range of the alarm lamp 11a is widened from the area R3 to the area R4 as depicted by the arrow Y24 of FIG. 15(2), so that the light of the alarm lamp 11a reaches the outside observation area Ax1, a resulting area after the operator N turns to the right side.

Furthermore, since the image sensor 208e may be enough if it may detect the motions of the eyes of the operator N, the image sensor 208e is not limited to be installed at the upper portion of the display 4a, and the image sensor 208e may be installed at an area between the display 4a and the operator N. Further, since the image sensor may be enough if it may detect the motions of the eyes of the operator N, the images of the eyes of the operator N do not need to be captured. For example, the actual motions of the eyes of the operator N may be determined in a manner such that the image sensor 208e is attached to the head of the operator N, various motions of the eyes of the operator N are continuously captured in advance, and the image group corresponding to the respective motions of the eyes captured in advance is compared with the series of images captured by the image sensor 208e attached to the head of the operator N during the in-vivo observation.

In this manner, in the second modification of the second embodiment, even when the viewing area moves by the motions of the eyes of the operator on the basis of the detection result of the image sensor 208e, the light emission range of the alarm lamp 11a is automatically adjusted so that the light of the alarm lamp 11a is restricted in the outside observation area that moves together with the viewing area. As a result, since the operator may visibly identify the alarm process by the alarm lamp 11a when any motions of the eyes are performed, the generation of the magnetic field may be accurately detected.

Next, referring to FIG. 16, the magnetic field generation notifying process of the in-vivo image acquiring system 201e will be described. FIG. 16 is a flowchart illustrating a process procedure of a magnetic field generation notifying process of the in-vivo image acquiring system 201e illustrated in FIG. 14.

As illustrated in FIG. 16, in the in-vivo image acquiring system 201e, the light amount of the alarm lamp 11a is adjusted so that the light emitted from the alarm lamp 11a is restricted in the outside observation area Ax of the operator N under the control of the control unit 210b, and the initial value setting process is performed so that a value obtained by adding the distance between the image sensor 208e and the alarm lamp 11a which are disposed in a fixed state to the distance between the image sensor 208e during the basic operation of the operator N and the eyes of the operator N is set as the initial value (step S252). In the initial value setting process, for example, the basic operation time is set as the case where the operator N visually identifies the display 4a directly from the front surface thereof, the imaging process using the image sensor 208e is performed, and the distance between the image sensor 208e and the eyes of the operator N is obtained on the basis of the image captured by the image sensor 208e. Further, the initial value setting process does not need to be performed for each in-vivo observation, but may be performed once when the operator is changed. Further, the process may be periodically performed in order to maintain the precision constant. Further, the alarm lamp controller 210e turns off the alarm lamp 11a after the initial value setting process.

Subsequently, as in step S2 illustrated in FIG. 5, the alarm lamp controller 210e determines whether the magnetic field generator 5 generates the magnetic field (step S254), and repeats the determination process of step S254 until the magnetic field generator 5 generates the magnetic field. Then, when it is determined that the magnetic field generator 5 generates the magnetic field (step S254: Yes), the alarm lamp controller 210e performs an alarm lamp emission process of emitting light from the alarm lamp 11a in the outside observation area Ax in order to warn the generation of the magnetic field (step S256).

Then, the alarm lamp controller 210e performs a distance detecting process of detecting the distance between the eyes of the operator and the alarm lamp 11a by capturing the eyes of the operator N using the image sensor 208e and processing the series of images captured by the image sensor 208e (step S258). In the distance detecting process, the distance between the eyes of the operator N and the image sensor 208e is calculated by processing a series of images captured by the image sensor 208e, and the distance between the eyes of the operator N and the alarm lamp 11a is detected by adding the calculated distance to the distance between the image sensor 208e and the alarm lamp 11a which are disposed in a fixed state.

Then, the alarm lamp controller 210e determines whether the detection value and the initial value are different from each other by comparing the detection value and the initial value with each other (step S260). Furthermore, in step S260, the matching degree between the detection value and the initial value may be set by including variations in accordance with the imaging precision and the like of the image sensor 208e.

When it is determined that the detection value and the initial value are different from each other (step S260: different), the alarm lamp controller 210e performs a light emission condition adjusting process of adjusting the light intensity of the alarm lamp 11a in accordance with a difference value between the detection value and the initial value (step S262). For example, in the light emission condition adjusting process, a table is stored in advance in which a change amount of the light intensity of the alarm lamp 11a is correlated to each difference value between the initial value and the detection value, that is, a distance change amount with respect to the initial value, and the alarm lamp controller 210e sets the change amount of the light intensity of the alarm lamp 11a by referring to the table. As a result, the alarm lamp 11a performs a light emission process with the changed light intensity, and the light of the alarm lamp 11a is restricted in the outside observation area moving together with the viewing area in accordance with the motion of the eyes of the operator N.

When it is determined that the detection value and the initial value are substantially equal to each other (step S260: equal) or the light emission condition adjusting process of step S262 is ended, the alarm lamp controller 210e determines whether the magnetic field generator 5 stops the generation of the magnetic field (step S264). When it is determined that the magnetic field generator 5 does not stop the generation of the magnetic field (step S264: No), the alarm lamp controller 210e continues the alarm lamp emission process of step S256. On the other hand, when it is determined that the magnetic field generator 5 stops the generation of the magnetic field (step S264: Yes), the alarm lamp controller 210e performs an alarm lamp off process of turning off the alarm lamp 11a in order to stop the alarm process (step S266). Then, the control unit 210b determines whether the in-vivo observation is ended (step S268). When it is determined that the in-vivo observation is not ended (step S268: No), the current step returns to step S254. When it is determined that the in-vivo observation is ended (step S268: Yes), the process of controlling the alarm lamp 11a of the alarm lamp controller 210e is also ended in accordance with the end of the in-vivo observation.

The in-vivo image acquiring system 201e automatically adjusts the light emission range of the alarm lamp 11a so that the light of the alarm lamp 11a is restricted in the outside observation area moved together with the viewing area by performing the respective process procedures illustrated in FIG. 16 even when the motions of the eyes of the operator is moved by the viewing area. Further, the in-vivo image acquiring system 201e may automatically adjust the light intensity of the alarm lamp 11a so that the alarm lamp 11a appropriately emits light inside the outside observation area moved together with the viewing area by performing the respective process procedures illustrated in FIG. 13 not only when a distance between the eyes of the operator N and the alarm lamp 11a increases but also when it decreases.

### Third Embodiment

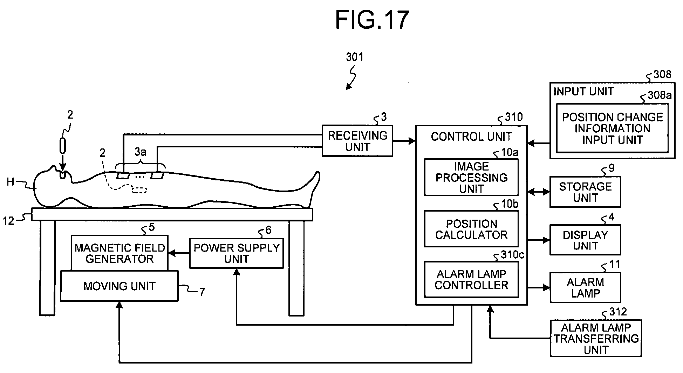

Next, a third embodiment will be described. In the third embodiment, the position of the alarm lamp is controlled by transferring the alarm lamp to be located inside the outside observation area so that the operator who visually identifies the in-vivo image is less disturbed by the light emitted from the alarm lamp. FIG. 17 is a block diagram schematically illustrating a configuration example of the in-vivo image acquiring system according to the third embodiment.

As illustrated in FIG. 17, compared to the in-vivo image acquiring system 1 illustrated in FIG. 1, an in-vivo image acquiring system 301 according to the third embodiment includes an input unit 308 including a position change information input unit 308a instead of the input unit 8, and includes a control unit 310 instead of the control unit 10. Then, the in-vivo image acquiring system 301 further includes an alarm lamp transferring unit 312 that transfers the alarm lamp 11. The position change information input unit 308a inputs instruction information for instructing a change of the position of the alarm lamp 11. Compared to the control unit 10, the control unit 310 includes an alarm lamp controller 310c instead of the alarm lamp controller 10c. The alarm lamp controller 310c controls a process of transferring the alarm lamp 11 on the basis of the instruction information input by the position change information input unit 308a together with the light emission process of the alarm lamp 11.

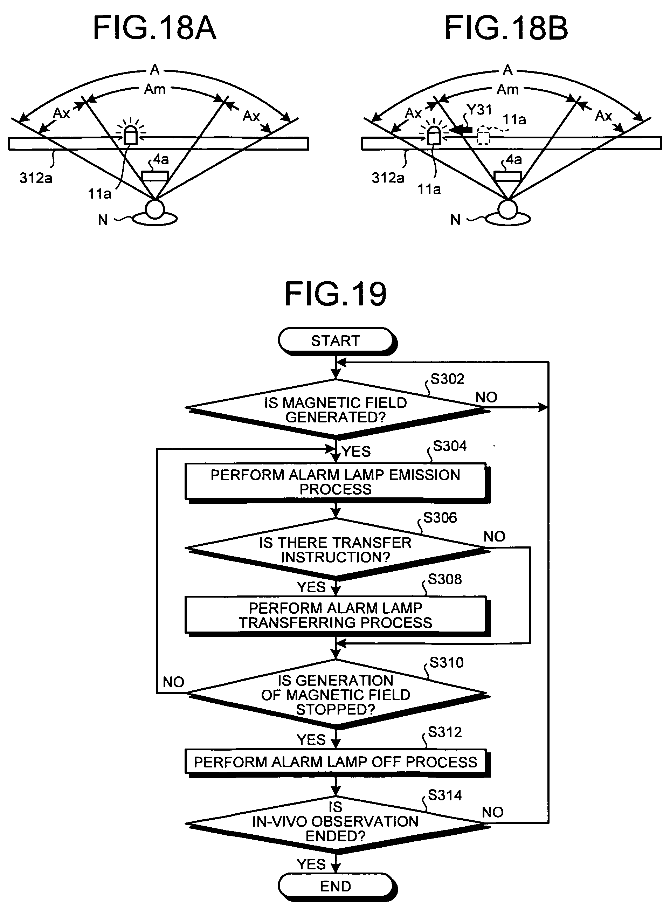

Specifically, referring to FIG. 18, control of transferring the alarm lamp 11 of the in-vivo image acquiring system 301 illustrated in FIG. 17 will be described. As illustrated in FIG. 18, in the third embodiment, the single alarm lamp 11a is disposed as in FIG. 4. Then, the alarm lamp transferring unit 312 includes a transfer mechanism 312a that is capable of transferring the alarm lamp 11a in an area including the viewing range as illustrated in FIG. 18. For example, the alarm lamp 11a disposed on the belt is transferred by rotating the belt.

Then, as illustrated in FIG. 18(1), when the alarm lamp 11a is located at the display corresponding area Am instead of in the outside observation area Ax, the operator N operates the position change information input unit 308a, and instructs to change the position of the alarm lamp 11a to the outside observation area Ax. As a result, the instruction information for changing the position of the alarm lamp 11a to the outside observation area Ax is input from the position change information input unit 308a. In accordance with this instruction information, the alarm lamp controller 310c controls the transfer process of the transfer mechanism 312a so that the alarm lamp 11a is located inside the outside observation area Ax as illustrated in FIG. 18(2). Accordingly, the alarm lamp 11a is transferred so that the light emission range is not included in the display area Am, but is included in the outside observation area as depicted by Ax the arrow Y31.

In this manner, in the third embodiment, the process of transferring the alarm lamp 11a is controlled so that the alarm lamp 11a is located inside the outside observation area Ax. Accordingly, it is ensured that the alarm lamp 11a is located inside the outside observation area Ax, so that the light of the alarm lamp 11a may be prevented from being reflected on the screen of the display 4a and the influence of the light of the alarm lamp 11a with respect to the operator may be reduced.

Next, referring to FIG. 19, the magnetic field generation notifying process of the in-vivo image acquiring system 301 will be described. FIG. 19 is a flowchart illustrating a process procedure of a magnetic field generation notifying process of the in-vivo image acquiring system 301 illustrated in FIG. 17.

As illustrated in FIG. 19, the illustrated alarm lamp controller 310c determines whether the magnetic field generator 5 generates the magnetic field (step S302) as in step S2 illustrated in FIG. 5, and repeats the determination process of step S302 until the magnetic field generator 5 generates the magnetic field. Then, when it is determined that the magnetic field generator 5 generates the magnetic field (step S302: Yes), the alarm lamp controller 310c performs an alarm lamp emission process of emitting light from the alarm lamp 11a in the outside observation area Ax in order to alarm the generation of the magnetic field (step S304).

Then, the alarm lamp controller 310c determines whether there is an instruction for transferring the alarm lamp 11a on the basis of the presence of the instruction information from the position change information input unit 308a (step S306). When it is determined that there is an instruction for transferring the alarm lamp 11a (step S306: Yes), the alarm lamp controller 310c performs an alarm lamp transferring process of transferring the position of the alarm lamp 11a in accordance with the instruction information from the position change information input unit 308a (step S308). Accordingly, as illustrated in FIG. 18(2), the position of the alarm lamp 11a changes from the display corresponding area Am to the outside observation area Ax as depicted by the arrow Y31.

When it is determined that there is no instruction for transferring the alarm lamp 11a (step S306: No) or the alarm lamp transferring process of step S308 is ended, the alarm lamp controller 310c determines whether the magnetic field generator 5 stops the generation of the magnetic field (step S310). When it is determined that the magnetic field generator 5 does not stop the generation of the magnetic field (step S310: No), the alarm lamp controller 310c continues the alarm lamp emission process of step S304. On the other hand, when it is determined that the magnetic field generator 5 stops the generation of the magnetic field (step S310: Yes), the alarm lamp controller 310c performs an alarm lamp off process of turning off the alarm lamp 11a in order to stop the alarm process (step S312). Then, the control unit 310 determines whether the in-vivo observation is ended (step S314). When it is determined that the in-vivo observation is not ended (step S314: No), the current step returns to step S302. When it is determined that the in-vivo observation is ended (step S314: Yes), the process of controlling the alarm lamp 11a of the alarm lamp controller 310c is also ended in accordance with the end of the in-vivo observation.

The in-vivo image acquiring system 301 controls the position of the alarm lamp 11a so that the alarm lamp 11a is located inside the outside observation area Ax corresponding to each operator N by performing the respective process procedures illustrated in FIG. 19.

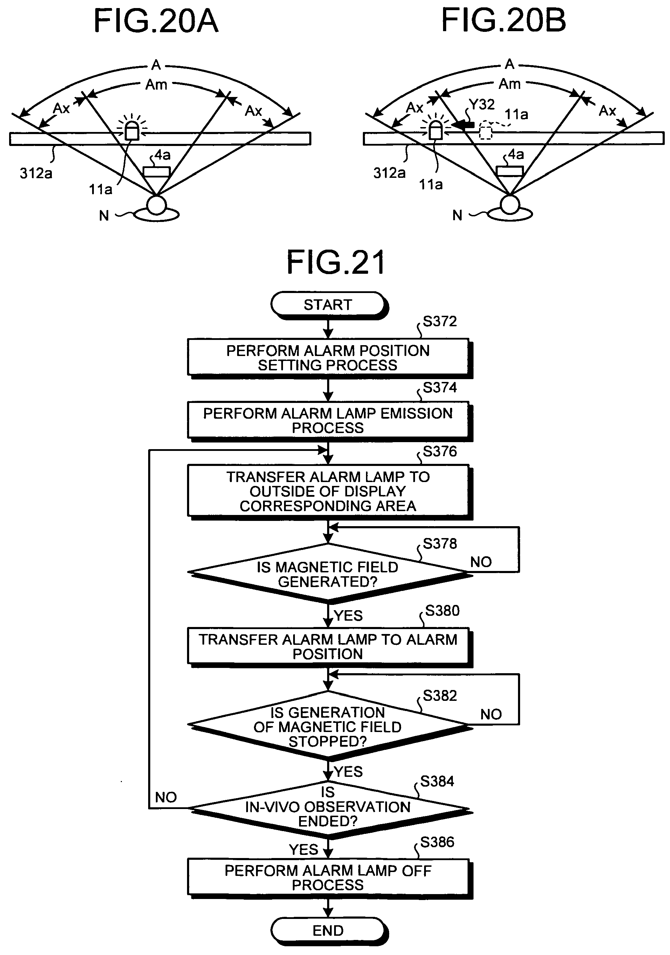

Furthermore, in the third embodiment, the generation of the magnetic field may be notified by changing the position of the alarm lamp 11a when the magnetic field is generated or is not generated while the alarm lamp 11a emits light. In this case, the alarm lamp controller 310c locates the alarm lamp 11a in the display corresponding area Am when the magnetic field generator 5 does not generate the magnetic field as illustrated in FIG. 20(1), and transfers the alarm lamp 11a to the outside observation area Ax using the transfer mechanism 312a as depicted by the arrow Y32 of FIG. 20(2) when the magnetic field generator 5 generates the magnetic field. Then, the alarm lamp controller 310c transfers the alarm lamp 11a to the display corresponding area Am using the transfer mechanism 312a when the magnetic field generator 5 stops the generation of the magnetic field.

In this case, since the alarm lamp 11a is located at the outside observation area Ax while the magnetic field is generated, that is, while the in-vivo image is displayed on the display screen of the display 4a, the generation of the magnetic field may be notified, and the light of the alarm lamp 11a may be prevented from being reflected on the display 4a.

Next, the magnetic field generation notifying process in this case will be described. FIG. 21 is a flowchart illustrating another process procedure of the magnetic field generation notifying process of the in-vivo image acquiring system 301 illustrated in FIG. 17.

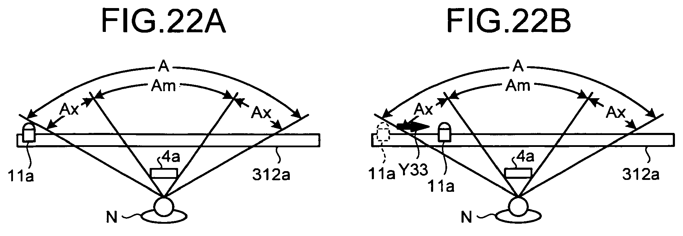

As illustrated in FIG. 21, in the in-vivo image acquiring system 301, the alarm position setting process is performed first so as to set the position of the alarm lamp 11a when warning the generation of the magnetic field (step S372). In the alarm position setting process, as illustrated in FIGS. 22(1) and 22(2), the transfer mechanism 312a transfers the position of the alarm lamp 11a to the outside observation area Ax as depicted by the arrow Y33 so that the alarm lamp 11a is located at the outside observation area Ax according to the operation on the position change information input unit 308a and under the control of the control unit 310, and the alarm lamp controller 310c sets the position as the alarm position for the alarm lamp 11a to take when the magnetic field is generated. Then, the alarm lamp controller 310c sets the display corresponding area Am out of the outside observation area Ax in the viewing area A as the position for the alarm lamp 11a to take when the magnetic field is not generated. Furthermore, the alarm position setting process needs not to be performed for each in-vivo observation, but may be performed once when the operator is changed. Further, the process may be periodically performed in order to maintain the precision constant.

Subsequently, the alarm lamp controller 310c performs an alarm lamp emission process of emitting light from the alarm lamp 11a (step S374), and then transfers the alarm lamp 11a to the display corresponding area Am instead of to the outside observation area Ax (step S376).

Then, as in step S2 illustrated in FIG. 5, the alarm lamp controller 310c determines whether the magnetic field generator 5 generates the magnetic field (step S378) and repeats the determination process of step S378 until the magnetic field generator 5 generates the magnetic field. Then, when it is determined that the magnetic field generator 5 generates the magnetic field (step S378: Yes), the alarm lamp controller 310c transfers the alarm lamp 11a to the alarm position in the outside observation area Ax in order to alarm the generation of the magnetic field (step S380).

Next, the alarm lamp controller 310c determines whether the magnetic field generator 5 stops the generation of the magnetic field (step S382) and repeats the determination process of step S382. When it is determined that the magnetic field generator 5 stops the generation of the magnetic field (step S382: Yes), the control unit 310 determines whether the in-vivo observation is ended (step S384). When the control unit 310 determines that the in-vivo observation is not ended (step S384: No), the control unit 310 returns the current step to step S376 and moves the alarm lamp 11a to the display corresponding area Am in order to stop the alarm process. On the other hand, when the control unit 310 determines that the in-vivo observation is ended (step S384: Yes), the alarm lamp controller 310c performs an alarm lamp off process of turning off the alarm lamp 11a (step S386) and ends the process of controlling the alarm lamp 11a in accordance with the end of the in-vivo observation.

The in-vivo image acquiring system 301 is capable of changing the position of the alarm lamp 11a to notify that the magnetic field is generated or that the magnetic field is not generated by performing the respective process procedures illustrated in FIG. 21.

### First Modification of Third Embodiment

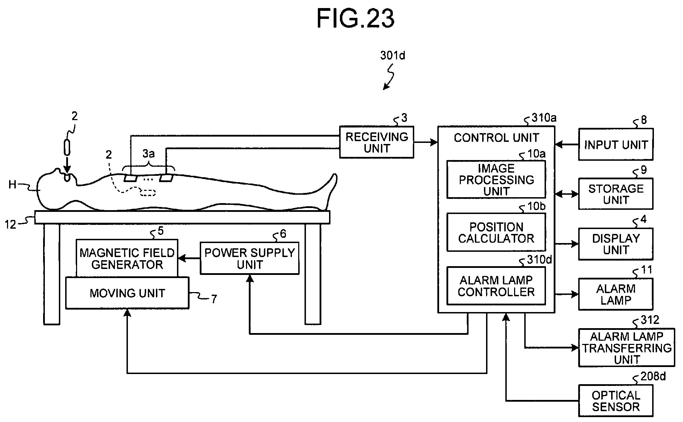

Next, a first modification of the third embodiment will be described. In the first modification of the third embodiment, the light emission state of the alarm lamp is detected by using the optical sensor, and the process of transferring the alarm lamp is performed on the basis of the detection result so that the alarm lamp is located inside the outside observation area. FIG. 23 is a block diagram schematically illustrating a configuration example of an in-vivo image acquiring system according to the first modification of the third embodiment.

As illustrated in FIG. 23, compared to the in-vivo image acquiring system 301 illustrated in FIG. 17, an in-vivo image acquiring system 301d according to the first modification of the third embodiment includes the input unit 8 instead of the input unit 308, and includes a control unit 310a instead of the control unit 310. The control unit 310a includes an alarm lamp controller 310d instead of the alarm lamp controller 310c. Then, the in-vivo image acquiring system 301d further includes the optical sensor 208d. The optical sensor 208d detects the light emission state of the alarm lamp. The alarm lamp controller 310d obtains the position of the alarm lamp 11a on the basis of the light emission state of the alarm lamp 11 detected by the optical sensor 208d, and controls the transfer process of the alarm lamp transferring unit 312 so that the alarm lamp 11a is located in the outside observation area Ax.

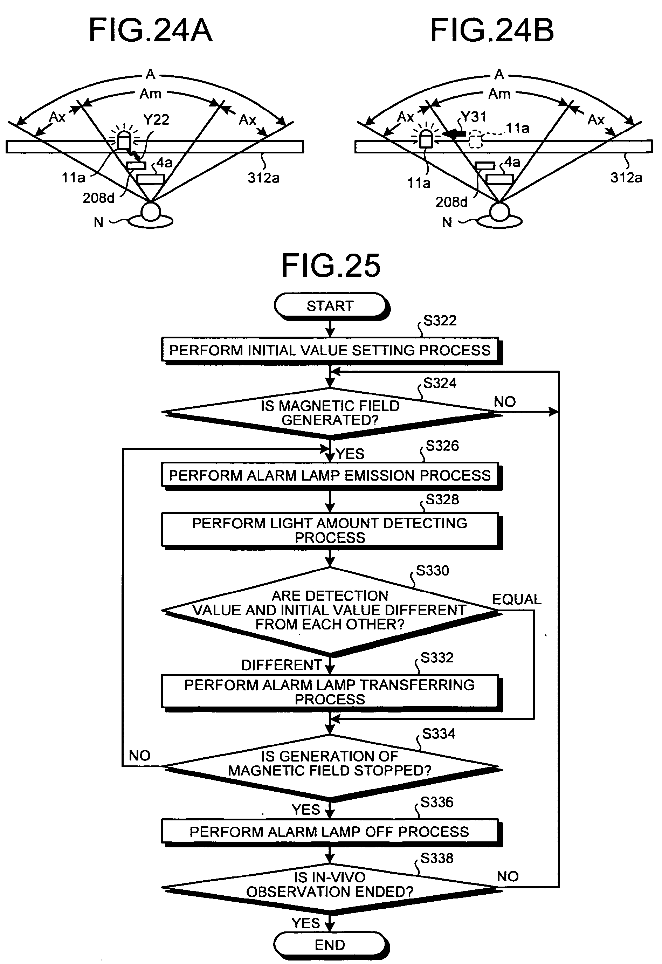

Specifically, referring to FIG. 24, the light emission control on the alarm lamp 11a in the in-vivo image acquiring system 301d illustrated in FIG. 23 will be described. As illustrated in FIG. 24(1), the optical sensor 208d is disposed, for example, near the display 4a in the display corresponding area Am. Then, as illustrated in FIG. 24(1), the optical sensor 208d detects the light amount of the light reaching the optical sensor 208d as depicted by the arrow Y22 as the light emission state of the alarm lamp 11a.

The alarm lamp controller 310d compares the light amount (initial value) when the light emitted from the alarm lamp 11a is restricted in the outside observation area Ax detected by the optical sensor 208d in advance and the light amount (detection value) detected by the optical sensor 208d.

When the initial value and the detection value are different from each other and the detection value becomes larger than the initial value, the illustrated alarm lamp 11a is located in the display corresponding area Am near the display 4a instead of in the outside observation area Ax as illustrated in FIG. 24(1). For this reason, the alarm lamp controller 310d controls the transfer mechanism 312a so that the alarm lamp 11a is located inside the outside observation area Ax by setting the transfer direction and the transfer distance of the alarm lamp 11a in accordance with the difference value between the initial value and the detection value. Accordingly, as illustrated in FIG. 24(2), the alarm lamp 11a is transferred into the outside observation area Ax as depicted by the arrow Y31. Further, the optical sensor 208d is not limited to be installed near the display 4a since it only needs to detect the light emission state of the alarm lamp 11a. Accordingly, the optical sensor may be installed near the operator N or may be directly attached to the head of the operator N.

In this manner, in the first modification of the third embodiment, since the position of the alarm lamp 11a is automatically controlled on the basis of the detection result of the optical sensor 208d so that the alarm lamp 11a is located inside the outside observation area Ax, the operator may concentrate on the in-vivo observation without performing the position adjusting process of the alarm lamp 11a.

Next, referring to FIG. 25, the magnetic field generation notifying process of the in-vivo image acquiring system 301d will be described. FIG. 25 is a flowchart illustrating a process procedure of a magnetic field generation notifying process of the in-vivo image acquiring system 301d illustrated in FIG. 23.

As illustrated in FIG. 25, in the in-vivo image acquiring system 301d, the alarm lamp 11a is located inside the outside observation area Ax under the control of the control unit 310a, the light amount in this case is detected by the optical sensor 208d, and the initial value setting process is performed to set the detected light amount as the initial value (step S322). The initial value setting process needs not to be performed for each in-vivo observation, but may be performed once when the operator is changed. Further, the process may be periodically performed in order to maintain the precision constant. Further, the alarm lamp controller 310d turns off the alarm lamp 11a after the initial value setting process.

Subsequently, as in step S2 illustrated in FIG. 5, the alarm lamp controller 310d determines whether the magnetic field generator 5 generates the magnetic field (step S324) and repeats the determination process of step S324 until the magnetic field generator 5 generates the magnetic field. Then, when it is determined that the magnetic field generator 5 generates the magnetic field (step S324: Yes), the alarm lamp controller 310d performs an alarm lamp emission process of emitting light from the alarm lamp 11a in the outside observation area Ax in order to alarm the generation of the magnetic field (step S326).

Then, the alarm lamp controller 310d performs a light amount detecting process on the optical sensor 208d so as to detect the light amount of the light reaching the optical sensor 208d (step S328). Then, the alarm lamp controller 310d determines whether the detection value and the initial value are different from each other by comparing the detection value and the initial value with each other (step S330). Furthermore, in step S330, the matching degree between the detection value and the initial value may be set by including variations in accordance with the detection precision of the optical sensor 208d, the posture of the operator N, the emission precision of the alarm lamp 11a, and the like.

When it is determined that the detection value and the initial value are different from each other (step S330: different), the alarm lamp controller 310d performs an alarm lamp transferring process of transferring the alarm lamp 11a using the alarm lamp transferring unit 312 by the distance and in the direction according to the difference value between the initial value and the detection value so that the alarm lamp 11a is located inside the outside observation area Ax (step S332). For example, in the alarm lamp transferring process, a table is stored in advance in which the distance and the direction of the alarm lamp 11a and the outside observation area Ax are correlated for each difference value between the initial value and the detection value, and the alarm lamp controller 310d sets the movement direction and the movement distance of the alarm lamp 11a using the transfer mechanism 312a by referring to the table. The transfer mechanism 312a transfers the alarm lamp 11a and locates the alarm lamp 11a at the outside observation area Ax under the control of the alarm lamp controller 310d.

When it is determined that the detection value and the initial value are substantially equal to each other (step S330: equal) or the alarm lamp transferring process of step S332 is ended, the alarm lamp controller 310d determines whether the magnetic field generator 5 stops the generation of the magnetic field (step S334). When it is determined that the magnetic field generator 5 does not stop the generation of the magnetic field (step S334: No), the alarm lamp controller 310d continues the alarm lamp emission process of step S326. On the other hand, when it is determined that the magnetic field generator 5 stops the generation of the magnetic field (step S334: Yes), the alarm lamp controller 310d performs an alarm lamp off process of turning off the alarm lamp 11a in order to stop the alarm process (step S336). Then, the control unit 310a determines whether the in-vivo observation is ended (step S338). When it is determined that the in-vivo observation is not ended (step S338: No), the current step returns to step S324. When it is determined that the in-vivo observation is ended (step S338: Yes), the process of controlling the alarm lamp 11a of the alarm lamp controller 310d is also ended in accordance with the end of the in-vivo observation.

The in-vivo image acquiring system 301a automatically controls the light emission process of the alarm lamp 11a so that the alarm lamp 11a is located inside the outside observation area Ax by performing the respective process procedures illustrated in FIG. 25.

### Second Modified Example of Third Embodiment

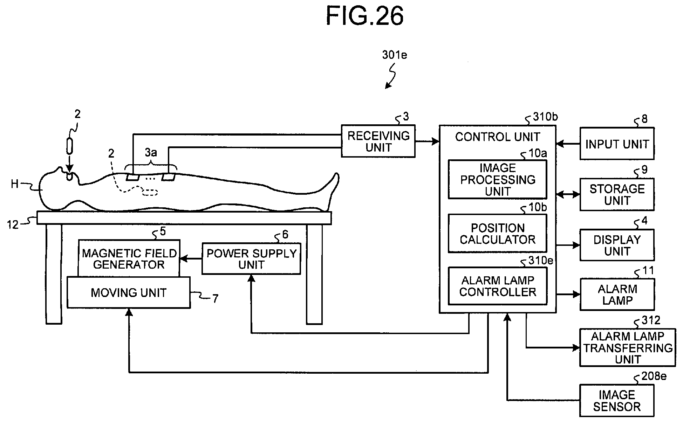

Next, a second modification of the third embodiment will be described. In the second modification of the third embodiment, the motions of the eyes of the operator is detected by using an image sensor instead of the optical sensor, a variation in distance between the eyes of the operator and the alarm lamp 11a is calculated on the basis of the detection result, and the light emission process of the alarm lamp 11a is controlled on the basis of the calculated distance variation. FIG. 26 is a block diagram schematically illustrating a configuration example of an in-vivo image acquiring system according to the second modification of the third embodiment.

As illustrated in FIG. 26, compared to the in-vivo image acquiring system 301d illustrated in FIG. 23, an in-vivo image acquiring system 301e according to the second modification of the third embodiment includes the image sensor 208e instead of the optical sensor 208d, and includes a control unit 310b instead of the control unit 310a. The control unit 310b includes an alarm lamp controller 310e instead of the alarm lamp controller 310d. The image sensor 208e detects the motions of the eyes of the operator. The alarm lamp controller 310d controls the position of the alarm lamp 11a on the basis of the detection result of the image sensor 208e.

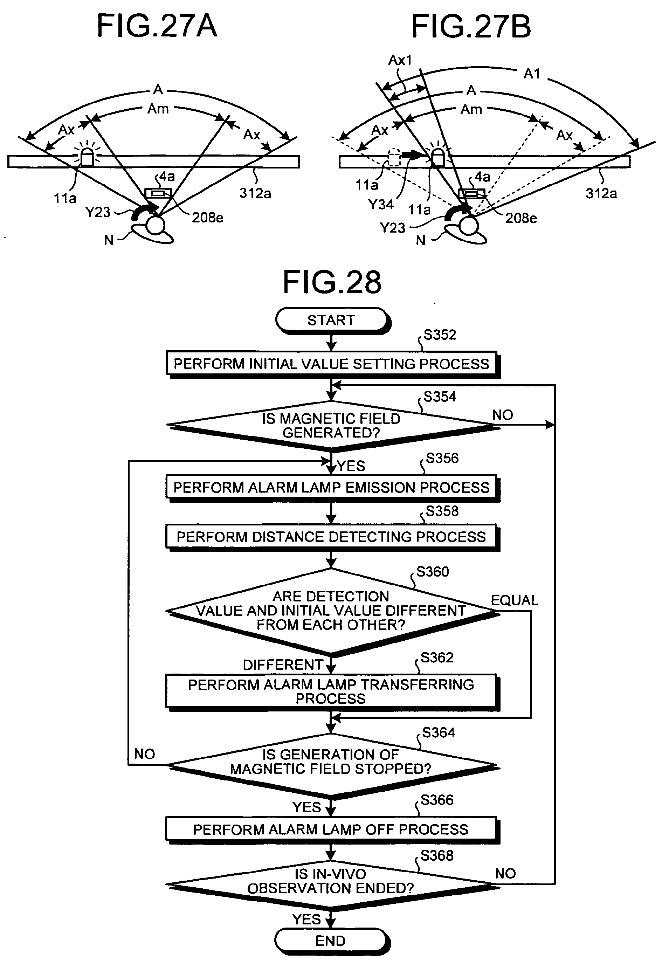

Specifically, referring to FIG. 27, the light emission control of the alarm lamp 11a of the in-vivo image acquiring system 301e illustrated in FIG. 26 will be described. As illustrated in FIG. 27(1), the image sensor 208e is located at the upper portion of the display 4a so that the eyes of the operator N are included in the imaging field. Then, the image sensor 208e detects the motions of the eyes of the operator N by continuously capturing the eyes of the operator N. The image sensor 208e outputs a series of captured images to the alarm lamp controller 310e.

The alarm lamp controller 310e calculates a variation in distance between the eyes of the operator N and the alarm lamp 11a by processing a series of captured images using the image sensor 208e as in the alarm lamp controller 210e illustrated in FIG. 14. For example, when the operator N faces the right side of the drawing as depicted by the arrow Y23 of FIG. 27(2), the eyes of the operator N also move to the right side of the drawing. Accordingly, the viewing area of the operator N also moves from the viewing area A to the viewing area A1 in the right direction of the drawing, and the distance between the eyes of the operator N and the alarm lamp 11a becomes larger than that when the operator N faces the right side of the drawing. In this case, the alarm lamp controller 310e transfers the alarm lamp 11a using the transfer mechanism 312a by the increasing distance between the eyes of the operator N and the alarm lamp 11a in accordance with the motion of the operator N. Accordingly, the alarm lamp 11a is transferred to the outside observation area Ax1, transferred in accordance with an operation of the operator N facing the right side, as depicted by the arrow Y34 of FIG. 27(2).

Furthermore since the image sensor 208e may be enough if it may detect the motions of the eyes of the operator N, the image sensor is not limited to be installed at the upper portion of the display 4a, and the image sensor 208e may be installed at an area between the display 4a and the operator N. Further, since the image sensor may be enough if it may detect the motions of the eyes of the operator N, the eyes of the operator N do not need to be captured. For example, the actual motions of the eyes of the operator N may be determined in a manner such that the image sensor 208e is attached to the head of the operator N, various motions of the eyes of the operator N are continuously captured in advance, and the image group corresponding to the respective motions of the eyes and captured in advance is compared with a series of images captured by the image sensor 208e attached to the head of the operator N during the in-vivo observation.

In this manner, in the second modification of the third embodiment, even when the viewing area moves by the motions of the eyes of the operator on the basis of the detection result of the image sensor 208e, the position of the alarm lamp 11a is automatically adjusted so that the light of the alarm lamp 11a is included in the outside observation area moving together with the viewing area. As a result, since the operator may visibly identify the alarm process using the alarm lamp 11a even when any motions of the eyes are performed, the generation of the magnetic field may be accurately detected.

Next, referring to FIG. 28, the magnetic field generation notifying process of the in-vivo image acquiring system 301e will be described. FIG. 28 is a flowchart illustrating a process procedure of a magnetic field generation notifying process of the in-vivo image acquiring system 301e illustrated in FIG. 26.

As illustrated in FIG. 28, in the in-vivo image acquiring system 301e, the transfer mechanism 312a adjusts the position of the alarm lamp 11a so that the alarm lamp 11a is located inside the outside observation area Ax of the operator N under the control of the control unit 310b, and the initial value setting process is performed so that a value obtained by adding the distance between the image sensor 208e and the alarm lamp 11a to the distance between the image sensor 208e and the eyes of the operator N during a basic operation of the operator N is set as the initial value (step S352). In the initial value setting process, for example, the basic operation time is set as the case where the operator N visually identifies the display 4a directly from the front surface thereof, the imaging process using the image sensor 208e is performed, and the distance between the image sensor 208e and the eyes of the operator N is obtained on the basis of the captured image using the image sensor 208e. Further, the initial value setting process needs not to be performed for each in-vivo observation, but may be performed once when the operator is changed. Further, the process may be periodically performed in order to maintain the precision constant. Further, the alarm lamp controller 310e turns off the alarm lamp 11a after the initial value setting process.

Subsequently, as in step S2 illustrated in FIG. 5, the alarm lamp controller 310e determines whether the magnetic field generator 5 generates the magnetic field (step S354) and repeats the determination process of step S354 until the magnetic field generator 5 generates the magnetic field. Then, when it is determined that the magnetic field generator 5 generates the magnetic field (step S354: Yes), the alarm lamp controller 310e performs an alarm lamp emission process of emitting light from the alarm lamp 11a in the outside observation area Ax in order to alarm the generation of the magnetic field (step S356).

Then, the alarm lamp controller 310e performs a distance detecting process of detecting the distance between the eyes of the operator and the alarm lamp 11a by capturing the eyes of the operator N using the image sensor 208e and processing a series of captured images using the image sensor 208e (step S358). In the distance detecting process, the distance between the eyes of the operator N and the image sensor 208e is calculated by processing a series of captured images using the image sensor 208e, and the distance between the eyes of the operator N and the alarm lamp 11a is detected by adding the calculated distance to the distance between the image sensor 208e and the alarm lamp 11a which are disposed in a fixed state.

Then, the alarm lamp controller 310e determines whether the detection value and the initial value are different from each other by comparing the detection value and the initial value with each other (step S360). Furthermore, in step S360, the matching degree between the detection value and the initial value may be set by including variations in accordance with the imaging precision of the image sensor 208e and the like.

When it is determined that the detection value and the initial value are different from each other (step S360: different), the alarm lamp controller 310e performs an alarm lamp transferring process of transferring the alarm lamp 11a using the alarm lamp transferring unit 312 by the distance and in the direction according to the difference value between the detection value and the initial value (step S362). For example, in the alarm lamp transferring process, a table is stored in advance in which the distance and the direction of the alarm lamp 11a and the outside observation area Ax are correlated for each difference value between the initial value and the detection value, and the alarm lamp controller 310e sets the transfer direction and the transfer distance of the alarm lamp 11a using the transfer mechanism 312a by referring to the table. The transfer mechanism 312a transfers the alarm lamp 11a, and locates the alarm lamp 11a at the outside observation area Ax under the control of the alarm lamp controller 310e.

When it is determined that the detection value and the initial value are substantially equal to each other (step S360: equal) or the alarm lamp transferring process of step S362 is ended, the alarm lamp controller 310e determines whether the magnetic field generator 5 stops the generation of the magnetic field (step S364). When it is determined that the magnetic field generator 5 does not stop the generation of the magnetic field (step S364: No), the alarm lamp controller 310e continues the alarm lamp emission process of step S356. On the other hand, when it is determined that the magnetic field generator 5 stops the generation of the magnetic field (step S364: Yes), the alarm lamp controller 310e performs an alarm lamp off process of turning off the alarm lamp 11a in order to stop the alarm process (step S366). Then, the control unit 310b determines whether the in-vivo observation is ended (step S368). When it is determined that the in-vivo observation is not ended (step S368: No), the current step returns to step S354. When it is determined that the in-vivo observation is ended (step S368: Yes), the process of controlling the alarm lamp 11a of the alarm lamp controller 310e is also ended in accordance with the end of the in-vivo observation.

The in-vivo image acquiring system 301e automatically controls the light emission process of the alarm lamp 11a so that the alarm lamp 11a is located inside the outside observation area Ax by performing the respective process procedures illustrated in FIG. 28.

### Fourth Embodiment

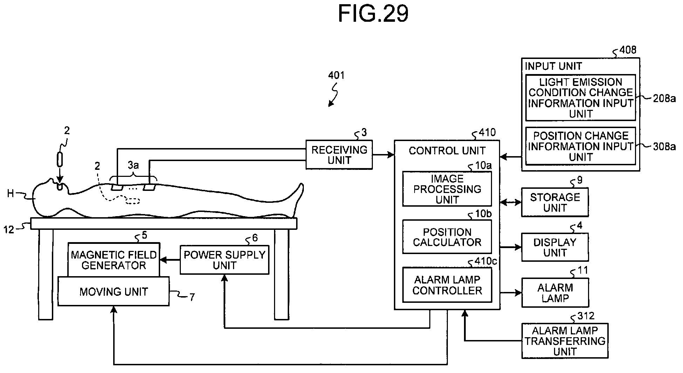

Next, a fourth embodiment will be described. In the fourth embodiment, the light emission control and the position control of the alarm lamp are both performed. FIG. 29 is a block diagram schematically illustrating a configuration example of an in-vivo image acquiring system according to the fourth embodiment.

As illustrated in FIG. 29, compared to the in-vivo image acquiring system 1 illustrated in FIG. 1, an in-vivo image acquiring system 401 according to the fourth embodiment includes an input unit 408 including the light emission condition change information input unit 208a and the position change information input unit 308a instead of the input unit 8, and includes a control unit 410 instead of the control unit 10. Then, the in-vivo image acquiring system 401 further includes the alarm lamp transferring unit 312 that transfers the alarm lamp 11. The position change information input unit 308a input instruction information for instructing a change of the position of the alarm lamp 11. Compared to the control unit 10, the control unit 410 includes an alarm lamp controller 410c instead of the alarm lamp controller 10c. The alarm lamp controller 410c controls the light emission process of the alarm lamp 11 on the basis of the instruction information input by the light emission condition change information input unit 208a, and performs a process of transferring the alarm lamp 11 on the basis of the instruction information input by the position change information input unit 308a.

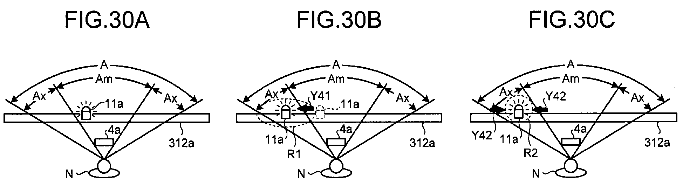

Specifically, referring to FIG. 30, the transfer control and the light emission control of the alarm lamp 11 of the in-vivo image acquiring system 401 illustrated in FIG. 29 will be described. As illustrated in FIG. 30(1), in the fourth embodiment, the single alarm lamp 11a is disposed as in FIG. 4. Then, as illustrated in FIG. 30(1), when the alarm lamp 11a is located at the display corresponding area Am instead of the outside observation area Ax, the operator N operates the position change information input unit 308a, and instructs that the position of the alarm lamp 11a is changed to the outside observation area Ax. As a result, instruction information for changing the position of the alarm lamp 11a to the outside observation area Ax is input from the position change information input unit 308a. In accordance with this instruction information, the alarm lamp controller 410c controls the transfer process of the transfer mechanism 312a so that the alarm lamp 11a is located inside the outside observation area Ax as illustrated in FIG. 30(2). Accordingly, the alarm lamp 11a is transferred into the outside observation area Ax as depicted by the arrow Y41.

Then, as illustrated in FIG. 30(2), when the light emission range of the alarm lamp 11a reaches the area R1 exceeding the outside observation area Ax, the operator N operates the light emission condition change information input unit 208a, and instructs that the light emission range of the alarm lamp 11a is narrowed to a desired range. As a result, the instruction information for changing the light emission condition of narrowing the light emission range of the alarm lamp 11a is input from the light emission condition change information input unit 208a. In accordance with this instruction information, the alarm lamp controller 310c controls so that the light intensity of the alarm lamp 11a decreases to include the light emitted from the alarm lamp 11a inside the outside observation area Ax. Accordingly, as illustrated in FIG. 30(3), the light emission range of the alarm lamp 11a is narrowed from the area R1 to the area R2 as depicted by the arrow Y42. Furthermore, the alarm lamp controller 410c may adjust not only the light intensity of the alarm lamp 11a, but also the emission color of the alarm lamp 11a so that the light emitted from the alarm lamp 11a is not included in the display area Am, but is included in the outside observation area Ax.

In this manner, in the fourth embodiment, the process of transferring the alarm lamp 11a is controlled so that the alarm lamp 11a is located inside the outside observation area Ax, and the light emission process of the alarm lamp 11a is controlled so that the light emitted from the alarm lamp 11a is included in the outside observation area Ax. Accordingly, the alarm lamp 11a is reliably located inside the outside observation area Ax, so that the light of the alarm lamp 11a may be prevented from being reflected on the screen of the display 4a and the influence of the light of the alarm lamp 11a with respect to the operator may be reduced.

Then, in the fourth embodiment, since the light emission control and the position control of the alarm lamp are both controlled, even when the operator is different, the light of the alarm lamp may be controlled so that it is accurately included in the outside observation area of the viewing area different for each operator.

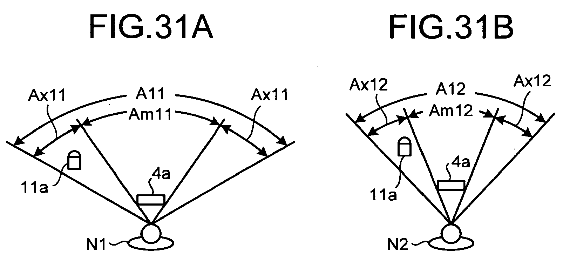

For example, the operator is changed from the operator N1 having the viewing area A11 illustrated in FIG. 31(1) to the operator N2 having the viewing area A12 illustrated in FIG. 31(2), the outside observation area Ax11 is changed to the outside observation area Ax12 in accordance with the change of the operator. Even in this case, the alarm lamp controller 410c may accurately include the light of the alarm lamp 11a inside the outside observation area of the operator N2 by changing the position of the alarm lamp 11a and the light intensity of the alarm lamp 11a in accordance with the instruction of the light emission condition change information input unit 208a and the position change information input unit 308a. In other words, the outside observation area where the alarm lamp 11a is located may be flexibly set for each operator. Information representing the viewing area state may be directly input.

Further, even in the first, second, or third embodiment, when the operator is changed from the operator N1 having the viewing area A11 illustrated in FIG. 31(1) to the operator N2 having the viewing area A12 illustrated in FIG. 31(2), the outside observation area where the alarm lamp 11a is located may be flexibly set in accordance with each operator. Information representing the viewing area state may be directly input.

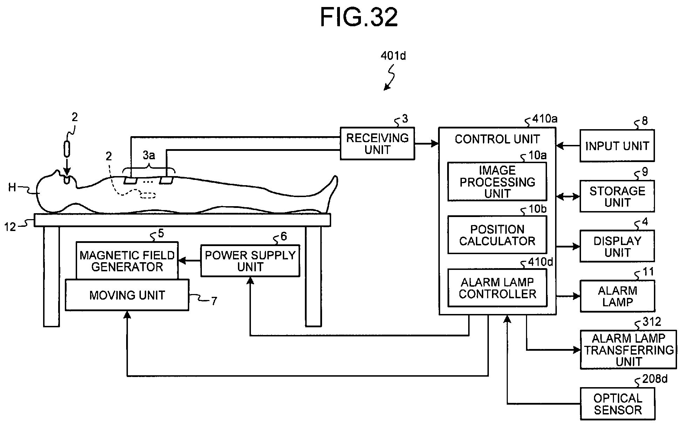

Furthermore, in the fourth embodiment, as in an in-vivo image acquiring system 401d illustrated in FIG. 32, the light emission state of the alarm lamp 11 and the position of the alarm lamp 11 may be detected by using the optical sensor 208d, the alarm lamp 11 may be transferred on the basis of the detection result so that the alarm lamp 11 is located inside the outside observation area, and the light emission process of the alarm lamp 11 may be controlled so that the light emitted from the alarm lamp 11 is included in the outside observation area. In this case, compared to the in-vivo image acquiring system 401 illustrated in FIG. 29, the in-vivo image acquiring system 401d includes the input unit 8 instead of the input unit 408, and includes a control unit 410a instead of the control unit 410. The control unit 410a includes an alarm lamp controller 410d instead of the alarm lamp controller 410c. Then, the in-vivo image acquiring system 401d further includes the optical sensor 208d. The alarm lamp controller 410d obtains the position of the alarm lamp 11a on the basis of the light emission state of the alarm lamp 11 detected by the optical sensor 208d, and controls the transfer process of the alarm lamp transferring unit 312 so that the alarm lamp 11a is located at the outside observation area Ax. Later, the alarm lamp controller 410d controls the light emission process of the alarm lamp 11 so that the light emitted from the alarm lamp 11 is included in the outside observation area on the basis of the light emission state of the alarm lamp 11 detected by the optical sensor 208d.

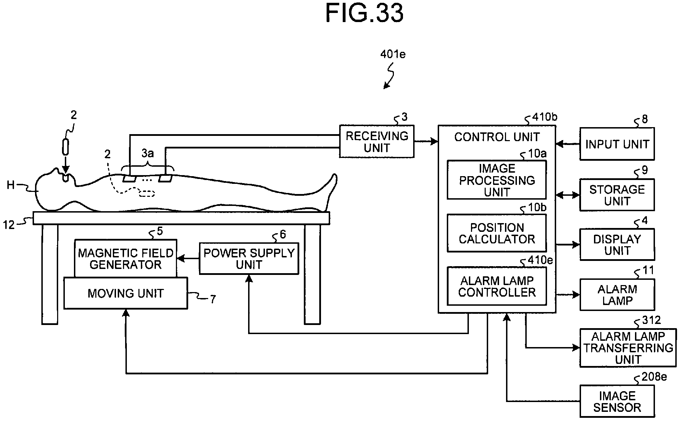

Further, in the fourth embodiment, as in an in-vivo image acquiring system 401e illustrated in FIG. 33, the motions of the eyes of the operator is detected by using the image sensor 208e, a variation in distance between the eyes of the operator and the alarm lamp 11 is calculated on the basis of the detection result, and the light emission process of the alarm lamp 11 and the process of transferring the alarm lamp 11 may be controlled on the basis of the calculated distance variation. In this case, compared to the in-vivo image acquiring system 401 illustrated in FIG. 29, the in-vivo image acquiring system 401e includes the input unit 8 instead of the input unit 408, and includes a control unit 410b instead of the control unit 410. The control unit 410b includes an alarm lamp controller 410e instead of the alarm lamp controller 410c. Then, the in-vivo image acquiring system 401e further includes an image sensor 208e. The alarm lamp controller 410e adjusts the position and the light intensity of the alarm lamp 11 on the basis of the detection result of the image sensor 208e by including the moved outside observation area when the outside observation area moves by the operation of the operator.

### Fifth Embodiment

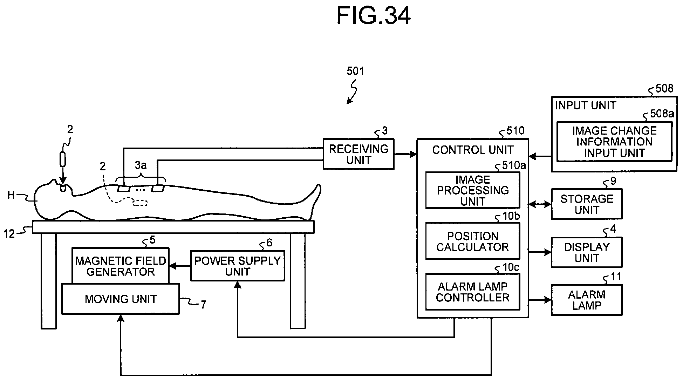

Next, a fifth embodiment will be described. In the fifth embodiment, the display color or the light intensity of the in-vivo image as the display parameter of the in-vivo image is controlled instead of controlling the alarm lamp, so that the operator may smoothly observe the subject information even when the light of the alarm lamp is reflected on the display screen. FIG. 34 is a block diagram schematically illustrating an in-vivo image acquiring system according to the fifth embodiment.

As illustrated in FIG. 34, compared to the in-vivo image acquiring system 1 illustrated in FIG. 1, an in-vivo image acquiring system 501 according to the fifth embodiment includes an input unit 508 including an image change information input unit 508a instead of the input unit 8, and includes a control unit 510 instead of the control unit 10. The image change information input unit 508a inputs a state where the light of the alarm lamp 11 is reflected on the display screen of the display unit 4. Compared to the control unit 10, the control unit 510 includes an image processing unit 510a instead of the image processing unit 10a. As in the image processing unit 10a, the image processing unit 510a creates the in-vivo image of the subject H, and changes the display color of the in-vivo image to the display color or the light intensity in accordance with the state where the light of the alarm lamp 11 is reflected on the display screen under the control of the control unit 510. The image processing unit 510a changes the display color of the in-vivo image on the basis of the state where the light of the alarm lamp 11 is reflected on the display screen input by the image change information input unit 508a.

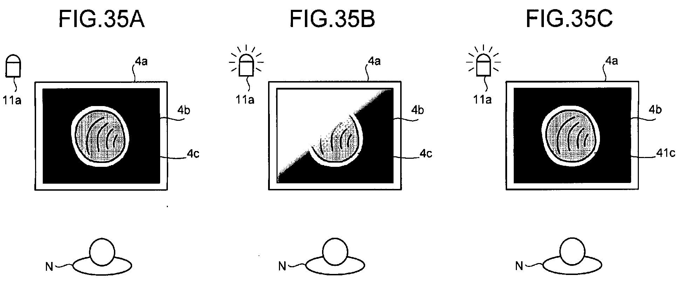

Next, referring to FIG. 35, the image processing of the in-vivo image acquiring system 501 will be described. In the fifth embodiment, the alarm lamp 11a is fixed to, for example, a predetermined position in the outside observation area.

When the alarm lamp 11a is turned on, as illustrated in FIG. 35(2), the light of the alarm lamp 11a is reflected on a display screen 4b of the display 4a, so that an in-vivo image 4c clearly displayed as illustrated in FIG. 35(1) is whitely blurred. In this case, the operator N operates the image change information input unit 508a, and instructs that the display color or the light intensity of the whitely blurred image is changed to the display color or the light intensity corresponding to the state where the light of the alarm lamp 11a is reflected on the display screen 4b.

The capsule endoscope 2 captures a reddish stomach wall or intestine wall as the in-vivo image. For this reason, for example, an instruction is generated to obtain an image in which the red light is suppressed by decreasing the reddish display ratio so that the white in-vivo image is darkened. As a result, instruction information for changing the display color of the in-vivo image is input from the image change information input unit 508a. In accordance with this instruction information, the image processing unit 510a performs image processing of changing the display color or the light intensity of the in-vivo image displayed on the display screen 4b, and displays the in-vivo image subjected to the image processing on the display unit 4. Accordingly, as illustrated in FIG. 35(3), an in-vivo image 41c in which red light is suppressed to handle the reflection of the light of the alarm lamp 11a is clearly displayed on the display screen 4b.

In this manner, in the fifth embodiment, the display parameter of the in-vivo image of the display subject is processed instead of the alarm lamp in accordance with the state where the light of the alarm lamp 11a is reflected on the display screen so that the operator who visually identifies the in-vivo image is less disturbed by the light emitted from the alarm lamp. Accordingly, even when the light of the alarm lamp 11a is reflected on the display screen, the operator N may smoothly observe the in-vivo image, and an influence of the light of the alarm lamp 11a with respect to the operator may be reduced.

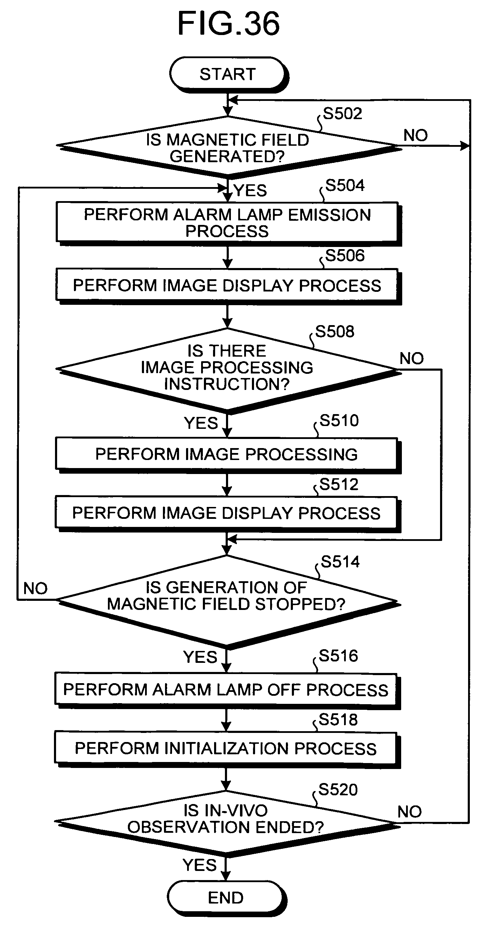

Next, referring to FIG. 36, the magnetic field generation notifying process of the in-vivo image acquiring system 501 will be described. FIG. 36 is a flowchart illustrating a process procedure of a magnetic field generation notifying process of the in-vivo image acquiring system 501 illustrated in FIG. 34.

As illustrated in FIG. 36, as in step S2 illustrated in FIG. 5, the alarm lamp controller 10c determines whether the magnetic field generator 5 generates the magnetic field (step S502) and repeats the determination process of step S502 until the magnetic field generator 5 generates the magnetic field. Then, when it is determined that the magnetic field generator 5 generates the magnetic field (step S502: Yes), the alarm lamp controller 10c performs an alarm lamp emission process of emitting light from the alarm lamp 11a in the outside observation area Ax in order to alarm the generation of the magnetic field (step S504). Then, the image processing unit 510a performs an image display process of processing the in-vivo image transmitted from the capsule endoscope 2 to be displayed on the display unit 4 (step S506).

Subsequently, the image processing unit 510a determines whether there is an instruction for the image processing with respect to the in-vivo image on the basis of the presence of the instruction information from the image change information input unit 508a (step S508). In this case, the image change information input unit 508a may input information representing the state where the light of the alarm lamp 11 is reflected on the display screen. Further, the image change information input unit 508a may input the range where the light of the alarm lamp 11 is reflected on the display screen.

When it is determined that there is an instruction for the image processing with respect to the in-vivo image (step S508: Yes), the image processing unit 510a performs image processing of changing the display color of the in-vivo image to the display color or the light intensity corresponding to the state where the light of the alarm lamp 11 is reflected on the display screen in accordance with the instruction (step S510), and performs an image display process of displaying the in-vivo image subjected to the image processing on the display unit 4 (step S512).

As described above, in the image processing, the image processing unit 510a may perform a process to obtain an image in which the red display ratio is decreased to suppress the red light so that the whitely blurred in-vivo image is darkened. Further, the image processing unit 510a may sequentially display an image in which the red light ratio is gradually decreased, and may perform image processing on the subsequent in-vivo image at the selected red light ratio when the image change information input unit 508a inputs information for selecting the red light ratio. Further, the reflection range of the light on the display screen for each light intensity of the alarm lamp 11 is obtained and stored in advance, the red light ratio capable of handling the reflection of the light of the alarm lamp 11a for each region is stored, and when the image change information input unit 508a inputs the reflection range of the light of the alarm lamp 11 on the display screen, image processing may be performed so that only the region displayed in the reflection range in the in-vivo image has the corresponding red light ratio on the basis of each red light ratio and each reflection range. Further, as the image processing, a case has been described in which a process is performed to obtain an image having suppressed red light, but the invention is not limited thereto. The image processing unit 510a may change the display color or the light intensity of the in-vivo image in accordance with the light intensity or the emission color of the alarm lamp 11.

When it is determined that there is no instruction for the image processing (step S508: No) or the image display process of step S512 is ended, the alarm lamp controller 10c determines whether the magnetic field generator 5 stops the generation of the magnetic field (step S514). When it is determined that the magnetic field generator 5 does not stop the generation of the magnetic field (step S514: No), the alarm lamp controller 10c returns the current step to step S504 so as to continue the alarm lamp emission process. On the other hand, when it is determined that the magnetic field generator 5 stops the generation of the magnetic field (step S514: Yes), the alarm lamp controller 10c performs an alarm lamp off process of turning off the alarm lamp 11a in order to stop the alarm process (step S516). Subsequently, the image processing unit 510a initializes the image processing setting (step S518). Then, the control unit 510 determines whether the in-vivo observation is ended (step S520). When it is determined that the in-vivo observation is not ended (step S520: No), the current step returns to step S502. When it is determined that the in-vivo observation is ended (step S520: Yes), the process of controlling the alarm lamp 11a in the alarm lamp controller 10c is also ended in accordance with the end of the in-vivo observation.

The in-vivo image acquiring system 501 changes the display color of the in-vivo image to the display color in accordance with the state where the light of the alarm lamp 11 is reflected on the display screen, accurately notifies the operator of the generation of the magnetic field, and allows the operator to smoothly observe the subject information by performing the respective process procedures illustrated in FIG. 36.

### First Modified Example of Fifth Embodiment

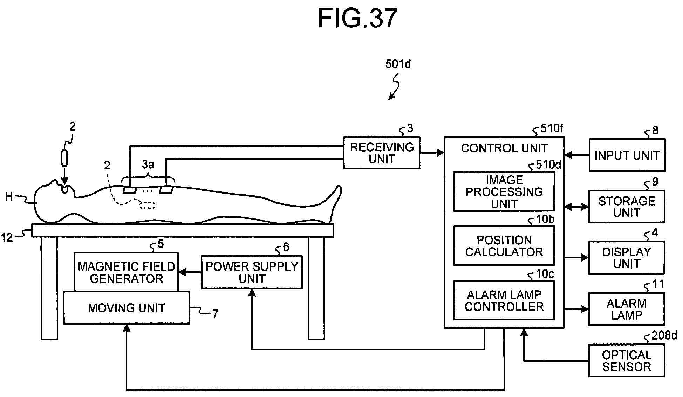

Next, a first modification of the fifth embodiment will be described. In the first modification of the fifth embodiment, the state where the light of the alarm lamp is reflected on the display screen is detected by using the optical sensor, and the display color of the in-vivo image is changed on the basis of the detection result. FIG. 37 is a block diagram schematically illustrating a configuration example of an in-vivo image acquiring system according to the first modification of the fifth embodiment.

As illustrated in FIG. 37, compared to the in-vivo image acquiring system 501 illustrated in FIG. 34, an in-vivo image acquiring system 501d according to the first modification of the fifth embodiment includes the input unit 8 instead of the input unit 508, and includes a control unit 510f instead of the control unit 510. The control unit 510f includes an image processing unit 510d instead of the image processing unit 510a. Then, the in-vivo image acquiring system 501d further includes the optical sensor 208d. The optical sensor 208d detects the state where the light of the alarm lamp 11 is reflected on the display screen. The image processing unit 510d changes the display color or the light intensity of the in-vivo image on the basis of the detection result of the optical sensor 208d.

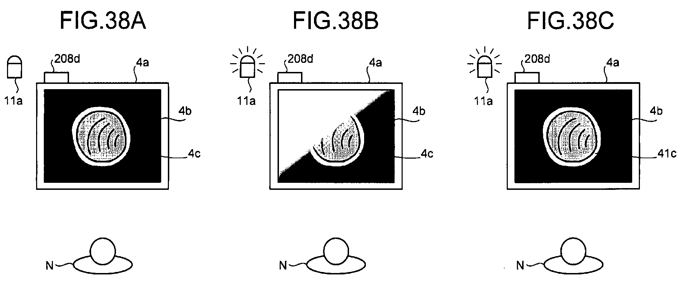

Specifically, referring to FIG. 38, the image processing with respect to the in-vivo image in the in-vivo image acquiring system 501d illustrated in FIG. 37 will be described. As illustrated in FIG. 38(1), the optical sensor 208d is disposed at, for example, the upper portion of the display 4a. Then, as illustrated in FIG. 38(2), when the alarm lamp 11a emits light, the optical sensor 208d detects the light amount of the light of the alarm lamp 11a reaching the optical sensor 208d.

The image processing unit 510d compares the light amount (detection value) detected by the optical sensor 208d with the light amount (initial value) of the alarm lamp 11a detected in advance by the optical sensor 208d when the light is not reflected on the display screen 4b.

When the initial value and the detection value are different from each other, so that the detection value becomes larger than the initial value, as illustrated in FIG. 38(2), it corresponds to the case where the light of the alarm lamp 11a is reflected on the display screen 4b of the display 4a. As a result, the in-vivo image 4c is whitely blurred as illustrated in FIG. 38(2). For this reason, the image processing unit 510d performs image processing of changing the display color of the in-vivo image in accordance with a difference value between the initial value and the detection value, and displays the in-vivo image subjected to the image processing on the display unit 4. Accordingly, as illustrated in FIG. 38(3), the display screen 4b clearly displays the in-vivo image 41c in which the red light is suppressed to handle the reflection of the light of the alarm lamp 11a. Further, the optical sensor 208d is not limited to be installed near the display 4a since it may detect only the light emission state of the alarm lamp 11a. Accordingly, the optical sensor may be installed near the operator N or may be directly attached to the head of the operator N.

In this manner, in the first modification of the fifth embodiment, since the display color of the in-vivo image of the display subject is automatically changed on the basis of the detection result of the optical sensor 208d in accordance with the state where the light of the alarm lamp 11a is reflected on the display screen, the operator may concentrate on the in-vivo observation without changing the display color or the light intensity.

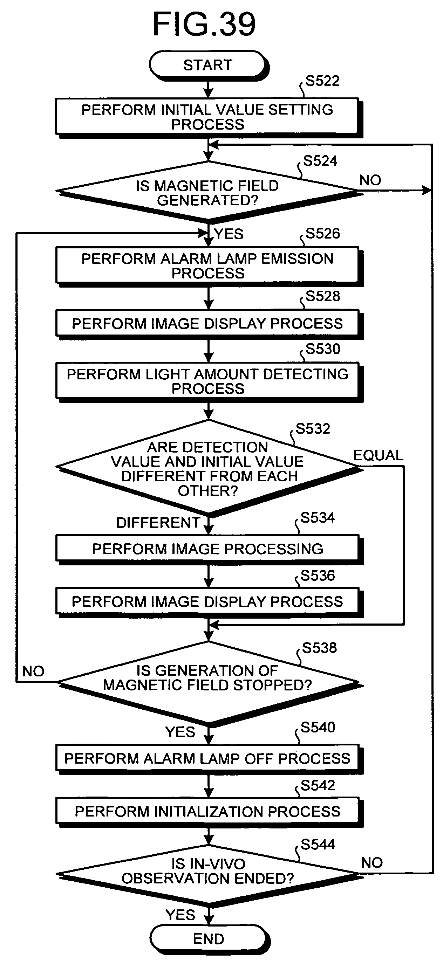

Next, referring to FIG. 39, the magnetic field generation notifying process of the in-vivo image acquiring system 501d will be described. FIG. 39 is a flowchart illustrating a process procedure of a magnetic field generation notifying process of the in-vivo image acquiring system 501d illustrated in FIG. 37.

As illustrated in FIG. 39, in the in-vivo image acquiring system 501d, the light amount of the alarm lamp 11a inside the outside observation area Ax is adjusted so that the light of the alarm lamp 11a is not reflected on the display screen of the display unit 4 under the control of the control unit 510f, the light amount in this case is detected by the optical sensor 208d, and the initial value setting process is performed to set the detected light amount as the initial value (step S522). The initial value setting process needs not to be performed for each in-vivo observation, but may be performed once when the operator is changed. Further, the process may be periodically performed in order to maintain the precision constant. Further, the alarm lamp controller 10c turns off the alarm lamp 11a after the initial value setting process.

Subsequently, as in step S2 illustrated in FIG. 5, the alarm lamp controller 10c determines whether the magnetic field generator 5 generates the magnetic field (step S524) and repeats the determination process of step S524 until the magnetic field generator 5 generates the magnetic field. Then, when it is determined that the magnetic field generator 5 generates the magnetic field (step S524: Yes), the alarm lamp controller 10c performs an alarm lamp emission process of emitting light from the alarm lamp 11a in the outside observation area Ax in order to alarm the generation of the magnetic field (step S526). Then, the image processing unit 510d performs an image display process of displaying the in-vivo image transmitted from the capsule endoscope 2 on the display unit 4 (step S528).

Then, the control unit 510f performs a light amount detecting process on the optical sensor 208d so as to detect the light amount of the light reaching the optical sensor 208d (step S530). Subsequently, the image processing unit 510d determines whether the detection value and the initial value are different from each other by comparing the detection value and the initial value with each other (step S532). Furthermore, in step S532, the matching degree between the detection value and the initial value may be set by including variations in accordance with the detection precision of the optical sensor 208d, the posture of the operator N, the emission precision of the alarm lamp 11a, and the like.

When it is determined that the detection value and the initial value are different from each other (step S532: different), the image processing unit 510d performs image processing of changing the display color or the light intensity of the in-vivo image in accordance with a difference value between the initial value and the detection value so as to handle the reflection of the light of the alarm lamp 11a (step S534), and performs an image display process of the in-vivo image subjected to the image processing on the display unit 4 (step S536). For example, in the light emission condition adjusting process, a table is stored in advance in which the appropriate red light ratio and the reflection range of the light on the display screen are correlated for each difference value between the initial value and the detection value, and the image processing unit 510d processes the in-vivo image by using the red light ratio in the process subject range and the process subject range of the in-vivo image with reference to the table. As a result, the display screen 4b clearly displays the in-vivo image 41c in which the red light is suppressed to handle the reflection of the light of the alarm lamp 11a.

When the image processing unit 510d determines that the detection value and the initial value are substantially equal to each other (step S532: equal) or the image display process of step S536 is ended, the control unit 510f determines whether the magnetic field generator 5 stops the generation of the magnetic field (step S538). When it is determined that the magnetic field generator 5 does not stop the generation of the magnetic field (step S538: No), the control unit 510f continues the alarm lamp emission process of step S526. On the other hand, when it is determined that the magnetic field generator 5 stops the generation of the magnetic field (step S538: Yes), the control unit 510f performs an alarm lamp off process of turning off the alarm lamp 11a in order to stop the alarm process (step S540). Subsequently, the image processing unit 510d initializes the image processing setting (step S542). Then, the control unit 510f determines whether the in-vivo observation is ended (step S544). When it is determined that the in-vivo observation is not ended (step S544: No), the current step returns to step S524. When it is determined that the in-vivo observation is ended (step S544: Yes), the process of controlling the alarm lamp 11a in the alarm lamp controller 10c is also ended in accordance with the end of the in-vivo observation.

The in-vivo image acquiring system 501d automatically changes the display color or the light intensity of the in-vivo image to the display color or the light intensity in accordance with the state where the light of the alarm lamp 11 is reflected to the display screen by performing the respective process procedures illustrated in FIG. 39.

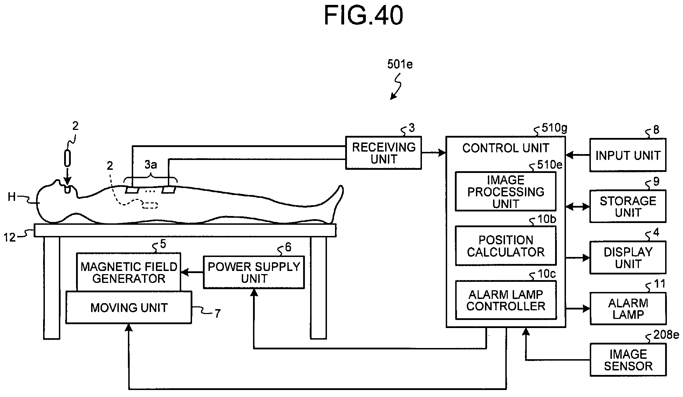

The second modification of the fifth embodiment Next, the second modification of the fifth embodiment will be described. In the second modification of the fifth embodiment, the state where the light of the alarm lamp is reflected on the display screen by using the image sensor instead of the optical sensor, and the display color of the in-vivo image is changed on the basis of the detection result. FIG. 40 is a block diagram schematically illustrating a configuration example of an in-vivo image acquiring system according to the second modification of the fifth embodiment.

As illustrated in FIG. 40, compared to the in-vivo image acquiring system 501d illustrated in FIG. 37, an in-vivo image acquiring system 501e according to the second modification of the fifth embodiment includes an image sensor 208e instead of the optical sensor 208d, and includes a control unit 510g instead of the control unit 510f. The control unit 510g includes an image processing unit 510e instead of the image processing unit 510d. The image sensor 208e detects the state where the light of the alarm lamp 11 is reflected on the display screen by capturing the display screen of the display unit 4. The image processing unit 510e changes the display color or the light intensity of the in-vivo image on the basis of the detection result of the image sensor 208e.

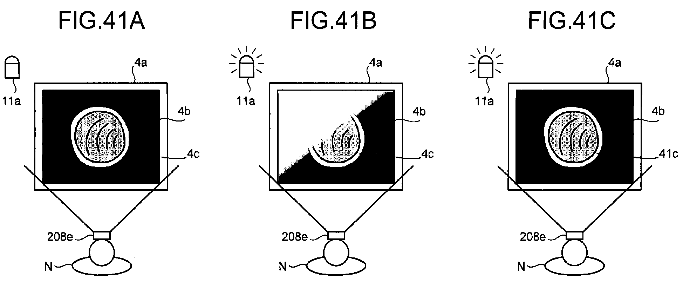

Specifically, referring to FIG. 41, the image processing with respect to the in-vivo image of the in-vivo image acquiring system 501e illustrated in FIG. 40 will be described. As illustrated in FIG. 41(1), the image sensor 208e is attached to, for example, the head of the operator N so that the display screen 4b of the display 4a is included in the imaging field. Then, when the alarm lamp 11a emits light as illustrated in FIG. 41(2), the image sensor 208e detects the state where the light of the alarm lamp 11a is reflected on the display screen 4b by continuously capturing the display screen 4b. The image sensor 208e outputs a series of captured images to the image processing unit 510e.

The image processing unit 510e detects the state where the light of the alarm lamp 11a is reflected on the display screen by processing a series of captured images using the image sensor 208e. For example, the image processing unit 510e compares the area (detection value) of the white region of the image of the display screen 4b captured by the image sensor 208e with the area (initial value) of the white region of the image of the display screen 4b displaying the in-vivo image captured by in advance by the image sensor 208e when there is no reflection of light.

When the initial value and the detection value are different from each other, so that the detection value becomes larger than the initial value, as illustrated in FIG. 41(2), it corresponds to the case where the light of the alarm lamp 11a is reflected on the display screen 4b of the display 4a. As a result, the in-vivo image 4c is whitely blurred as illustrated in FIG. 41(2). For this reason, the image processing unit 510e performs image processing of changing the display color of the in-vivo image in accordance with a difference value between the initial value and the detection value, and displays the in-vivo image subjected to the image processing on the display unit 4. Accordingly, as illustrated in FIG. 41(3), the display screen 4b clearly displays the in-vivo image 41c in which the red light is suppressed to handle the reflection of the light of the alarm lamp 11a. Further, the image sensor 208e is not limited to be installed at the head of the operator N since it may capture the display screen of the display unit 4, and may be disposed between the operator N and the display unit 4 so that the display screen 4b of the display 4a is included in the imaging field.

In this manner, in the second modification of the fifth embodiment, since the display color of the in-vivo image of the display subject is automatically changed on the basis of the detection result of the image sensor 208e in accordance with the state where the light of the alarm lamp 11a is reflected on the display screen, the operator may concentrate on the in-vivo observation without changing the display color or the light intensity.

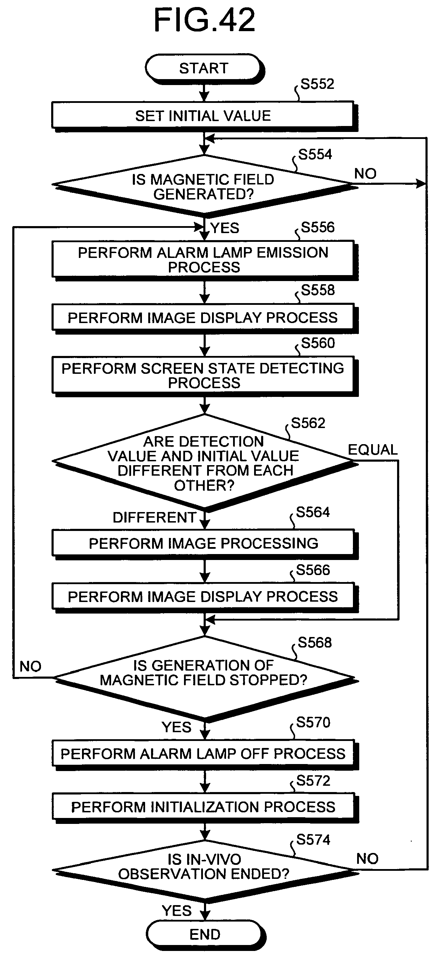

Next, referring to FIG. 42, the magnetic field generation notifying process of the in-vivo image acquiring system 501e will be described. FIG. 42 is a flowchart illustrating a process procedure of a magnetic field generation notifying process of the in-vivo image acquiring system 501e illustrated in FIG. 40.

As illustrated in FIG. 42, in the in-vivo image acquiring system 501e, the light amount of the alarm lamp 11a inside the outside observation area Ax is adjusted so that the light of the alarm lamp 11a is not reflected on the display screen of the display unit 4 under the control of the control unit 510g, the display screen in this case is captured by the image sensor 208e, and the initial value setting process is performed so as to set the area of the white region of the captured display screen image as the initial value (step S552). The initial value setting process needs not to be performed for each in-vivo observation, but may be performed once when the operator is changed. Further, the process may be periodically performed in order to maintain the precision constant. Further, the alarm lamp controller 10c turns off the alarm lamp 11a after the initial value setting process. Subsequently, as in step S2 illustrated in FIG. 5, the alarm lamp controller 10c determines whether the magnetic field generator 5 generates the magnetic field (step S554) and repeats the process of step S554 until the magnetic field generator 5 generates the magnetic field. Then, when it is determined that the magnetic field generator 5 generates the magnetic field (step S554: Yes), the alarm lamp controller 10c performs an alarm lamp emission process of emitting light from the alarm lamp 11a in the outside observation area Ax in order to alarm the generation of the magnetic field (step S556). Then, the image processing unit 510e performs an image display process of displaying the in-vivo image transmitted from the capsule endoscope 2 on the display unit 4 (step S558).

Then, the image processing unit 510e performs a screen state detecting process of calculating the area of the white region of the captured display screen image as the detection value by capturing the display screen of the display unit 4 using the image sensor 208e (step S560). Subsequently, the image processing unit 510e determines whether the detection value and the initial value are different from each other by comparing the detection value and the initial value with each other (step S562). Furthermore, in step S560, the matching degree between the detection value and the initial value may be set by including variations in accordance with the detection precision of the image sensor 208e, the posture of the operator N, the emission precision of the alarm lamp 11a, and the like.

When it is determined that the detection value and the initial value are different from each other (step S562: different), the image processing unit 510e performs image processing of changing the display color or the light intensity of the in-vivo image in accordance with a difference value between the initial value and the detection value so as to handle the reflection of the light of the alarm lamp 11a (step S564), and performs an image display process of displaying the in-vivo image subjected to the image processing on the display unit 4 (step S566). For example, in the light emission condition adjusting process, a table is stored in advance in which the appropriate red light ratio and the reflection range of the light on the display screen are correlated for each difference value between the initial value and the detection value, and the image processing unit 510e processes the in-vivo image by using the red light ratio of the process subject range and the process subject range of the in-vivo image with reference to the table. As a result, the display screen 4b clearly displays the in-vivo image 41c in which the red light is suppressed to handle the reflection of the light of the alarm lamp 11a.

When the image processing unit 510e determines that the detection value and the initial value are substantially equal to each other (step S562: equal) or the image display process of step S566 is ended, the control unit 510g determines whether the magnetic field generator 5 stops the generation of the magnetic field (step S568). When it is determined that the magnetic field generator 5 does not stop the generation of the magnetic field (step S568: No), the control unit 510g continues the alarm lamp emission process of step S556. On the other hand, when it is determined that the magnetic field generator 5 stops the generation of the magnetic field (step S568: Yes), the control unit 510g performs an alarm lamp off process of turning off the alarm lamp 11a in order to stop the alarm process (step S570). Subsequently, the image processing unit 510e initializes the image processing setting (step S572). Then, the control unit 510g determines whether the in-vivo observation is ended (step S574). When it is determined that the in-vivo observation is not ended (step S574: No), the current step returns to step S554. When it is determined that the in-vivo observation is ended (step S574: Yes), the process of controlling the alarm lamp 11a in the alarm lamp controller 10c is also ended in accordance with the end of the in-vivo observation.

The in-vivo image acquiring system 501e automatically changes the display color of the in-vivo image to the display color or the light intensity in accordance with the state where the light of the alarm lamp 11 is reflected on the display screen by performing the respective process procedures illustrated in FIG. 42.

### Sixth Embodiment

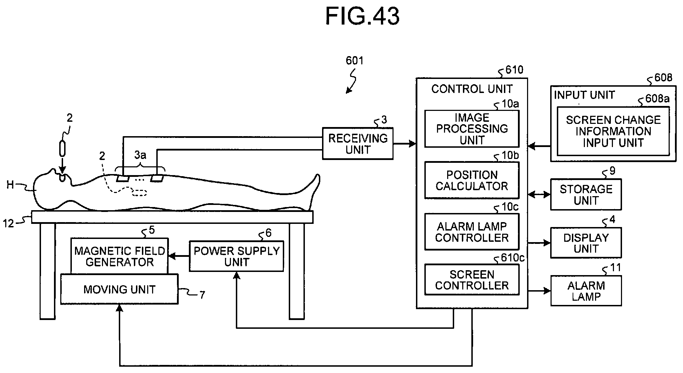

Next, the sixth embodiment will be described. In the sixth embodiment, the display screen of the display unit 4 is controlled, so that the operator may smoothly observe the subject information even when the light of the alarm lamp is reflected on the display screen. FIG. 43 is a block diagram schematically illustrating an in-vivo image acquiring system according to the sixth embodiment.

As illustrated in FIG. 43, compared to the in-vivo image acquiring system 1 illustrated in FIG. 1, an in-vivo image acquiring system 601 according to the sixth embodiment includes an input unit 608 including a screen change information input unit 608a instead of the input unit 8, and includes a control unit 610 instead of the control unit 10. The screen change information input unit 608a inputs the state where the light of the alarm lamp 11 is reflected on the display screen of the display unit 4. The control unit 610 further includes a screen controller 610c compared to the control unit 10. The screen controller 610c changes the display parameter of the display screen of the display unit 4 so as to correspond to the state where the light of the alarm lamp 11 is reflected on the display screen. The screen controller 610c changes the display parameter of the display screen of the display unit 4, for example, the display color or the light intensity of the display screen on the basis of the state where the light of the alarm lamp 11 is reflected on the display screen which is input from the screen change information input unit 608a.

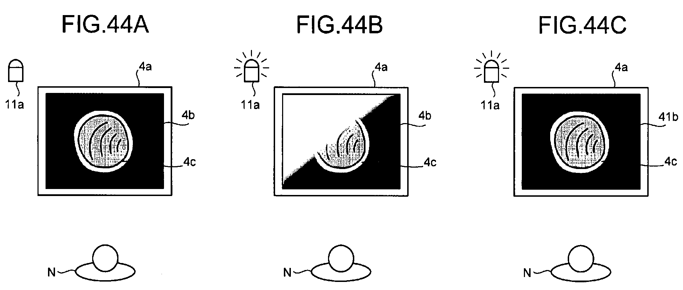

Next, referring to FIG. 44, the screen control process of the in-vivo image acquiring system 601 will be described. In the sixth embodiment, the alarm lamp 11a is fixed to, for example, a predetermined position in the outside observation area.

When the alarm lamp 11a is turned on, as illustrated in FIG. 44(2), the light of the alarm lamp 11a is reflected on the display screen 4b of the display 4a, so that the in-vivo image 4c clearly displayed as illustrated in FIG. 44(1) is whitely blurred. In this case, the operator N operates the screen change information input unit 608a, and instructs that the display color or the light intensity of the region on which the light of the alarm lamp 11a is reflected in the display screen 4b is changed. For example, the display color or the light intensity of the region is changed so that the region to which the light of the alarm lamp 11a is reflected is darkened. As a result, instruction information for changing the display color or the light intensity of the display screen is input from the screen change information input unit 608a. In accordance with this instruction information, the screen controller 610c performs a screen change process of changing the display color or the light intensity of the region on which the light of the alarm lamp 11a is reflected in the display screen 4b. Accordingly, as illustrated in FIG. 44(3), the region on which the light of the alarm lamp 11a is reflected becomes a display screen 41b which is darkened to be substantially similar to the other regions.

In this manner, in the sixth embodiment, the display parameter of the display screen is changed in accordance with the state where the light of the alarm lamp 11a is reflected on the display screen so that the operator who visually identifies the in-vivo image is less disturbed by the light emitted from the alarm lamp. Accordingly, even when the light of the alarm lamp 11a is reflected on the display screen, the operator N may smoothly observe the in-vivo image, so that the influence of the light of the alarm lamp 11a with respect to the operator is reduced.

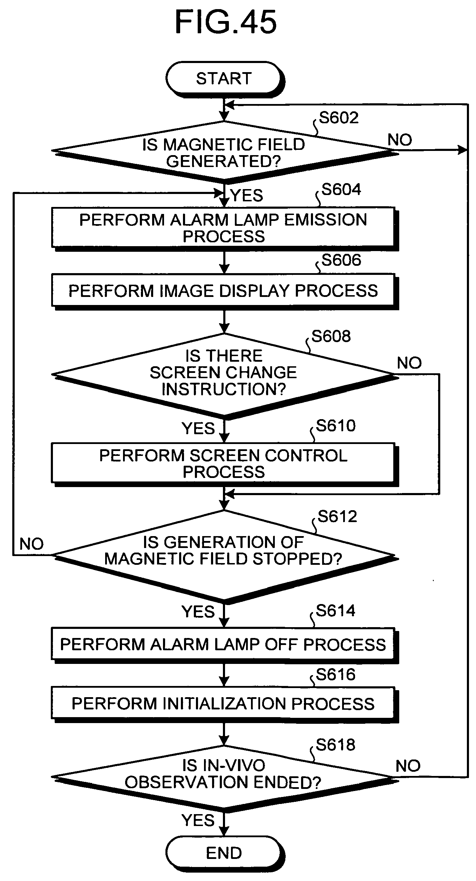

Next, referring to FIG. 45, the magnetic field generation notifying process of the in-vivo image acquiring system 601 will be described. FIG. 45 is a flowchart illustrating a process procedure of a magnetic field generation notifying process of the in-vivo image acquiring system 601 illustrated in FIG. 43.

As illustrated in FIG. 45, as in step S2 illustrated in FIG. 5, the alarm lamp controller 10c determines whether the magnetic field generator 5 generates the magnetic field (step S602) and repeats the determination process of step S602 until the magnetic field generator 5 generates the magnetic field. Then, when it is determined that the magnetic field generator 5 generates the magnetic field (step S602: Yes), the alarm lamp controller 10c performs an alarm lamp emission process of emitting light from the alarm lamp 11a in the outside observation area Ax in order to alarm the generation of the magnetic field (step S604). Then, the image processing unit 10a performs an image display process of processing the in-vivo image transmitted from the capsule endoscope 2 to be displayed on the display unit 4 (step S606).

Subsequently, the screen controller 610c determines whether there is an instruction for changing the display parameter of the display screen on the basis of the presence of the instruction information from the screen change information input unit 608a (step S608). In this case, the screen change information input unit 608a may input information representing a state where the light of the alarm lamp 11 is reflected on the display screen. Further, the screen change information input unit 608a may input the reflection range of the display screen of the light using the alarm lamp 11.

When it is determined that there is an instruction for changing the display color parameter of the display screen (step S608: Yes), the screen controller 610c performs a screen control process of changing the display color or the light intensity of the display screen so as to correspond to the state where the light of the alarm lamp 11 is reflected on the display screen in accordance with the instruction (step S610). The screen controller 610c controls the display color or the light intensity of the display screen of the region so that the region on which the light of the alarm lamp 11a is reflected is darkened.

When it is determined that there is no screen change instruction (step S608: No) or the screen control process of step S610 is ended, the alarm lamp controller 10c determines whether the magnetic field generator 5 stops the generation of the magnetic field (step S612). When it is determined that the magnetic field generator 5 does not stop the generation of the magnetic field (step S612: No), the alarm lamp controller 10c returns the current step to step S604 so as to continue the alarm lamp emission process. On the other hand, when it is determined that the magnetic field generator 5 stops the generation of the magnetic field (step S612: Yes), the alarm lamp controller 10c performs an alarm lamp off process of turning off the alarm lamp 11a in order to stop the alarm process (step S614). Subsequently, the screen controller 610c initializes the screen display setting (step S616). Then, the control unit 610 determines whether the in-vivo observation is ended (step S618). When it is determined that the in-vivo observation is not ended (step S618: No), the current step returns to step S602. When it is determined that the in-vivo observation is ended (step S618: Yes), the process of controlling the alarm lamp 11a in the alarm lamp controller 10c is also ended in accordance with the end of the in-vivo observation.

The in-vivo image acquiring system 601 changes the display parameter of the display screen in accordance with the state where the light of the alarm lamp 11 is reflected on the display screen by performing the respective process procedures illustrated in FIG. 45. Accordingly, it is possible to accurately notify the operator of the generation of the magnetic field and allow the operator to smoothly observe the subject information.

### First Modified Example of Sixth Embodiment

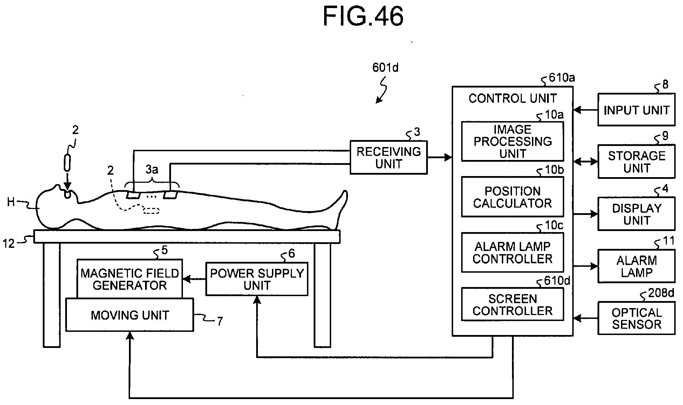

Next, a first modification of the sixth embodiment will be described. In the first modification of the sixth embodiment, the state where the light of the alarm lamp is reflected on the display screen is detected by using the optical sensor, and the display color or the light intensity of the display screen is changed on the basis of the detection result. FIG. 46 is a flowchart schematically illustrating a configuration example of an in-vivo image acquiring system according to the first modification of the sixth embodiment.

As illustrated in FIG. 46, compared to the in-vivo image acquiring system 601 illustrated in FIG. 43, an in-vivo image acquiring system 601d according to the first modification of the sixth embodiment includes the input unit 8 instead of the input unit 608, and includes a control unit 610a instead of the control unit 610. The control unit 610a includes a screen controller 610d instead of the screen controller 610c. Then, the in-vivo image acquiring system 601d further includes the optical sensor 208d. The optical sensor 208d detects the state where the light of the alarm lamp 11 is reflected on the display screen. The screen controller 610d changes the display color or the light intensity of the display screen on the basis of the detection result of the optical sensor 208d.

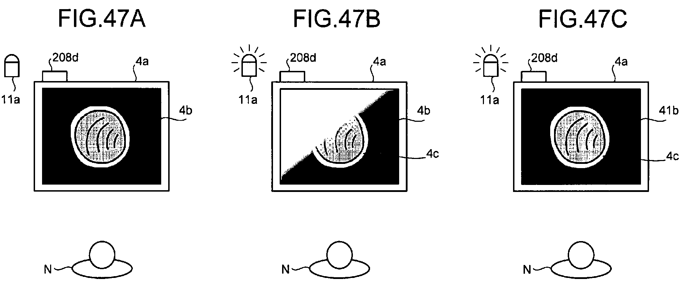

Specifically, referring to FIG. 47, the display screen control process of the in-vivo image acquiring system 601d illustrated in FIG. 46 will be described. As illustrated in FIG. 47(1), the optical sensor 208d is disposed at, for example, the upper portion of the display 4a. Then, as illustrated in FIG. 47(2), when the alarm lamp 11a emits light, the optical sensor 208d detects the light amount of the light of the alarm lamp 11a reaching the optical sensor 208d.

The screen controller 610d compares the light amount (detection value) detected by the optical sensor 208d with the light amount (initial value) of the alarm lamp 11a detected in advance by the optical sensor 208d when the light is not reflected on the display screen 4b.

When the initial value and the detection value are different from each other, so that the detection value becomes larger than the initial value, as illustrated in FIG. 47(2), it corresponds to the case where the light of the alarm lamp 11a is reflected on the display screen 4b of the display 4a. As a result, the in-vivo image 4c is whitely blurred as illustrated in FIG. 47(2). For this reason, the screen controller 610d instructs that the display color or the light intensity of the region on which the light of the alarm lamp 11a is reflected in the display screen 4b is changed in accordance with a difference value between the initial value and the detection value. Accordingly, as illustrated in FIG. 47(3), the region on which the light of the alarm lamp 11a is reflected becomes the display screen 41b which is darkened similar to the other regions. Further, the optical sensor 208d is not limited to be installed near the display 4a since it may detect only the light emission state of the alarm lamp 11a. Accordingly, the optical sensor may be installed near the operator N or may be directly attached to the head of the operator N.

In this manner, in the first modification of the sixth embodiment, since the display color or the light intensity as the display parameter of the display screen is automatically changed on the basis of the detection result of the optical sensor 208d in accordance with the state where the light of the alarm lamp 11a is reflected on the display screen, the operator may concentrate on the in-vivo observation without changing the display color or the light intensity of the display screen.

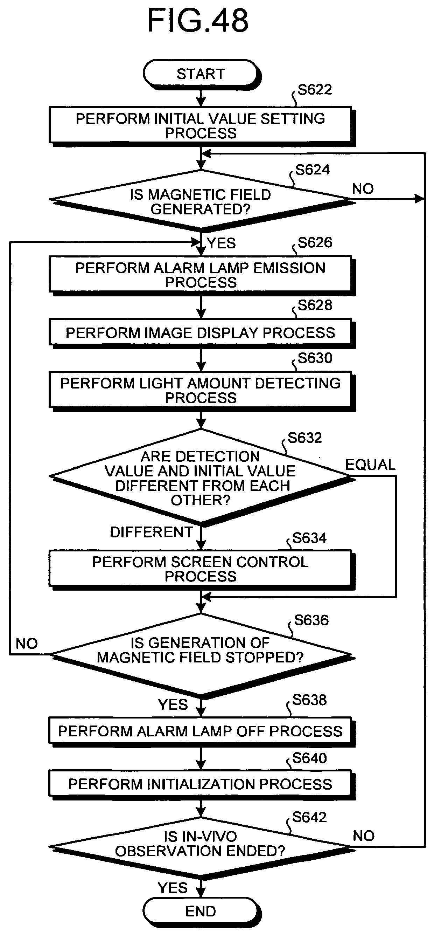

Next, referring to FIG. 48, the magnetic field generation notifying process of the in-vivo image acquiring system 601d will be described. FIG. 48 is a flowchart illustrating a process procedure of a magnetic field generation notifying process of the in-vivo image acquiring system 601d illustrated in FIG. 46.

As illustrated in FIG. 48, in the in-vivo image acquiring system 601d, the light amount of the alarm lamp 11a inside the outside observation area Ax is adjusted so that the light of the alarm lamp 11a is not reflected on the display screen of the display unit 4 under the control of the control unit 610a, the light amount in this case is detected by the optical sensor 208d, and the initial value setting process is performed to set the detected light amount as the initial value (step S622). The initial value setting process needs not to be performed for each in-vivo observation, but may be performed once when the operator is changed. Further, the process may be periodically performed in order to maintain the precision constant. Further, the alarm lamp controller 10c turns off the alarm lamp 11a after the initial value setting process.

Subsequently, as in step S2 illustrated in FIG. 5, the alarm lamp controller 10c determines whether the magnetic field generator 5 generates the magnetic field (step S624) and repeats the determination process of step S624 until the magnetic field generator 5 generates the magnetic field. Then, when it is determined that the magnetic field generator 5 generates the magnetic field (step S624: Yes), the alarm lamp controller 10c performs an alarm lamp emission process of emitting light from the alarm lamp 11a in the outside observation area Ax in order to alarm the generation of the magnetic field (step S626). Then, the image processing unit 10a performs an image display process of displaying the in-vivo image transmitted from the capsule endoscope 2 on the display unit 4 (step S628).

Then, the control unit 610a performs a light amount detecting process on the optical sensor 208d so as to detect the light amount of the light reaching the optical sensor 208d (step S630). Subsequently, the screen controller 610d determines whether the detection value and the initial value are different from each other by comparing the detection value and the initial value with each other (step S632). Furthermore, in step S632, the matching degree between the detection value and the initial value may be set by including variations in accordance with the detection precision of the optical sensor 208d, the posture of the operator N, the emission precision of the alarm lamp 11a, and the like.

When it is determined that the detection value and the initial value are different from each other (step S632: different), the screen controller 610d performs a screen control process of changing the luminance of the display screen in accordance with a difference value between the initial value and the detection value so as to handle the reflection of the light of the alarm lamp 11a (step S634). For example, in the screen control process, a table is stored in advance in which the display color information or the light intensity information capable of handling the reflection and the display color change range or the light intensity change range as the reflection range of the light on the display screen are correlated with each other for each difference value between the initial value and the detection value, and the screen controller 610d changes the display color or the light intensity of the display screen by using the display color information or the light intensity information and the light intensity change range or the display color change range in the display screen with reference to the table. As a result, since the region on which the light of the alarm lamp 11a is reflected becomes the darkened display screen 4b, the in-vivo image 4c is clearly displayed.

When the screen controller 610d determines that the detection value and the initial value are substantially equal to each other (step S632: equal) or the screen control process of step S634 is ended, the control unit 610a determines whether the magnetic field generator 5 stops the generation of the magnetic field (step S636). When it is determined that the magnetic field generator 5 does not stop the generation of the magnetic field (step S636: No), the control unit 610a continues the alarm lamp emission process of step S626. On the other hand, when it is determined that the magnetic field generator 5 stops the generation of the magnetic field (step S636: Yes), the control unit 610a performs an alarm lamp off process of turning off the alarm lamp 11a so as to stop the alarm process (step S638). Subsequently, the screen controller 610d initializes the screen display setting (step S640). Then, the control unit 610a determines whether the in-vivo observation is ended (step S642). When it is determined that the in-vivo observation is not ended (step S642: No), the current step returns to step S624. When it is determined that the in-vivo observation is ended (step S642: Yes), in accordance with the end of the in-vivo observation, the process of controlling the alarm lamp 11a in the alarm lamp controller 10c is also ended in accordance with the end of the in-vivo observation.

The in-vivo image acquiring system 601d automatically changes the display screen to have the display color or the light intensity in accordance with the state where the light of the alarm lamp 11 is reflected on the display screen by performing the respective process procedures illustrated in FIG. 48.

### Second Modified Example of Sixth Embodiment

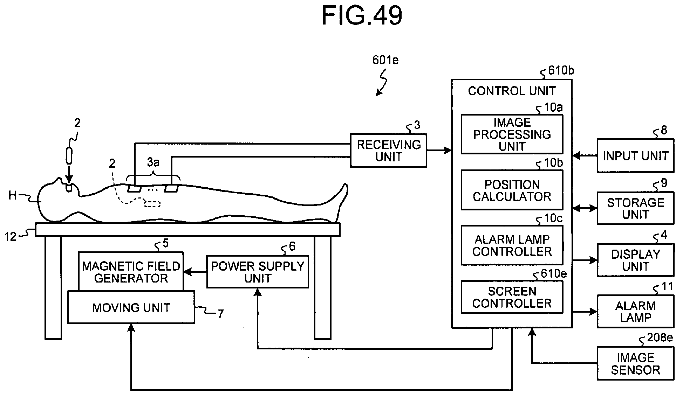

Next, a second modification of the sixth embodiment will be described. In the second modification of the sixth embodiment, the state where the light of the alarm lamp is reflected on the display screen by using the image sensor instead of the optical sensor, and the display color or the light intensity of the in-vivo image is changed on the basis of the detection result. FIG. 49 is a block diagram schematically illustrating a configuration example of an in-vivo image acquiring system according to the second modification of the sixth embodiment.

As illustrated in FIG. 49, referring to the in-vivo image acquiring system 601d illustrated in FIG. 46, an in-vivo image acquiring system 601e according to the second modification of the sixth embodiment includes the image sensor 208e instead of the optical sensor 208d, and includes a control unit 610b instead of the control unit 610a. The control unit 610b includes a screen controller 610e instead of the screen controller 610d. The image sensor 208e detects the state where the light of the alarm lamp 11 is reflected on the display screen by capturing the display screen of the display unit 4. The screen controller 610e changes the display color or the light intensity of the display screen on the basis of the detection result of the image sensor 208e.

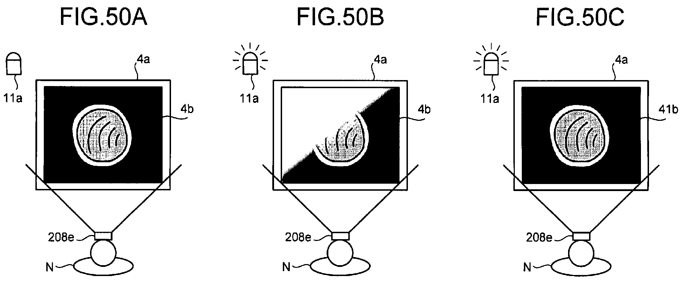

Specifically, referring to FIG. 50, the display screen control process of the in-vivo image acquiring system 601e illustrated in FIG. 49 will be described. As illustrated in FIG. 50(1), the image sensor 208e is attached to, for example, the head of the operator N so that the display screen 4b of the display 4a is included in the imaging field. Then, as illustrated in FIG. 50(2), when the alarm lamp 11a emits light, the image sensor 208e detects the state where the light of the alarm lamp 11a is reflected on the display screen by continuously capturing the display screen 4b. The image sensor 208e outputs a series of captured images to the screen controller 610e.

The screen controller 610e detects the state where the light of the alarm lamp 11a is reflected on the display screen by processing a series of captured images using the image sensor 208e. For example, the screen controller 610e compares the area (detection value) of the white region of the image of the display screen 4b captured by the image sensor 208e with the area (initial value) of the white region of the image of the display screen 4b displaying the in-vivo image captured by in advance by the image sensor 208e when there is no reflection of light.

When the initial value and the detection value are different from each other, so that the detection value becomes larger than the initial value, the screen controller 610e instructs that the display color or the light intensity of the region on which the light of the alarm lamp 11a is reflected in the display screen 4b in accordance with a difference value between the initial value and the detection value. Accordingly, as illustrated in FIG. 50(3), the region on which the light of the alarm lamp 11a is reflected becomes the display screen 41b which is darkened to be substantially similar to the other regions. Further, the image sensor 208e is not limited to be installed at the head of the operator N since it may capture the display screen of the display unit 4, and may be disposed between the operator N and the display unit 4 so that the display screen 4b of the display 4a is included in the imaging field.

In this manner, in the second modification of the sixth embodiment, since the display color or the light intensity of the display color is automatically changed on the basis of the detection result of the image sensor 208e in accordance with the state where the light of the alarm lamp 11a is reflected on the display screen, the operator may concentrate on the in-vivo observation without changing the display color or the light intensity.

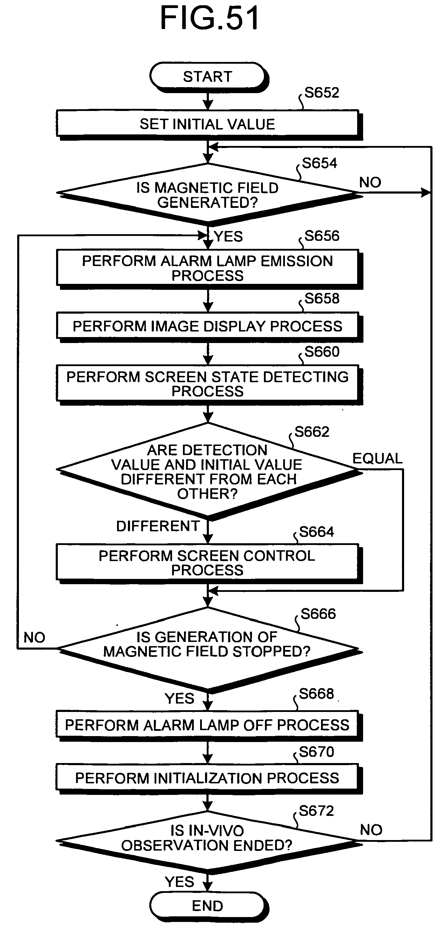

Next, referring to FIG. 51, the magnetic field generation notifying process of the in-vivo image acquiring system 601e will be described. FIG. 51 is a flowchart illustrating a process procedure of a magnetic field generation notifying process of the in-vivo image acquiring system 601e illustrated in FIG. 49.

As illustrated in FIG. 51, in the in-vivo image acquiring system 601e, the light amount of the alarm lamp 11a in the outside observation area Ax is adjusted so that the light of the alarm lamp 11a is not reflected on the display screen of the display unit 4 under the control of the control unit 610b, the display screen in this case is captured by the image sensor 208e, and the initial value setting process is performed so as to set the area of the white region of the captured display screen image as the initial value (step S652). The initial value setting process needs not to be performed for each in-vivo observation, but may be performed once when the operator is changed. Further, the process may be periodically performed in order to maintain the precision constant. Further, the alarm lamp controller 10c turns off the alarm lamp 11a after the initial value setting process.

Subsequently, as in step S2 illustrated in FIG. 5, the alarm lamp controller 10c determines whether the magnetic field generator 5 generates the magnetic field (step S654) and repeats the determination process of step S654 until the magnetic field generator 5 generates the magnetic field. Then, when it is determined that the magnetic field generator 5 generates the magnetic field (step S654: Yes), the alarm lamp controller 10c performs an alarm lamp emission process of emitting light from the alarm lamp 11a in the outside observation area Ax in order to alarm the generation of the magnetic field (step S656). Then, the image processing unit 10a performs an image display process of displaying the in-vivo image transmitted from the capsule endoscope 2 on the display unit 4 (step S658).

Then, the screen controller 610e performs a screen state detecting process of calculating the area of the white region in the captured display screen image as the detection value by capturing the display screen of the display unit 4 using the image sensor 208e (step S660). Subsequently, the screen controller 610e determines whether the detection value and the initial value are different from each other by comparing the detection value and the initial value with each other (step S662). Furthermore, in step S662, the matching degree between the detection value and the initial value may be set by including variations in accordance with the detection precision of the optical sensor 208d, the posture of the operator N, the emission precision of the alarm lamp 11a, and the like.

When it is determined that the detection value and the initial value are different from each other (step S662: different), the screen controller 610e performs a screen control process of changing the display color or the light intensity of the display screen in accordance with a difference value between the initial value and the detection value so as to handle the reflection of the light of the alarm lamp 11a (step S664). For example, in the screen control process, a table is stored in advance in which the display color information or the light intensity information capable of handling the reflection and the display color change range or the light intensity change range as the reflection range of the light on the display screen are correlated with each other for each difference value between the initial value and the detection value, and the screen controller 610e changes the display color or the light intensity of the display screen by using the display color information or the light intensity information and the light intensity change range or the display color change range in the display screen with reference to the table. As a result, since the region on which the light of the alarm lamp 11a is reflected becomes the display screen 41b, the in-vivo image 4c is clearly displayed.

When the screen processing unit 610e determines that the detection value and the initial value are substantially equal to each other (step S662: equal) or the screen control process of step S664 is ended, the control unit 610b determines whether the magnetic field generator 5 stops the generation of the magnetic field (step S666). When it is determined that the magnetic field generator 5 does not stop the generation of the magnetic field (step S666: No), the control unit 610b continues the alarm lamp emission process of step S656. On the other hand, when it is determined that the magnetic field generator 5 stops the generation of the magnetic field (step S666: Yes), the control unit 610b performs an alarm lamp off process of turning off the alarm lamp 11a so as to stop the alarm process (step S668). Subsequently, the screen controller 610e initializes the screen display setting (step S670). Then, the control unit 610b determines whether the in-vivo observation is ended (step S672). When it is determined that the in-vivo observation is not ended (step S672: No), the current step returns to step S654. When it is determined that the in-vivo observation is ended (step S672: Yes), the process of controlling the alarm lamp 11a in the alarm lamp controller 10c is also ended in accordance with the end of the in-vivo observation.

The in-vivo image acquiring system 601e automatically changes the display color or the light intensity of the display screen in accordance with the state where the light of the alarm lamp 11 is reflected on the display screen by performing the respective process procedures illustrated in FIG. 51.

### Seventh Embodiment

In the observation performed by guiding the capsule endoscope, when the operator finds out an interest portion while visually identifying an image captured by the capsule endoscope and displayed on the display unit, the operator observes that portion in more detail by moving the capsule endoscope closer thereto in many cases. When the operator carefully watches the image, in many cases, the capsule endoscope stays at a predetermined position or is guided at a comparatively low moving speed. Therefore, in the seventh embodiment, when the capsule endoscope is guided at a low moving speed, the magnetic field intensity with respect to the capsule endoscope is constantly maintained without any change, and the operator who carefully watches the image does not move. Accordingly, the alarm lamp is turned off to help the operator more smoothly observe the image.

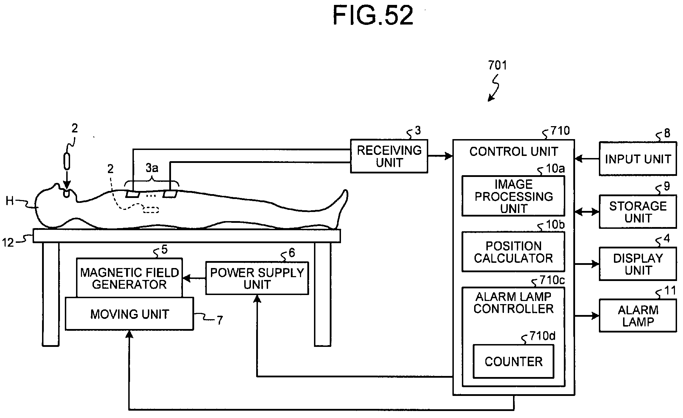

FIG. 52 is a block diagram schematically illustrating a configuration example of an in-vivo image acquiring system according to the seventh embodiment. As illustrated in FIG. 52, compared to the in-vivo image acquiring system 1 illustrated in FIG. 1, an in-vivo image acquiring system 701 according to the seventh embodiment includes a control unit 710 instead of the control unit 10.

Compared to the control unit 10, the control unit 710 includes an alarm lamp controller 710c including a counter 710d with a counting function instead of the alarm lamp controller 10c. The alarm lamp controller 710c controls the light emission process of the alarm lamp 11. The alarm lamp controller 710c generates light in the alarm lamp 11 when the magnetic field generator 5 generates the magnetic field, and stops the generation of light in the alarm lamp 11 when a variation amount of the intensity of the magnetic field generated by the magnetic field generator 5 becomes less than a predetermined reference value for a predetermined time. The predetermined reference value is set in advance so as to correspond to the variation amount of the magnetic field intensity demanding the attention of the operator in accordance with the usage status or the like of each in-vivo image acquiring system 701.

However, when a joystick constituting the input unit 8 is operated, the control unit 710 generates the magnetic field in the magnetic field generator 5 so as to have an intensity corresponding to the operation amount of the joystick. In other words, when the joystick is operated, operation information instructing the generation of the magnetic field with an intensity corresponding to the operation amount of the joystick is input from the input unit 8, and the control unit 710 generates the magnetic field in the magnetic field generator 5 to have an intensity instructed by the input operation information. That is, the operation amount of the joystick and the intensity of the magnetic field generated by the magnetic field generator 5 correspond to each other.

For this reason, the alarm lamp controller 710c acquires the operation amount of the joystick a plurality of times. Then, when the variation amount of the operation amount of the joystick becomes less than a predetermined operation amount reference value for a predetermined time, the alarm controller determines that the variation amount of the magnitude of the magnetic field generated by the magnetic field generator 5 is less than the predetermined reference value for a predetermined time, and stops the generation of light in the alarm lamp 11.

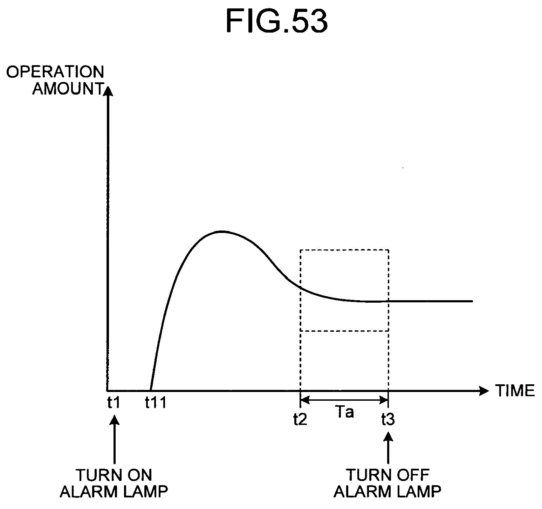

Specifically, referring to FIG. 53, the light emission control of the alarm lamp 11 using the alarm lamp controller 710c will be described. FIG. 53 is a diagram illustrating time dependence of the operation amount of the joystick.

As illustrated in FIG. 53, the alarm lamp controller 710c turns on the alarm lamp 11 at the time t1 at which a main switch (not illustrated) of the in-vivo image acquiring system 701 is turned on so that the magnetic field may be generated. The lamp on operation is started regardless of the operation of the joystick. In FIG. 53, the joystick is operated from the time t11 subsequent to the time t1. Then, when the variation amount of the operation amount of the joystick is less than a predetermined operation amount reference value for a predetermined period Ta from the time t2, the alarm lamp controller 710c turns off the alarm lamp 11 at the time t3 elapsed from the time t2 by a predetermined period Ta. Furthermore, when the variation amount of the operation amount of the joystick becomes the predetermined operation amount reference value or more, the alarm lamp controller 710c turns on the alarm lamp 11 again. Further, the predetermined period Ta used as a reference for turning off the alarm lamp 11 may be changed in accordance with the operation amount of each in-vivo image acquiring system 701.

In this manner, in the seventh embodiment, when the variation amount of the operation amount of the joystick becomes less than the predetermined operation amount reference value for a predetermined time, the alarm lamp 11 is turned off to help the operator more smoothly observe the image.

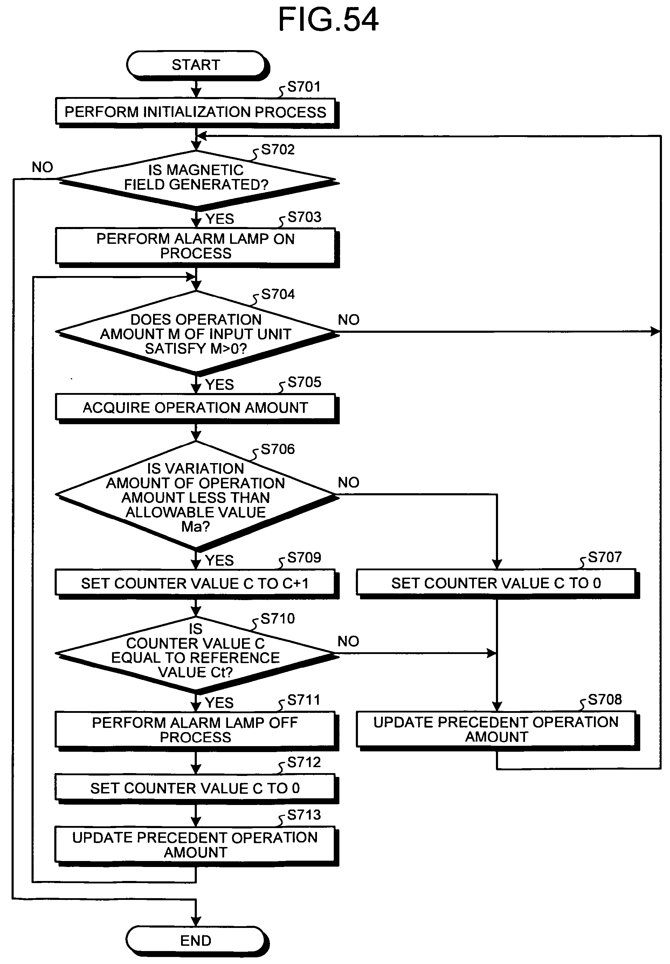

Next, referring to FIG. 54, a process of controlling the alarm lamp 11 of the in-vivo image acquiring system 701 will be described. FIG. 54 is a flowchart illustrating a process procedure of a magnetic field generation notifying process of the in-vivo image acquiring system 701 illustrated in FIG. 52.

As illustrated in FIG. 54, the alarm lamp controller 710c first performs the initialization process of initializing each numerical value used for the process of controlling the alarm lamp 11 (step S701). For example, the alarm lamp controller 710c sets the precedent operation amount of the joystick to 0, sets the counter value C of the counter 710d to 0, and turns off the control signal of the alarm lamp 11.

Subsequently, the alarm lamp controller 710c determines whether the main switch of the in-vivo image acquiring system 701 is turned on so that the magnetic field generator 5 may generate the magnetic field (step S702). For example, when it is determined that the main switch of the in-vivo image acquiring system 701 is turned off so that the magnetic field generator 5 stops the generation of the magnetic field (step S702: No), the alarm lamp controller 710c directly ends the process of controlling the alarm lamp 11.

On the contrary, when it is determined that the magnetic field generator 5 may generate the magnetic field (step S702: Yes), the alarm lamp controller 710c performs an alarm lamp on process of turning on the alarm lamp 11 (step S703).

Later, the alarm lamp controller 710c determines whether the operation amount M of the joystick of the input unit 8 satisfies M>0 (step S704). The determination process of step S704 is periodically performed.

When it is determined that the operation amount M of the joystick of the input unit 8 does not satisfies M>0 (step S704: No), that is, M = 0 and the joystick is not operated, the alarm lamp controller 710c returns the current step to step S702, and determines whether the magnetic field generator 5 may generate the magnetic field.

On the other hand, when the operation amount M of the joystick of the input unit 8 satisfies M>0 (step S704: Yes), that is, the joystick is operated, the alarm lamp controller 710c acquires the operation amount of the joystick of the input unit 8 (step S705). Subsequently, the alarm lamp controller 710c obtains the variation amount of the operation amount by comparing the acquired operation amount and the precedent acquired operation amount, and determines whether the obtained variation amount of the operation amount is less than the allowable value Ma as the predetermined operation amount reference value (step S706). The allowable value Ma is set in advance in accordance with the usage status of each in-vivo image acquiring system 701.

When it is determined that the variation amount of the operation amount is not less than the predetermined allowable value Ma (step S706: No), that is, the variation amount of the operation amount is the predetermined allowable value Ma or more, the alarm lamp controller 710c sets the counter value C of the counter 710d to C = 0 (step S707). Subsequently, the alarm lamp controller 710c updates the precedent operation amount of the joystick to the newly acquired operation amount (step S708), and returns the current step to step S702.

On the other hand, when it is determined that the variation amount of the operation amount is less than the predetermined allowable value Ma (step S706: Yes), the alarm lamp controller 710c performs a process of obtaining the counter value C = C + 1 by performing an adding process using the counter 710d (step S709). Subsequently, the alarm lamp controller 710c determines whether the counter value C is a predetermined reference value Ct (step S710). Since the determination process of step S706 is periodically performed, the predetermined reference value Ct with respect to the counter value C is set so as to correspond to the predetermined time Ta used as the reference for turning off the alarm lamp 11.

When the alarm lamp controller 710c determines that the counter value C is not the predetermined reference value Ct (step S710: No), the period in which the variation amount of the operation amount is less than the predetermined operation amount reference value does not reach the predetermined time Ta used as the reference for tuning off the alarm lamp 11. For this reason, the alarm lamp controller 710c updates the precedent operation amount of the joystick to the newly acquired operation amount (step S708), and returns the current step to step S702 so as to perform the determination process again.

Further, the alarm lamp controller 710c determines that the counter value C is the predetermined reference value Ct (step S710: Yes), the period in which the variation amount of the operation amount is less than the predetermined operation amount reference value reaches the predetermined time Ta used as the reference for turning off the alarm lamp 11. For this reason, the alarm lamp controller 710c performs an alarm lamp off process of turning off the alarm lamp 11 (step S711). Subsequently, the alarm lamp controller 710c initializes the counter value C to become C = 0 (step S712), updates the precedent operation amount of the joystick to the newly acquired operation amount (step S713), and returns the current step to step S704 so as to perform a determination process with respect to the operation amount of the joystick of the input unit 8 again.

Since the in-vivo image acquiring system 701 performs the respective process procedures illustrated in FIG. 54, when the variation amount of the operation amount of the joystick is less than the predetermined operation amount reference value for a predetermined time, the alarm lamp 11 is turned off to help the operator smoothly observe the image.

Furthermore, a case has been described in which the alarm lamp 11 is controlled on the basis of the operation amount of the joystick so that the operation amount of the joystick and the intensity of the magnetic field generated by the magnetic field generator 5 correspond to each other, but of course, the invention is not limited thereto. For example, a magnetic field sensor may be provided so as to detect the intensity of the magnetic field generated by the magnetic field generator 5, so that the alarm lamp 11 may be controlled on the basis of the intensity of the magnetic field generated by the magnetic field generator 5. A current detecting unit may be provided so as to detect a value of a current flowing to the coil-shaped electromagnet constituting the magnetic field generator 5, so that the alarm lamp 11 may be controlled on the basis of the current value detected by the current detecting unit.

### Eighth Embodiment

Next, an eighth embodiment will be described. In the eighth embodiment, when the intensity of the magnetic field generated by the magnetic field generator becomes a predetermined threshold value or more, another alarm lamp emits light so as to alarm the operator for the generation of the magnetic field.

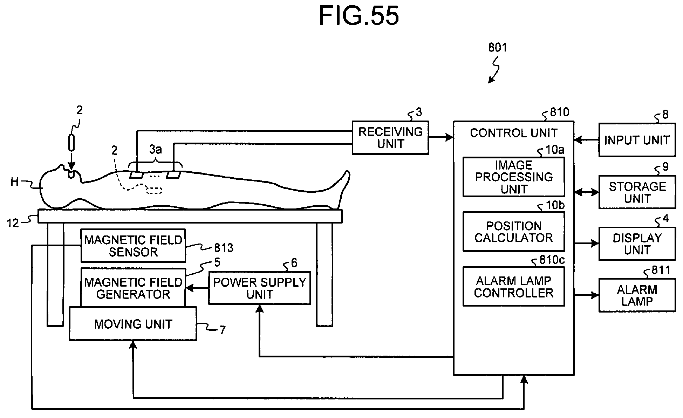

FIG. 55 is a block diagram schematically illustrating a configuration example of an in-vivo image acquiring system according to the eighth embodiment. As illustrated in FIG. 55, compared to the in-vivo image acquiring system 1 illustrated in FIG. 1, the in-vivo image acquiring system 801 according to the eighth embodiment includes a control unit 810 including an alarm lamp controller 810c instead of the control unit 10. Further, the in-vivo image acquiring system 801 includes an alarm lamp 811 that warns the operator that the magnetic field intensity demanding the attention of the operator is generated, and includes a magnetic field sensor 813 that detects the intensity of the magnetic field generated by the magnetic field generator 5. As in the alarm lamp 11, the alarm lamp 811 is installed in the above-described outside observation area in order to reduce the amount of the light intruding into the above-described display corresponding area when light is generated.

The alarm lamp controller 810c generates the alarm lamp 811 when the intensity of the magnetic field generated by the magnetic field generator 5 becomes more than the predetermined threshold value. The alarm lamp controller 810c generates light in the alarm lamp 811 when the intensity of the magnetic field detected by the magnetic field sensor 813 becomes more than the predetermined threshold value. The predetermined threshold value is set in advance so as to correspond to the intensity of the magnetic field demanding the attention of the operator in accordance with the usage status or the like of each in-vivo image acquiring system 801. Furthermore, when the magnetic field generator 5 generates the magnetic field, another alarm lamp (not illustrated) may be turned on.

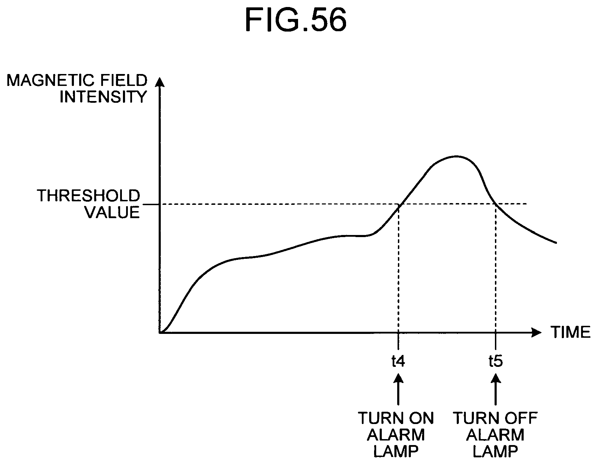

Specifically, referring to FIG. 56, the light emission control of the alarm lamp 811 using the alarm lamp controller 810c will be described. FIG. 56 is a diagram illustrating time dependence of the operation amount of the joystick.

As illustrated in FIG. 56, the alarm lamp controller 810c turns on the alarm lamp 811 at the time t4 when the intensity of the magnetic field generated by the magnetic field generator 5 becomes more than a predetermined threshold value at the time t4 after a main switch (not illustrated) of the in-vivo image acquiring system 801 is turned on so that the magnetic field may be generated. Subsequently, the alarm lamp controller 810c turns off the alarm lamp 811 when the intensity of the magnetic field generated by the magnetic field generator 5 becomes less than the predetermined threshold value at the time t5.

In this manner, in the eighth embodiment, when the intensity of the magnetic field generated by the magnetic field generator 5 becomes more than the predetermined threshold value, the alarm lamp 811 is turned on so as to reliably warn the operator that the magnetic field intensity demanding the attention of the operator is generated. Further, when the magnetic field generator 5 generates the magnetic field with intensity more than the predetermined threshold value, it corresponds to the case where the operator operates the input unit 8 to guide the capsule endoscope 2. Accordingly, the operator does not carefully watch the image of the capsule endoscope 2 for the purpose of diagnosis in many cases, so that even when the alarm lamp 811 is turned on, the operator who observes the image is not disturbed.

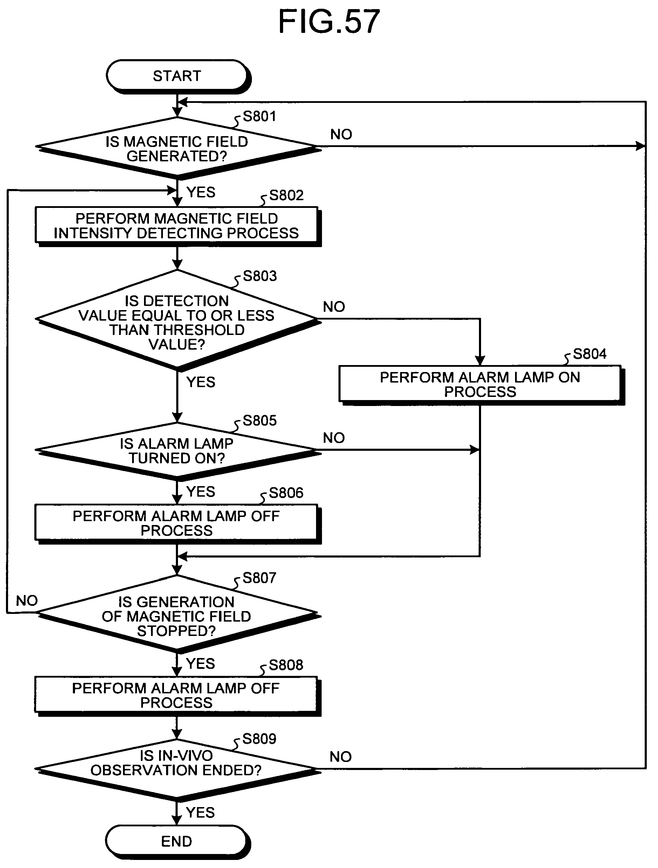

Next, referring to FIG. 57, a process of controlling the alarm lamp of the in-vivo image acquiring system 801 will be described. FIG. 57 is a flowchart illustrating a process procedure of a magnetic field generation notifying process of the in-vivo image acquiring system 801 illustrated in FIG. 55.

As illustrated in FIG. 57, the alarm lamp controller 810c determines whether the magnetic field generator 5 generates the magnetic field (step S801). The alarm lamp controller 810c repeats the determination process of step S801 until the magnetic field generator 5 generates the magnetic field.

When it is determined that the magnetic field generator 5 generates the magnetic field (step S801: Yes), the alarm lamp controller 810c performs a magnetic field intensity detecting process on the magnetic field sensor 813 so as to detect the intensity of the magnetic field generated by the magnetic field generator 5 (step S802). The alarm lamp controller 810c determines whether the detection value of the intensity of the magnetic field using the magnetic field sensor 813 is a predetermined threshold value or less (step S803).

When it is determined that the detection value of the intensity of the magnetic field obtained by the magnetic field sensor 813 is not the predetermined threshold value or less (step S803: No), that is, the detection value of the intensity of the magnetic field obtained by the magnetic field sensor 813 becomes more than the predetermined threshold value, the alarm lamp controller 810c performs an alarm lamp on process of turning on the alarm lamp 811 so as to prompt the attention of the operator (step S804).

On the other hand, when it is determined that the detection value of the intensity of the magnetic field obtained by the magnetic field sensor 813 is the predetermined threshold value or less (step S803: Yes), the alarm lamp controller 810c determines whether the alarm lamp 811 is turned on (step S805). When it is determined that the alarm lamp 811 is turned on (step S805: Yes), the alarm lamp controller 810c performs an alarm lamp off process of turning off the alarm lamp 811 (step S806).
When it is determined that the alarm lamp 811 is not turned on (step S805: No), the alarm lamp off process (step S806) is ended, or the alarm lamp on process (step S804) is ended, the alarm lamp controller 810c determines whether the magnetic field generator 5 stops the generation of the magnetic field (step S807).

When it is determined that the magnetic field generator 5 does not stop the generation of the magnetic field (step S807: No), the alarm lamp controller 810c returns the current step to step S802 so as to perform a magnetic field intensity detecting process, and performs a determination process with respect to the intensity of the magnetic field again. On the other hand, when it is determined that the magnetic field generator 5 stops the generation of the magnetic field (step S807: Yes), the alarm lamp controller 810c performs an alarm lamp off process of turning off the alarm lamp 811 when the alarm lamp 811 is turned on (step S808). Subsequently, the control unit 810 determines whether the in-vivo observation is ended (step S809). When it is determined that the in-vivo observation is not ended (step S809: No), the control unit 810 returns the current step to step S801. On the other hand, when it is determined that the in-vivo observation is ended (step S809: Yes), the control unit 810 ends the process of controlling the alarm lamp 811 in the alarm lamp controller 810c in accordance with the end of the in-vivo observation.

Since the in-vivo image acquiring system 801 performs the respective process procedures illustrated in FIG. 57, when the intensity of the magnetic field generated by the magnetic field generator 5 becomes more than the predetermined threshold value, the alarm lamp 811 is turned on so as to reliably warn the operator.

### First Modified Example of Eighth Embodiment

Next, a first modification of the eighth embodiment will be described. In the first modification of the eighth embodiment, a value of a current flowing to the coil-shaped electromagnet constituting the magnetic field generator is detected, and the alarm lamp is controlled on the basis of the detection value.

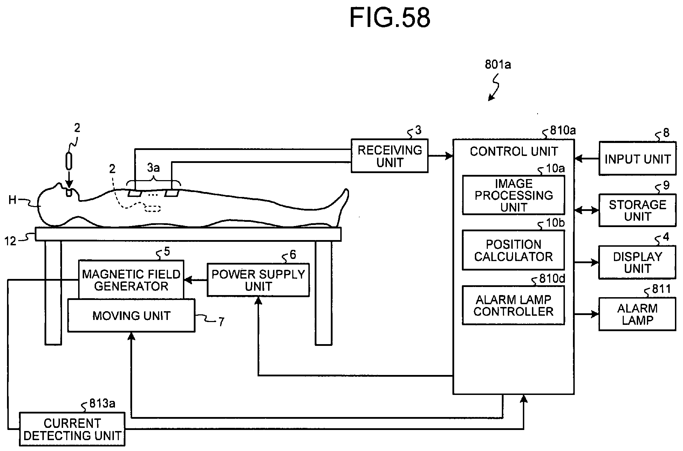

FIG. 58 is a block diagram schematically illustrating a configuration example of an in-vivo image acquiring system according to the first modification of the eighth embodiment. As illustrated in FIG. 58, compared to the in-vivo image acquiring system 801 illustrated in FIG. 55, an in-vivo image acquiring system 801a according to the first modification of the eighth embodiment includes a control unit 810a including an alarm lamp controller 810d instead of the control unit 810. Further, the in-vivo image acquiring system 801a includes a current detecting unit 813a detecting a value of a current flowing to the coil-shaped electromagnet constituting the magnetic field generator 5 instead of the magnetic field sensor 813. Since the value of the current flowing to the electromagnet is proportional to the intensity of the magnetic field generated from the electromagnet, the alarm lamp controller 810d performs an alarm lamp control process using the value of the current flowing to the coil-shaped electromagnet constituting the magnetic field generator 5.

When it is determined that the current value detected by the current detecting unit 813a becomes more than a predetermined current value, the alarm lamp controller 810d generates light in the alarm lamp 811. The predetermined current value is a value of a current supplied in order to generate the magnetic field with intensity in accordance with the predetermined threshold value described in the eighth embodiment, and is set in accordance with the usage status or the like of each in-vivo image acquiring system 801.

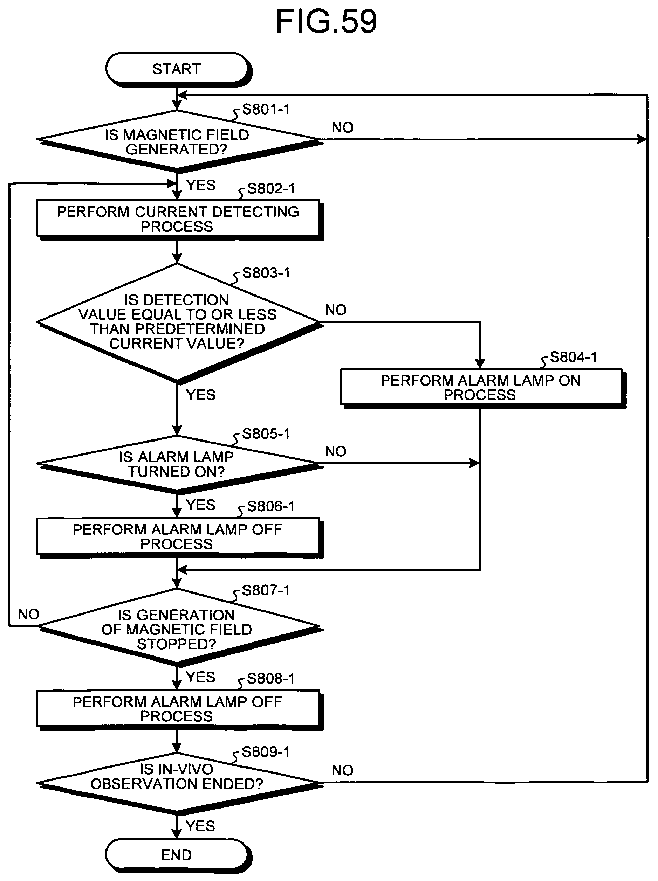

Next, referring to FIG. 59, the process of controlling the alarm lamp in the in-vivo image acquiring system 801a will be described. FIG. 59 is a flowchart illustrating a process procedure of a magnetic field generation notifying process of the in-vivo image acquiring system 801a illustrated in FIG. 58.

As illustrated in FIG. 59, as in step S801 and step S802 illustrated in FIG. 57, the alarm lamp controller 810d determines whether the magnetic field generator 5 generates the magnetic field until the magnetic field generator 5 generates the magnetic field (step S801-1).

Then, when it is determined that the magnetic field generator 5 generates the magnetic field (step S801-1: Yes), the alarm lamp controller 810d performs a current detecting process on the current detecting unit 813a so as to detect the value of the current flowing to the coil-shaped electromagnet constituting the magnetic field generator 5 (step S802-1). Later, the alarm lamp controller 810d determines whether the current value detected by the current detecting unit 813a is the predetermined current value or less (step S803-1).

When it is determined that the current value detected by the current detecting unit 813a is not the predetermined current value or less (step S803-1: No) and the current value detected by the current detecting unit 813a becomes more than the predetermined current value, the alarm lamp controller 810d performs an alarm lamp on process of turning on the alarm lamp 811 so as to prompt the attention of the operator (step S804-1).

On the other hand, when it is determined that the current value detected by the current detecting unit 813a is the predetermined current value or less (step S803-1: Yes), the alarm lamp controller 810d determines whether the alarm lamp 811 is turned on (step S805-1). When it is determined that the alarm lamp 811 is turned on (step S805-1: Yes), the alarm lamp controller performs an alarm lamp off process of turning off the alarm lamp 811 (step S806-1). When the alarm lamp controller 810d determines that the alarm lamp 811 is not turned on (step S805-1: No), the alarm lamp off process (step S806-1) is ended, or the alarm lamp on process (step S804-1) is ended, the alarm lamp controller 810d determines whether the magnetic field generator 5 stops the generation of the magnetic field (step S807-1).

When it is determined that the magnetic field generator 5 does not stop the generation of the magnetic field (step S807-1: No), the alarm lamp controller 810d returns the current step to step S802-1. On the other hand, when it is determined that the magnetic field generator 5 stops the generation of the magnetic field (step S807-1: Yes), the alarm lamp controller 810d performs an alarm lamp off process of turning off the alarm lamp 811 when the alarm lamp 811 is turned on (step S808-1). Subsequently, the control unit 810a determines whether the in-vivo observation is ended (step S809-1). When it is determined that the in-vivo observation is not ended (step S809-1: No), the control unit 810a returns the current step to step S801-1. On the other hand, when it is determined that the in-vivo observation is ended (step S809-1: Yes), the control unit 810a ends the process of controlling the alarm lamp 811 in the alarm lamp controller 810d in accordance with the end of the in-vivo observation.

Since the in-vivo image acquiring system 801a performs the respective process procedures illustrated in FIG. 59, when the value of the current flowing to the coil-shaped electromagnet constituting the magnetic field generator 5 becomes more than the predetermined current value, the alarm lamp 811 is further turned on as to reliably warn the operator.

### Ninth Embodiment

Next, a ninth embodiment will be described. In the ninth embodiment, an on period of turning on the alarm lamp in accordance with the operation information input from the input unit is set on the basis of the falling time of the intensity of the magnetic field generated by the magnetic field generator.

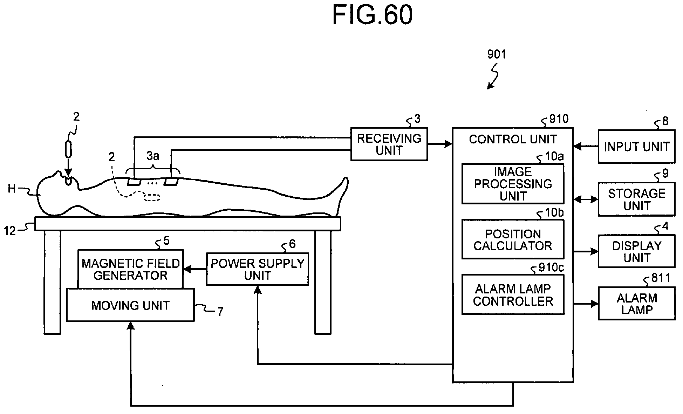

FIG. 60 is a block diagram schematically illustrating a configuration example of an in-vivo image acquiring system according to the ninth embodiment. As illustrated in FIG. 60, compared to the in-vivo image acquiring system 1 illustrated in FIG. 1, an in-vivo image acquiring system 901 according to the ninth embodiment includes a control unit 910 including an alarm lamp controller 910c instead of the control unit 10. Further, the in-vivo image acquiring system 901 includes the alarm lamp 811.

When the operation information input by the operation of the joystick of the input unit 8 is predetermined operation information, the alarm lamp controller 910c turns on the alarm lamp 811. In this case, when the operation information input by the input unit 8 is predetermined operation information for instructing the generation of the magnetic field with a predetermined intensity or more, the alarm lamp controller 910c sets an on period of turning on the alarm lamp 811, and turns on the alarm lamp 811 during the set on period. The alarm lamp controller 910c sets the on period of the alarm lamp 811 on the basis of the falling time of the intensity of the magnetic field generated by the magnetic field generator 5.

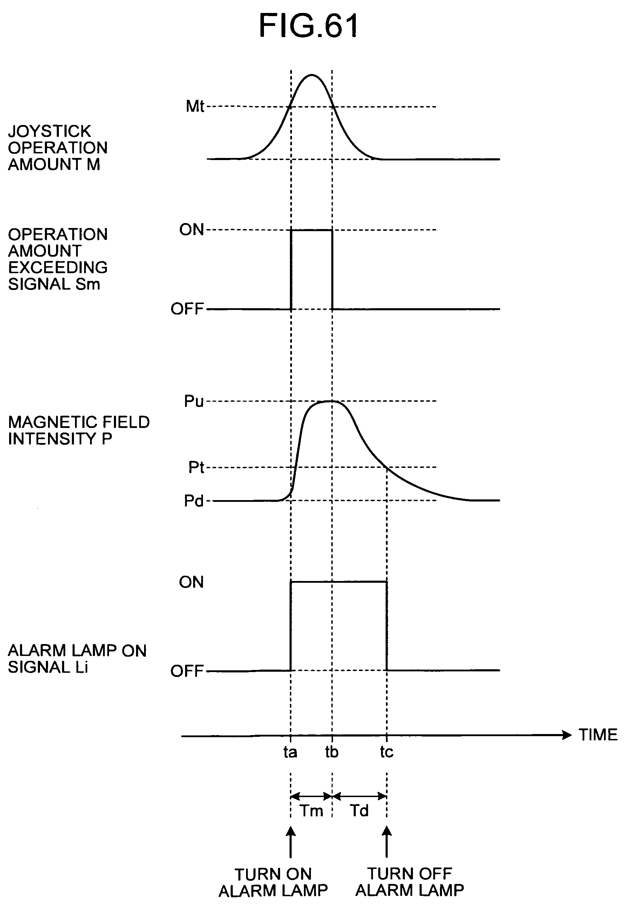

Specifically, referring to FIG. 61, the light emission control of the alarm lamp 811 using the alarm lamp controller 910c will be described. FIG. 61 is a diagram illustrating time dependence of each of the operation amount M of the joystick, the operation amount exceeding signal Sm, the intensity P of the magnetic field generated by the magnetic field generator 5, and the alarm lamp on signal Li for turning on the alarm lamp 811. The operation amount exceeding signal Sm is a signal that is input to the alarm lamp controller 910c when the operation amount of the joystick of the input unit 8 becomes more than the operation amount for instructing the magnetic field generator 5 to generate the magnetic field with an intensity demanding the attention of the operator.

As illustrated in FIG. 61(1), the operation amount exceeding signal Sm is turned on from an off state as illustrated in FIG. 61(2) at the time ta at which the operation amount M of the joystick becomes more than the operation amount Mt, and is input to the alarm lamp controller 910c. The operation amount Mt corresponds to the operation amount for instructing the magnetic field generator 5 to generate the magnetic field with the intensity Pt (refer to FIG. 61(3)) demanding the attention of the operator. Further, the intensity P of the magnetic field generated by the magnetic field generator 5 gradually increases in accordance with an increase in the operation amount M of the joystick, so that it becomes intensity Pu (>Pt). Later, when the operation amount M decreases and the operation amount M becomes less than the operation amount Mt, the operation amount exceeding signal Sm becomes an off state. However, in fact, as illustrated in FIG. 61(3), an increase and decrease in the intensity of the magnetic field generated by the magnetic field generator 5 does not match an increase and decrease in the operation amount in terms of timing, but is delayed with respect to an increase and decrease in the operation amount.

Accordingly, as illustrated in FIG. 61(4), the alarm lamp controller 910c sets the on period of the alarm lamp 811 by adding the period Tm from the time ta actually turning on the operation amount exceeding signal Sm to the time tb to the period Td until the time tc at which the magnetic field intensity P decreases to the intensity Pt in consideration of a deviation in timing.

Therefore, the alarm lamp controller 910c turns on the alarm lamp on signal Li from an off state during an on period from the time ta to the time tc set as the on period of the alarm lamp 811. As a result, the alarm lamp 811 is turned on at the time ta, and is turned off at the time tc.

In this manner, in the ninth embodiment, the alarm lamp 811 is turned on during a time in which the intensity of the magnetic field generated by the magnetic field generator decreases to the intensity Pt demanding the attention of the operator, and the alarm lamp is turned off after the intensity of the magnetic field becomes less than the intensity Pt, thereby reliably warning the operator. Further, when the variation amount of the operation amount of the joystick is the predetermined operation amount reference value or more, it corresponds to the case in which the operator operates the joystick to guide the capsule endoscope 2. Accordingly, the operator does not carefully watch the image of the capsule endoscope 2 for the purpose of diagnosis in many cases, so that even when the alarm lamp 811 is turned on, the operator who observes the image is not disturbed.

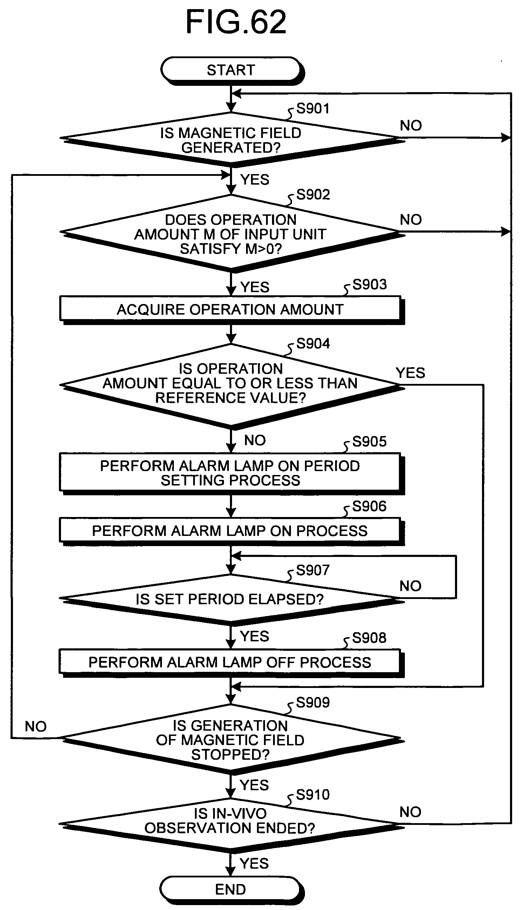

Next, referring to FIG. 62, the process of controlling the alarm lamp in the in-vivo image acquiring system 901 will be described. FIG. 62 is a flowchart illustrating a process procedure of a magnetic field generation notifying process of the in-vivo image acquiring system 901 illustrated in FIG. 60.

As illustrated in FIG. 62, the alarm lamp controller 910c determines whether the magnetic field generator 5 generates the magnetic field (step S901). The alarm lamp controller 910c repeats the determination process of step S901 until the magnetic field generator 5 generates the magnetic field.

When it is determined that the magnetic field generator 5 generates the magnetic field (step S901: Yes), the alarm lamp controller 910c determines whether the operation amount M of the joystick of the input unit 8 becomes M>0 (step S902). When it is determined that the operation amount M of the joystick of the input unit 8 is not M>0 (step S902: No), that is, M = 0 and the joystick is not operated, the alarm lamp controller 910c returns the current step to step S901 so as to determine whether the magnetic field generator 5 may generate the magnetic field. On the other hand, when it is determined that the operation amount M of the joystick of the input unit 8 is M>0 (step S902: Yes), that is, the joystick is operated, the alarm lamp controller 910c acquires the operation amount of the joystick of the input unit 8 (step S903).

Subsequently, the alarm lamp controller 910c determines whether the acquired operation amount is a reference value or less by comparing the acquired operation amount with a reference value of the operation amount corresponding to the magnetic field intensity demanding the attention of the operator (step S904).

When the acquired operation amount is not a reference value or less (step S904: No), the magnetic field with an intensity demanding the attention of the operator is generated from the magnetic field generator 5. Accordingly, the alarm lamp controller 910c performs an alarm lamp on period setting process of setting the on period of the alarm lamp 811 so as to prompt the attention of the operator (step S905). In the alarm lamp on period setting process, the alarm lamp controller 910c sets the on period of the alarm lamp 811 by adding the extension time based on the falling time of the intensity of the magnetic field generated by the magnetic field generator 5 to the period in which the joystick actually instructs the generation of the magnetic field with an intensity demanding the attention of the operator. The extension period is set in advance, and is stored in the storage unit 9. As the extension period, a constant period may be set, and different periods may be set in accordance with the time dependence of the intensity of the magnetic field generated by the magnetic field generator 5 and the operation amount of the joystick.

Subsequently, the alarm lamp controller 910c performs an alarm lamp on process of turning on the alarm lamp 811 during the set on period (step S906). Later, the alarm lamp controller 910c determines whether the set on period is elapsed after turning on the alarm lamp 811 (step S907). When it is determined that the set on period is not elapsed (step S907: No), the alarm lamp controller 910c repeats the determination process of step S907. On the other hand, when it is determined that the set on period is elapsed (step S907: Yes), the alarm lamp controller 910c performs an alarm lamp off process of turning off the alarm lamp 811 (step S908).

Then, when the alarm lamp controller 910c determines that the acquired operation amount is a reference value or less (step S904: Yes) or the alarm lamp off process (step S908) is ended, the alarm lamp controller 910c determines whether the magnetic field generator 5 stops the generation of the magnetic field (step S909).

When it is determined that the magnetic field generator 5 does not stop the generation of the magnetic field (step S909: No), the alarm lamp controller 910c returns the current step to step S902 so as to perform the determination process with respect to the operation amount M of the joystick of the input unit 8 again. On the other hand, when the alarm lamp controller 910c determines that the magnetic field generator 5 stops the generation of the magnetic field (step S909: Yes), the control unit 910 determines whether the in-vivo observation is ended (step S910). When it is determined that the in-vivo observation is not ended (step S910: No), the control unit 910 returns the current step to step S901. On the other hand, when it is determined that the in-vivo observation is ended (step S910: Yes), the control unit 910 ends the process of controlling the alarm lamp 811 in the alarm lamp controller 910c in accordance with the end of the in-vivo observation.

Since the in-vivo image acquiring system 901 performs the respective process procedures illustrated in FIG. 62, the alarm lamp 811 is turned on so as to reliably warn the operator until the intensity of the magnetic field generated by the magnetic field generator 5 decreases to the intensity demanding the attention of the operator.

Furthermore, in the ninth embodiment, a case has been described in which the on period of the alarm lamp 811 is set on the basis of the operation amount of the joystick, but of course, the invention is not limited thereto.

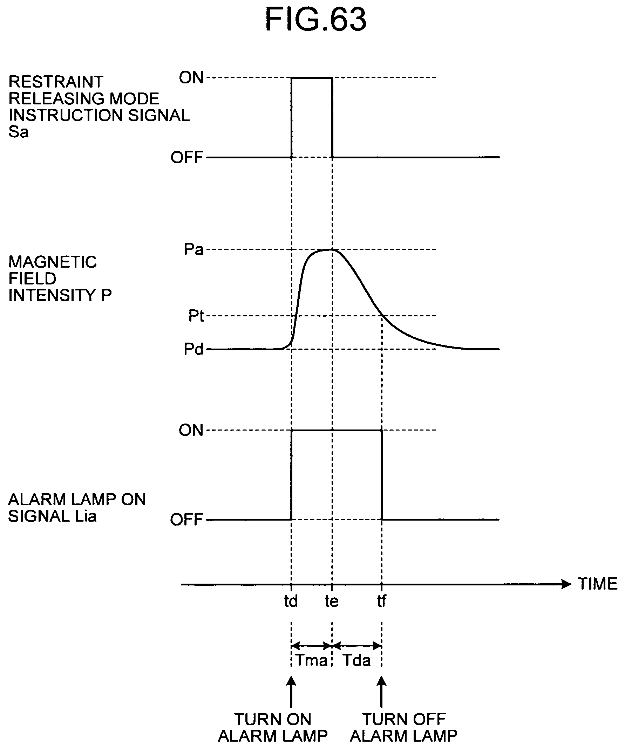

For example, in order to release the capsule endoscope 2 from the restraint of mucus, a restraint releasing mode button is installed in the joystick of the input unit 8 so as to instruct the capsule endoscope 2 to be oscillated. In the in-vivo image acquiring system 901, as illustrated in FIG. 63(1), when the restraint releasing mode button is pressed at the time td so that a restraint releasing mode instruction signal Sa is input from the input unit 8, it is controlled so that the magnetic field generator 5 generates a magnetic field with a predetermined intensity Pa (>Pt) until the predetermined time te as illustrated in FIG. 63(2). In this case, the timing at which the intensity P of the magnetic field generated by the magnetic field generator 5 reaches the intensity Pa is delayed from the timing at which the restraint releasing mode instruction signal Sa is turned on.

For this reason, as illustrated in FIG. 63(3), the alarm lamp controller 910c sets the on period of the alarm lamp 811 by adding the period Tda until the time tf at which the magnetic field intensity P decreases to the intensity Pt to the period Tma from the time td at which the restraint releasing mode signal Sa is actually turned on to the time te at which the magnetic field intensity P generated by the magnetic field generator 5 reaches the intensity Pa in consideration of a deviation in timing. Furthermore, the period Tda may be a constant period, and when there is a plurality of restraint releasing modes, different periods may be set so as to correspond to the time dependence of the intensity of the magnetic field generated by the magnetic field generator 5 in accordance with each type. Further, the period Tda is set in advance and is stored in the storage unit 9.

Therefore, the alarm lamp controller 910c turns on the alarm lamp on signal Lia from an off state during a period from the time td to the time tf set as the on period of the alarm lamp 811. As a result, the alarm lamp 811 is turned on at the time td, and is turned off at the time tf.

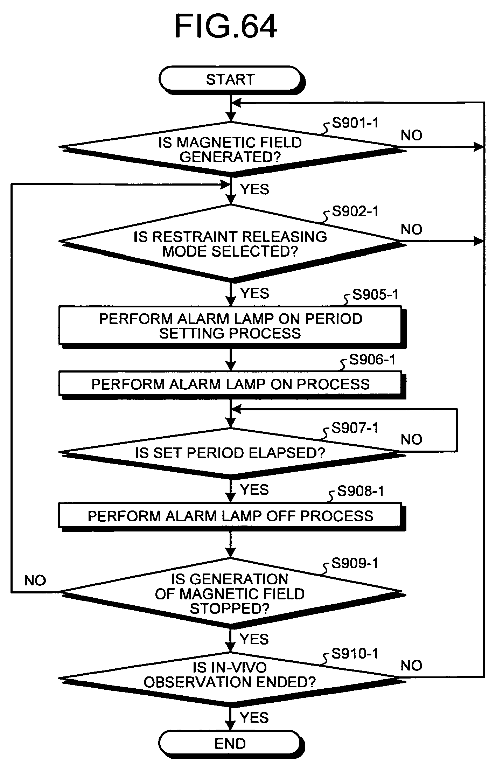

Next, referring to FIG. 64, the process of controlling the alarm lamp in the in-vivo image acquiring system 901 will be described. FIG. 64 is a flowchart illustrating another process procedure of the magnetic field generation notifying process of the in-vivo image acquiring system 901 illustrated in FIG. 60.

As illustrated in FIG. 64, as in step S901 of FIG. 62, the alarm lamp controller 910c performs a determination process (step S901-1) so as to determine whether the magnetic field generator 5 generates the magnetic field.

Later, the alarm lamp controller 910c determines whether the restraint releasing mode is selected on the basis of the instruction information input from the input unit 8 (step S902-1). When it is determined that the restraint releasing mode is not selected (step S902-1: No), the alarm lamp controller 910c returns the current step to step S901-1.

On the other hand, when it is determined that the restraint releasing mode is selected (step S902-1: Yes), the alarm lamp controller 910c performs an alarm lamp on period setting process of setting the on period of turning on the alarm lamp 811 so as to prompt the attention of the operator (step S905-1).

Subsequently, the alarm lamp controller 910c performs an alarm lamp on process (step S906-1) of turning on the alarm lamp 811 during the set on period. Later, as in step S907 of FIG. 62, the alarm lamp controller 910c performs a determination process (step S907-1) with respect to the elapsing of the set on period, an alarm lamp off process (step S908-1), and a determination process (step S909-1) with respect to the stop of the generation of the magnetic field in the magnetic field generator 5.

When it is determined that the magnetic field generator 5 does not stop the generation of the magnetic field (step S909-1: No), the alarm lamp controller 910c returns the current step to step S902-1. On the other hand, when the alarm lamp controller 910c determines that the magnetic field generator 5 stops the generation of the magnetic field (step S909-1: Yes), the control unit 910 performs a determination process (step S910-1) so as to determine whether the in-vivo observation is ended.

### Tenth Embodiment

Next, a tenth embodiment will be described. In the tenth embodiment, when a variation amount of the intensity of the magnetic field generated by the magnetic field generator becomes a predetermined reference value or more, the alarm lamp emits light so as to alarm the generation of the magnetic field to the operator or the like by use of light.

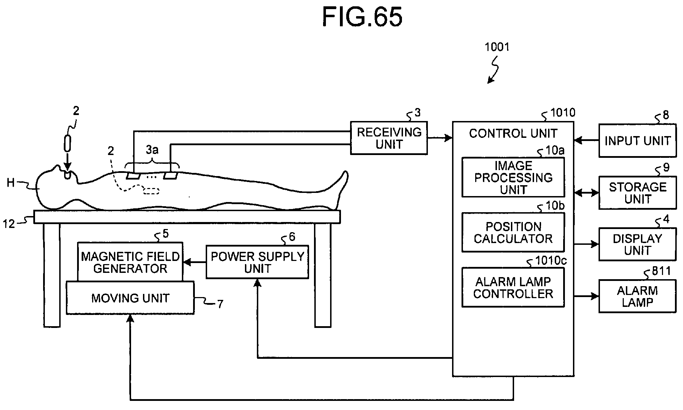

FIG. 65 is a block diagram schematically illustrating a configuration example of an in-vivo image acquiring system according to the tenth embodiment. As illustrated in FIG. 65, compared to the in-vivo image acquiring system 701 illustrated in FIG. 52, an in-vivo image acquiring system 1001 according to the tenth embodiment includes a control unit 1010a including an alarm lamp controller 1010c instead of the control unit 710. Further, the in-vivo image acquiring system 1001 includes the alarm lamp 811.

When a variation amount of the .intensity of the magnetic field generated by the magnetic field generator 5 becomes a predetermined reference value or more, the alarm lamp controller 1010c generates light in the alarm lamp 811 for warning. Specifically, the alarm lamp controller 1010c acquires the operation amount input by the joystick of the input unit 8 a plurality of times. When the variation amount of the operation amount of the joystick becomes the predetermined operation amount reference value or more, the alarm lamp controller generates light in the alarm lamp 811. In other words, when the variation amount of the intensity of the magnetic field generated by the magnetic field generator 5 so as to correspond to the operation information input by the joystick of the input unit 8 becomes the predetermined reference value or more, the alarm lamp controller 1010c turns on the alarm lamp 811.

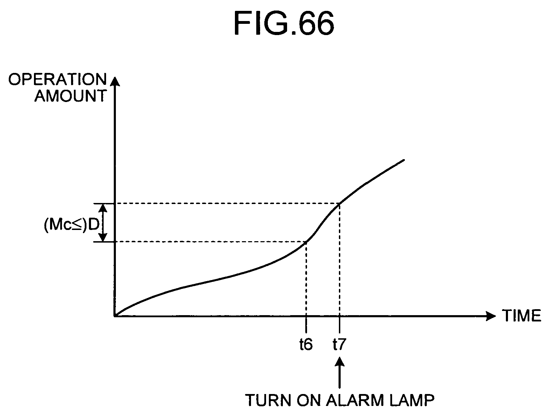

Specifically, referring to FIG. 66, the light emission control of the alarm lamp 811 using the alarm lamp controller 1010c will be described. FIG. 66 is a diagram illustrating time dependence of the operation amount of the joystick.

As illustrated in FIG. 66, when the variation amount D of the operation amount of the joystick from the time t6 to the time t7 elapsed by a predetermined unit time becomes the allowable value Mc as the predetermined operation amount reference value or more, the alarm lamp controller 1010c turns on the alarm lamp 811 at the time t7.

In this manner, in the tenth embodiment, when the variation amount of the operation amount of the joystick becomes the predetermined operation amount reference value or more, the alarm lamp 811 is turned on so as to reliably alarm the generation of the magnetic field with an intensity demanding the attention to the operator or the like. Further, when the variation amount of the operation amount of the joystick becomes the predetermined operation amount reference value or more, it corresponds to a case in which the operator operates the joystick so as to guide the capsule endoscope 2. Accordingly, the operator does not carefully watch the image of the capsule endoscope 2 for the purpose of diagnosis in many cases, so that even when the alarm lamp 811 is turned on, the operator who observes the image is not disturbed.

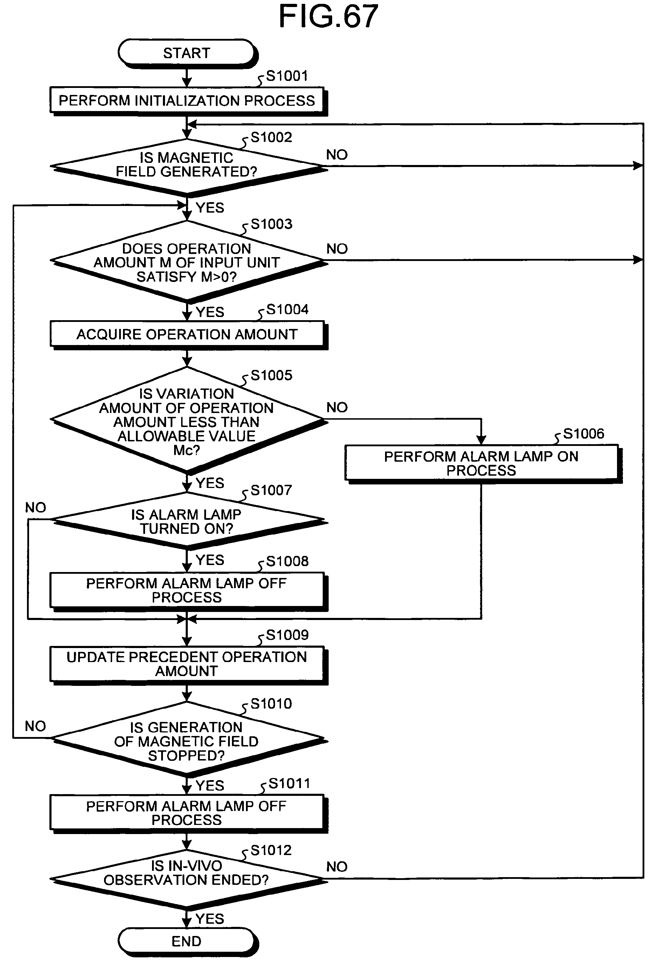

Next, referring to FIG. 67, the alarm lamp control process of the in-vivo image acquiring system 1001 will be described. FIG. 67 is a flowchart illustrating a process procedure of a magnetic field generation notifying process of the in-vivo image acquiring system 1001 illustrated in FIG. 65.

As illustrated in FIG. 67, the alarm lamp controller 1010c performs first an initialization process of initializing each numerical value used for the process of controlling the alarm lamp 811 (step S1001). For example, the alarm lamp controller 1010c sets the precedent operation amount of the joystick to 0, and turns off the control signal of the alarm lamp 811.

Subsequently, the alarm lamp controller 1010c determines whether the magnetic field generator 5 generates the magnetic field (step S1002). The alarm lamp controller 1010c repeats the determination process of step S1002 until the magnetic field generator 5 generates the magnetic field.

When it is determined that the magnetic field generator 5 generates the magnetic field (step S1002: Yes), the alarm lamp controller 1010c determines whether the operation amount M of the joystick of the input unit 8 satisfies M>0 as in step S704 of FIG. 54 (step S1003). The determination process of step S1003 is periodically performed.

When the alarm lamp controller 1010c determines that the operation amount M of the joystick of the input unit 8 does not satisfy M>0 (step S1003: No), this is a state where the joystick is not operated. Accordingly, the current step returns to step S1002 so as to determine whether the magnetic field generator 5 may generate the magnetic field.

On the other hand, when it is determined that the operation amount M of the joystick of the input unit 8 satisfies M>0 (step S1003: Yes), the alarm lamp controller 1010c acquires the operation amount of the joystick of the input unit 8 (step S1004). Subsequently, the alarm lamp controller 1010c obtains the variation amount of the operation amount by comparing the acquired operation amount and the precedent acquired operation amount, and determines whether the obtained variation amount of the operation amount is less than the allowable value Mc as the predetermined operation amount reference value (step S1005).

When it is determined that the variation amount of the operation amount is not less than the predetermined allowable value Mc (step S1005: No), that is, the variation amount of the operation amount is the predetermined allowable value Mc or more, the alarm lamp controller 1010c performs an alarm lamp on process of turning on the alarm lamp 811 so as to prompt the attention of the operator (step S1006).

On the other hand, when it is determined that the variation amount of the operation amount is less than the predetermined allowable value Mc (step S1005: Yes), the alarm lamp controller 1010c determines whether the alarm lamp 811 is turned on (step S1007). When it is determined that the alarm lamp 811 is turned on (step S1007: Yes), the alarm lamp controller 1010c performs an alarm lamp off process of turning off the alarm lamp 811 (step S1008).

When it is determined that the alarm lamp 811 is not turned on (step S1007: No), the alarm lamp controller 1010c performs the alarm lamp on process (step S1006). Alternatively, when the alarm lamp off process (step S1008) is ended, the alarm lamp controller 1010c updates the precedent operation amount of the joystick to the newly acquired operation amount (step S1009), and determines whether the magnetic field generator 5 stops the generation of the magnetic field (step S1010).

When it is determined that the magnetic field generator 5 does not stop the generation of the magnetic field (step S1010: No), the alarm lamp controller 1010c returns the current step to step S1003 so as to perform a determination process with respect to the operation amount of the input unit 8. On the other hand, when it is determined that the magnetic field generator 5 stops the generation of the magnetic field (step S1010: Yes), the alarm lamp controller 1010c performs an alarm lamp off process of turning off the alarm lamp 811 when the alarm lamp 811 is turned on (step S1011). Subsequently, the control unit 1010 determines whether the in-vivo observation is ended (step S1012). When it is determined that the in-vivo observation is not ended (step S1012: No), the control unit 1010 returns the current step to step S1002. On the other hand, when it is determined that the in-vivo observation is ended (step S1012: Yes), the control unit 1010 ends the process of controlling the alarm lamp 811 in the alarm lamp controller 1010c in accordance with the end of the in-vivo observation.

Since the in-vivo image acquiring system 1001 performs the respective process procedures illustrated in FIG. 67, when the variation amount of the operation amount of the joystick becomes the predetermined operation amount reference value or more, the alarm lamp 811 is turned on so as to reliably warn the operator or the like.

### Eleventh Embodiment

Next, an eleventh embodiment will be described. In the eleventh embodiment, when the intensity of the magnetic field generated by the magnetic field generator becomes a predetermined threshold value or more, the method of turning on the alarm lamp is changed so as to alarm the generation of the magnetic field to the operator or the like.

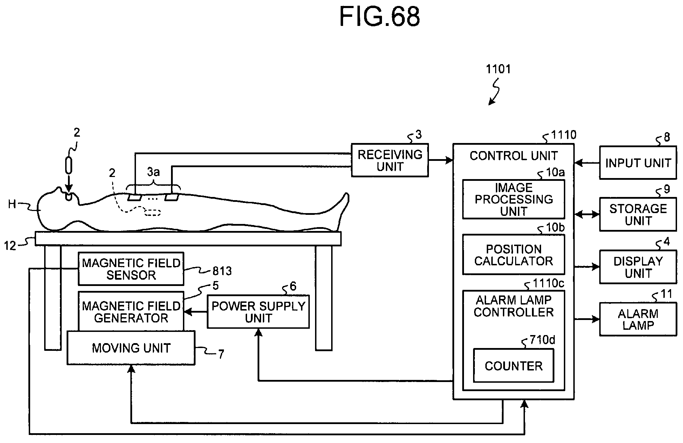

FIG. 68 is a block diagram schematically illustrating a configuration example of an in-vivo image acquiring system according to the eleventh embodiment. As illustrated in FIG. 68, compared to the in-vivo image acquiring system 1 illustrated in FIG. 1, an in-vivo image acquiring system 1101 according to the eleventh embodiment includes a control unit 1110 instead of the control unit 10. Further, the in-vivo image acquiring system 1101 includes the magnetic field sensor 813 that detects the intensity of the magnetic field generated by the magnetic field generator 5.

Compared to the control unit 10, the control unit 1110 includes an alarm lamp controller 1110c including the counter 710d with a counting function instead of the alarm lamp controller 10c. The alarm lamp controller 1110c controls the light emission process of the alarm lamp 11.

When the magnetic field generator 5 generates the magnetic field, the alarm lamp controller 1110c generates light in the alarm lamp 11 through a first lamp on method. Then, when the intensity of the magnetic field generated by the magnetic field generator 5 becomes more than a predetermined threshold value, the alarm lamp controller 1110c generates light in the alarm lamp 11 through a second lamp on method different from the first lamp on method. Further, when a variation amount of the intensity of the magnetic field generated by the magnetic field generator 5 becomes less than the predetermined reference value for a predetermined time, the alarm lamp controller 1110c stops the generation of light in the alarm lamp 11.

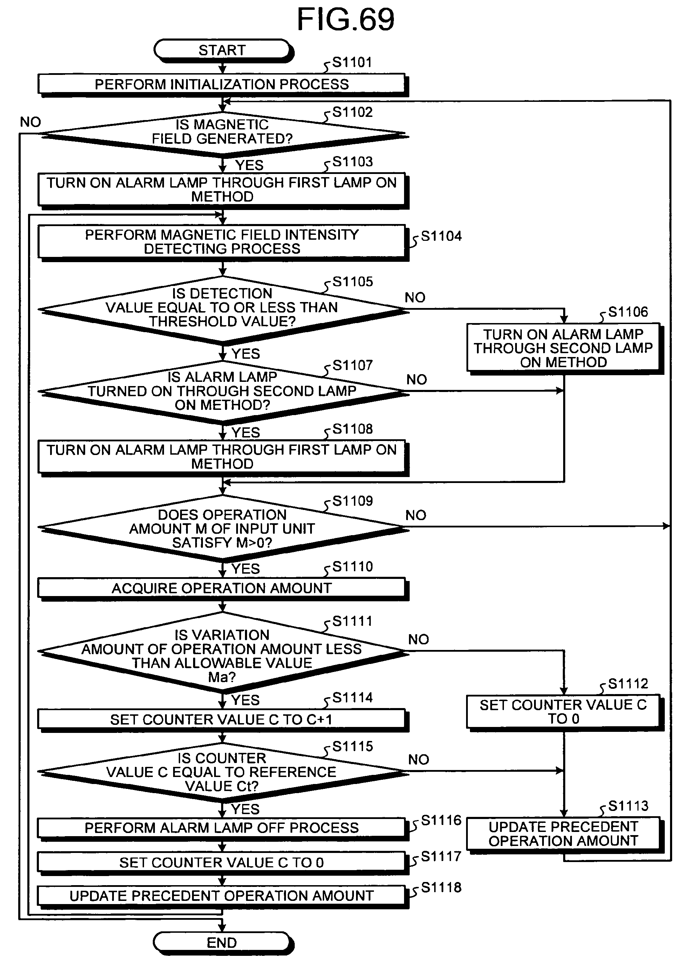

Next, referring to FIG. 69, the alarm lamp control process in the in-vivo image acquiring system 1101 will be described. FIG. 69 is a flowchart illustrating a process procedure of a magnetic field generation notifying process of the in-vivo image acquiring system 1101 illustrated in FIG. 68.

As illustrated in FIG. 69, as in step S701 and step S702 of FIG. 54, the alarm lamp controller 1110c first performs an initialization process of initializing each numerical value used for the control process of the alarm lamp 11 (step S1101), and determines whether the magnetic field generator 5 may generate the magnetic field (step S1102). For example, when the main switch of the in-vivo image acquiring system 1101 is turned off so that the generation of the magnetic field using the magnetic field generator 5 is stopped (step S1102: No), the alarm lamp controller 1110c directly ends the control process of the alarm lamp 11. On the contrary, when it is determined that the magnetic field generator 5 may generate the magnetic field (step S1102: Yes), the alarm lamp controller 1110c turns on the alarm lamp 11 through the first lamp on method (step S1103).

Subsequently, the alarm lamp controller 1110c performs a magnetic field intensity detecting process on the magnetic field sensor 813 so as to detect the intensity of the magnetic field generated by the magnetic field generator 5 (step S1104). The alarm lamp controller 1110c determines whether the detection value of the intensity of the magnetic field using the magnetic field sensor 813 becomes a predetermined threshold value or less (step S1105).

When it is determined that the detection value of the intensity of the magnetic field using the magnetic field sensor 813 is not the predetermined threshold value or less (step S1105: No), that is, the detection value of the intensity of the magnetic field using the magnetic field sensor 813 becomes more than the predetermined threshold value, the alarm lamp controller 1110c turns on the alarm lamp 11 through the second lamp on method so as to prompt the attention of the operator (step S1106). In this case, the alarm lamp controller 1110c may flicker the alarm lamp 11 through the second lamp on method, and may change the emission color of the alarm lamp 11 from the emission color in the first lamp on method.

On the other hand, when it is determined that the detection value of the intensity of the magnetic field using the magnetic field sensor 813 becomes the predetermined threshold value or less (step S1105: Yes), the alarm lamp controller 1110c determines whether the alarm lamp 11 is turned on through the second lamp on method (step S1107). When it is determined that the alarm lamp 11 is turned on through the second lamp on method (step S1107: Yes), the alarm lamp controller 1110c turns on the alarm lamp 11 through the first lamp on method (step S1108). When it is determined that the alarm lamp 11 is not turned on through the second lamp on method (step S1107: No), when step S1106 or step S1108 is ended, the alarm lamp controller 1110c determines whether the operation amount M of the joystick of the input unit 8 satisfies M>0 as in step S704 of FIG. 54 (step S1109).

When it is determined that the operation amount M of the joystick of the input unit 8 does not satisfy M>0 (step S1109: No), the alarm lamp controller 1110c returns the current step to step S1102. On the other hand, when it is determined that the operation amount M of the joystick of the input unit 8 satisfies M>0 (step S1109: Yes), the alarm lamp controller 1110c acquires the operation amount of the joystick of the input unit 8 (step S1110). Subsequently, as in step S706 of FIG. 54, the alarm lamp controller 1110c obtains the variation amount of the operation amount by comparing the acquired operation amount and the precedent acquired operation amount, and determines whether the obtained variation amount of the operation amount is less than the allowable value Ma as the predetermined operation amount reference value (step S1111).

When it is determined that the variation amount of the operation amount is not less than the predetermined allowable value Ma (step S1111: No), as in step S707 and step S708 of FIG. 54, the alarm lamp controller 1110c sets the counter value of the counter 1110d as C = 0 (step S1112), updates the precedent operation amount of the joystick to the newly acquired operation amount (step S1113), and returns to step S1102.

On the other hand, when it is determined that the variation amount of the operation amount is less than the predetermined allowable value Ma (step S1111: Yes), as in step S709 and step S710 of FIG. 54, the alarm lamp controller 1110c sets the counter value C = C + 1 (step S1114), and determines whether the counter value C is a predetermined reference value Ct (step S1115).

When the alarm lamp controller 1110c determines that the counter value C is not the predetermined reference value Ct (step S1115: No), this corresponds to the case where the period in which the variation amount of the operation amount is less than the predetermined operation amount reference value does not reach the predetermined time Ta used as the reference for turning off the alarm lamp 11. Accordingly, the alarm lamp controller updates the precedent operation amount of the joystick to the newly acquired operation amount (step S1113), and returns the current step to step S1102.

Further, when the alarm lamp controller 1110c determines that the counter value C is the predetermined reference value Ct (step S1115: Yes), this corresponds to the case where the period in which the variation amount of the operation amount is less than the predetermined operation amount reference value reaches the predetermined time Ta used as the reference for turning off the alarm lamp 11. For this reason, as in step S711 to step S713 of FIG. 54, the alarm lamp controller 1110c performs an alarm lamp off process of turning off the alarm lamp 11 (step S1116), sets the counter value C as C = 0 (step S1117), updates the precedent operation amount of the joystick to the newly acquired operation amount (step S1118), and returns the current step to step S1104.

In this manner, in the eleventh embodiment, when the intensity of the magnetic field generated by the magnetic field generator 5 becomes more than the predetermined threshold value, the method of turning on the alarm lamp 11 is changed so as to reliably alarm the generation of the magnetic field with an intensity demanding the attention to the operator or the like. Then, in the eleventh embodiment, when the variation amount of the operation amount of the joystick becomes less than the predetermined operation amount reference value for a predetermined time, the alarm lamp 11 is turned off so as to help the operator smoothly observe the image.

Furthermore, in the eleventh embodiment, a current detecting unit may be provided instead of the magnetic field sensor 813 so as to detect the value of the current flowing to the coil-shaped electromagnet constituting the magnetic field generator 5, and the method of turning on the alarm lamp 11 may be changed on the basis of whether the current value detected by the current detecting unit is the current value supplied to generate a magnetic field with an intensity in accordance with a predetermined threshold value.

Further, in the first to eleventh embodiments, a case has been described in which the capsule endoscope 2 with a plurality of single imaging units is used, but of course, a pantoscopic capsule endoscope with a plurality of imaging units may be used.

Further, in the first to eleventh embodiments, an example has been described in which the capsule endoscope 2 using the magnet 24 is used, but of course, the invention is not limited thereto. Instead of the magnet 24, a capsule endoscope including an electromagnet may be used.

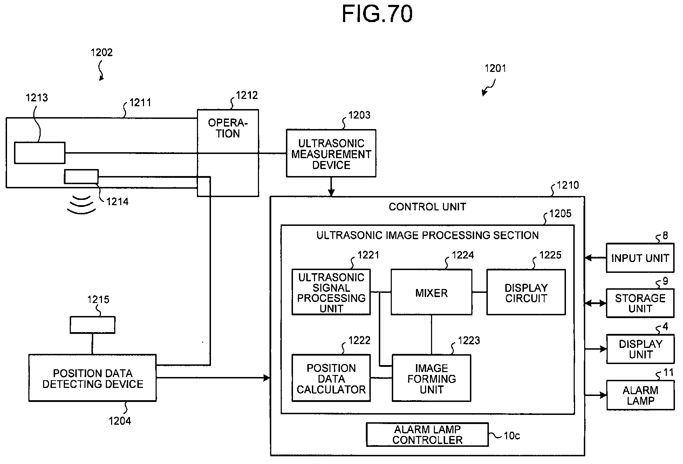

Further, in the first to eleventh embodiments, the in-vivo image acquiring system acquiring the in-vivo image using the capsule endoscope 2 with a capturing function has been exemplified, but of course, the invention is not limited thereto. The invention may be applied to the case of using an ultrasonic endoscope which inserts a probe into a body and emits an ultrasonic wave thereto. Referring to FIG. 70, a case will be described in which the first embodiment is applied to the ultrasonic diagnosis device.

As illustrated in FIG. 70, an ultrasonic diagnosis device 1201 includes a probe 1202, an ultrasonic measurement device 1203, a position data detecting device 1204, a control unit 1210 including an ultrasonic image processing unit 1205 and the alarm lamp controller 10c, the display unit 4, the input unit 8, the storage unit 9, and the alarm lamp 11.

The probe 1202 is inserted into a body cavity, and includes an insertion section that has an ultrasonic vibrator 1213 and a receiving coil 1214 provided at the distal end thereof and is formed of a flexible material and an operation unit 1212. The ultrasonic vibrator 1213 radially transmits and receives an ultrasonic wave in the direction perpendicular to the insertion shaft of an insertion section 1211, and converts a received reflected wave into an electrical ultrasonic signal. Further, the receiving coil 1214 is set near the ultrasonic vibrator 1213 so as to have a predetermined position and a predetermined direction with respect to the probe 1202, receives a magnetic field from a transmitting coil 1215, and outputs an electrical signal corresponding to a received magnetic field to the position data detecting device 1204. Further, the ultrasonic measurement device 1203 drives the ultrasonic vibrator 1213, and outputs an ultrasonic signal converted by the ultrasonic vibrator 1213 to an ultrasonic signal processing portion 1221 of the ultrasonic image processing unit 1205. Further, the transmitting coil 1215 transmits a magnetic field to the peripheral space. The position data detecting device 1204 excites the transmitting coil 1215, converts an electrical signal input from the receiving coil 1214 into a digital numerical position signal and an alignment signal, and outputs the result to a position data calculator 1222 of the ultrasonic image processing unit 1205.

The control unit 1210 controls the respective components of the ultrasonic diagnosis device 1201. The ultrasonic image processing unit 1205 includes the ultrasonic signal processing portion 1221, the position data calculator 1222, an image forming unit 1223, a mixer 1224, and a display circuit 1225. The ultrasonic signal processing portion 1221 performs a predetermined process, creates one sheet of two-dimensional ultrasonic image data perpendicular to the insertion shaft of the insertion section 1211 with respect to a scanning operation performed once by the ultrasonic vibrator 1213, and outputs the result to the image forming unit 1223 and the mixer 1224. When the operator manually extracts the insertion section 1211 from the body cavity while repeating the scanning operation using the ultrasonic vibrator 1213, a plurality of continuous two-dimensional ultrasonic image data in the extraction path is output to the image forming unit 1223 and the mixer 1224. The position data calculator 1222 obtains the position alignment information of the transmitting coil 1215 from the input position signal, and outputs the result to the image forming unit 1223. The image forming unit 1223 specifies a positional relation between images by using the two-dimensional ultrasonic image data and the alignment information simultaneously obtained through the scanning operation, interpolates the adjacent two-dimensional ultrasonic image data to create three-dimensional ultrasonic image data, and outputs the result to the mixer 1224. The mixer 1224 outputs the two-dimensional ultrasonic image data and the three-dimensional ultrasonic image data to the display circuit 1225. The display circuit 1225 outputs the three-dimensional ultrasonic image data or the two-dimensional ultrasonic image data or both to the display unit in accordance with the display type input by the user. Then, the display unit 4 displays the input image data.

Then, the alarm lamp 11 is disposed at the outside observation area as an area excluding a straight extension line connecting the display constituting the display unit 4 and a diagnostician inside the viewing area of the diagnostician of the ultrasonic diagnosis device 1201.
Then, the alarm lamp controller 10c emits light from the alarm lamp 11 which has been turned off when the transmitting coil 1215 transmits a magnetic field. Then, the alarm lamp controller 10c stops the generation of light in the alarm lamp 11 when the transmitting coil 1215 stops the transmission of the magnetic field.

In this manner, even when the invention is applied to the ultrasonic diagnosis device 1201, the alarm lamp 11 is disposed at the outside observation area of the diagnostician, and the light emission of the alarm lamp 11 is controlled in accordance with the transmission of the magnetic field, and a is installed in the viewing area of the operator N. Accordingly, it is possible to accurately notify the diagnostician of the generation of the magnetic field and help the diagnostician smoothly observe the ultrasonic image by emitting light from the alarm lamp 11a. Of course, the invention is not limited to the ultrasonic diagnosis device, and may be applied to an X-ray diagnosis device using an X-ray.

### EXPLANATIONS OF LETTERS OR NUMERALS

1, 201, 201d, 201e, 301, 301d, 301e, 401, 401d, 401e, 501, 501d, 501e, 601, 601d, 601e, 701, 801, 801a, 901, 1001, 1101 IN-VIVO IMAGE ACQUIRING SYSTEM
2 CAPSULE ENDOSCOPE
3 RECEIVING UNIT
3a ANTENNA
4 DISPLAY UNIT
4a DISPLAY
4b, 41b DISPLAY SCREEN
4c, 41c IN-VIVO IMAGE
5 MAGNETIC FIELD GENERATOR
5a to 5e ELECTROMAGNETS
5f TABLE
6 POWER SUPPLY UNIT
7 MOVING UNIT
8 INPUT UNIT
9 STORAGE UNIT
10, 210, 210a, 210b, 310, 310a, 310b, 410, 410a, 410b, 510, 510f, 510g, 610, 610a, 610b, 710, 810, 810a, 910, 1010, 1110 CONTROL UNIT
10a, 510a, 510d, 510e IMAGE PROCESSING UNIT
10b POSITION CALCULATOR
10c, 210c, 210d, 210e, 310c, 310d, 310e, 410c, 410d, 410e, 710c, 810c, 810d, 910c, 1010c, 1110c ALARM LAMP
CONTROLLER
11, 11a, 11b, 811 ALARM LAMP
13 THREE-DIMENSIONAL SPACE
20 CAPSULE-SHAPED CASING
20a CYLINDRICAL CASING
20b DOME-SHAPED CASING
21a ILLUMINATION UNIT
21b OPTICAL SYSTEM
21c IMAGING UNIT
22 SIGNAL PROCESSING UNIT
23 TRANSMITTING UNIT
24 MAGNET
28 CONTROL UNIT
29 POWER SUPPLY UNIT
208d OPTICAL SENSOR
208e IMAGE SENSOR
312 ALARM LAMP TRANSFERRING UNIT
610c, 610d, 610e SCREEN CONTROLLER
710d COUNTER
813 MAGNETIC FIELD SENSOR
813a CURRENT DETECTING UNIT
1201 ULTRASONIC DIAGNOSIS DEVICE
1202 PROBE
1203 ULTRASONIC MEASUREMENT DEVICE
1204 POSITION DATA DETECTING DEVICE
1205 ULTRASONIC IMAGE PROCESSING UNIT
1211 INSERTION SECTION
1212 OPERATION UNIT
1213 ULTRASONIC VIBRATOR
1214 RECEIVING COIL
1215 TRANSMITTING COIL
1221 ULTRASONIC SIGNAL PROCESSING PORTION
1222 POSITION DATA CALCULATOR
1223 IMAGE FORMING UNIT
1224 MIXER
1225 DISPLAY CIRCUIT

## Claims

1. An in-vivo information acquiring system (1; 801; 801a) comprising:
a body-insertable apparatus (2) that includes a magnetic field responding unit (24), and which is adapted to acquire in-vivo information and to transmit the acquired in-vivo information to the outside thereof;
a magnetic field generator (5) that generates a magnetic field for the magnetic field responding unit (24);
a magnetic field control unit (10; 810; 810a) that controls a generation of the magnetic field using the magnetic field generator (5);
a receiving unit (3) that receives the in-vivo information transmitted from the body-insertable apparatus (2);
a processing unit (10a) that processes the in-vivo information received by the receiving unit (3); and
a display unit (4) that displays the in-vivo information processed by the processing unit (10a) on a predetermined display screen, **characterized in that** the in-vivo information acquiring system (1; 801; 801a) comprises:
an alarm lamp (11; 811) adapted to notify a generation state of the magnetic field using the magnetic field generator (5) by use of light; and
a control unit (10c; 810c; 810d) adapted to control a light emission process of the alarm lamp (11; 811) such that an operator who visually identifies the in-vivo information is less disturbed by the light from the alarm lamp (11; 811),
wherein the control unit (10c; 810c; 810d) is adapted to generate light in the alarm lamp
(11; 811) when an intensity of the magnetic field generated by the magnetic field generator (5) becomes more than a predetermined threshold value.

2. The in-vivo information acquiring system (801) according to claim 1, further comprising a magnetic field intensity detecting unit (813) that detects the intensity of the magnetic field generated by the magnetic field generator (5),
wherein the control unit (810c) generates light in the alarm lamp (811) when the intensity of the magnetic field detected by the magnetic field intensity detecting unit (813) becomes more than the predetermined threshold value.

3. The in-vivo information acquiring system (801a) according to claim 1, wherein
the magnetic field generator (5) includes at least one or more coils (5a to 5e),
the in-vivo information acquiring system (1) further comprises a current detecting unit (813a) that detects a value of current flowing to the coil (5a to 5e), and
the control unit (810d) generates light in the alarm lamp (811) when the current value detected by the current detecting unit (813a) becomes more than a value of a current supplied to generate a magnetic field with an intensity corresponding to the predetermined threshold value.

## Patentansprüche

1. In-vivo Informationsaufnahmesystem (1; 801; 801a), das umfasst:
ein in einen Körper einführbares Gerät (2), das eine auf ein Magnetfeld reagierende Einheit (24) umfasst und das dazu eingerichtet ist, eine in-vivo Information aufzunehmen und die aufgenommene in-vivo Information nach außen zu übertragen;
einen Magnetfeldgenerator (5), der ein Magnetfeld für die auf ein Magnetfeld reagierende Einheit (24) erzeugt;
eine Magnetfeldsteuereinheit (10; 810; 810a), die eine Erzeugung des Magnetfelds unter Verwendung des Magnetfeldgenerators (5) steuert;
eine Empfangseinheit (3), die die von dem in einen Körper einführbaren Gerät (2) übertragene in-vivo Information empfängt;
eine Verarbeitungseinheit (10a), die die von der Empfangseinheit (3) empfangene in-vivo Information verarbeitet; und
eine Anzeigeeinheit (4), die die von der Verarbeitungseinheit (10a) verarbeitete in-vivo Information auf einem vorbestimmten Anzeigebildschirm anzeigt, **dadurch gekennzeichnet, dass** das in-vivo Informationsaufnahmesystem (1; 801; 801a) umfasst:
eine Alarmlampe (11; 811), die dazu eingerichtet ist, einen Erzeugungsstatus des Magnetfelds unter Verwendung des Magnetfeldgenerators (5) durch unter Verwendung von Licht anzuzeigen; und
eine Steuereinheit (10c; 810c; 810d), die dazu eingerichtet ist, einen Lichtemissionsprozess der Alarmlampe (11; 811) so zu steuern, dass ein Operateurs, der die in-vivo Information visuell identifiziert durch das Licht von der Alarmlampe (11; 811) weniger gestört ist,
wobei die Steuereinheit (10c; 810c; 810d) dazu eingerichtet ist, Licht in der Alarmlampe (11; 811) zu erzeugen, wenn eine Intensität des von dem Magnetfeldgenerator (5) erzeugten Magnetfelds größer als ein vorbestimmter Schwellwert wird.

2. In-vivo Informationsaufnahmesystem (801) nach Anspruch 1, das ferner eine Magnetfeldintensitätserfassungseinheit (813) umfasst, die die Intensität des von dem Magnetfeldgenerator (5) erzeugten Magnetfelds erfasst,
wobei die Steuereinheit (810c) Licht in der Alarmlampe (811) erzeugt, wenn eine Intensität des von der Magnetfeldintensitätserfassungseinheit (813) erfassten Magnetfelds größer als ein vorbestimmter Schwellwert wird.

3. In-vivo Informationsaufnahmesystem (801a) nach Anspruch 1, wobei
der Magnetfeldgenerator (5) mindestens eine oder mehrere Spule(n) (5a bis 5e) umfasst,
das in-vivo Informationsaufnahmesystem (1) ferner eine Stromerfassungseinheit (813a) umfasst, die einen Wert eines zu der Spule (5a bis 5e) fließenden Stroms erfasst, und
die Steuereinheit (810d) Licht in der Alarmlampe (811) erzeugt, wenn der von der Stromerfassungseinheit (813a) erfasste Stromwert größer als ein Wert eines Stroms wird, der zugeführt wird, um ein Magnetfeld mit einer dem vorbestimmten Schwellwert entsprechenden Intensität zu erzeugen.

## Revendications

1. Système (1 ; 801 ; 801a) d'acquisition d'informations *in vivo* comprenant :
un appareil (2) insérable dans un corps qui inclut une unité (24) de réponse à un champ magnétique, et qui est adapté à acquérir des informations *in vivo* et à transmettre les informations *in vivo* acquises à l'extérieur de celui-ci ;
un générateur (5) de champ magnétique qui génère un champ magnétique pour l'unité (24) de réponse à un champ magnétique ;
une unité (10 ; 810 ; 810a) de commande de champ magnétique qui commande une génération du champ magnétique en utilisant le générateur (5) de champ magnétique ;
une unité (3) de réception qui reçoit les informations *in vivo* transmises depuis l'appareil (2) insérable dans un corps ;
une unité (10a) de traitement qui traite les informations *in vivo* reçues par l'unité (3) de réception ; et
une unité (4) d'affichage qui affiche les informations *in vivo* traitées par l'unité (10a) de traitement sur un écran d'affichage prédéterminé,
**caractérisé en ce que** le système (1 ; 801 ; 801a) d'acquisition d'informations *in vivo* comprend :
une lampe (11 ; 811) d'alarme adaptée à notifier un état de génération du champ magnétique en utilisant le générateur (5) de champ magnétique par l'utilisation d'une lumière ; et
une unité (10c ; 810c ; 810d) de commande adaptée à commander un processus d'émission de lumière de la lampe (11 ; 811) d'alarme de façon à ce qu'un opérateur qui identifie visuellement les informations *in vivo* soit moins troublé par la lumière de la lampe (11 ; 811) d'alarme,
dans lequel l'unité (10c ; 810c ; 810d) de commande est adaptée à générer une lumière dans la lampe (11 ; 811) d'alarme lorsqu'une intensité du champ magnétique généré par le générateur (5) de champ magnétique devient supérieure à une valeur de seuil prédéterminée.

2. Système (801) d'acquisition d'informations *in vivo* selon la revendication 1, comprenant en outre une unité (813) de détection d'intensité de champ magnétique qui détecte l'intensité du champ magnétique généré par le générateur (5) de champ magnétique,
dans lequel l'unité (810c) de commande génère une lumière dans la lampe (811) d'alarme lorsque l'intensité du champ magnétique détectée par l'unité (813) de détection d'intensité de champ magnétique devient supérieure à la valeur de seuil prédéterminée.

3. Système (801a) d'acquisition d'informations *in vivo* selon la revendication 1, dans lequel
le générateur (5) de champ magnétique inclut au moins une ou plusieurs bobine(s) (5a à 5e),
le système (1) d'acquisition d'informations *in vivo* comprend en outre une unité (813a) de détection de courant qui détecte une valeur de courant atteignant la bobine (5a à 5e), et
l'unité (810d) de commande génère une lumière dans la lampe (811) d'alarme lorsque la valeur de courant détectée par l'unité (813a) de détection de courant devient supérieure à une valeur de courant délivrée pour générer un champ magnétique avec une intensité correspondant à la valeur de seuil prédéterminée.
